(19) Europäisches Patentamt
European Patent Office
Office européen des brevets

(11) Veröffentlichungsnummer: **0 129 128**
**B1**

(12) **EUROPÄISCHE PATENTSCHRIFT**

(45) Veröffentlichungstag der Patentschrift: **22.11.90**

(21) Anmeldenummer: **84106336.5**

(22) Anmeldetag: **04.06.84**

(51) Int. Cl.$^5$: **C 07 D 403/12,** C 07 D 417/12, C 07 D 239/42, C 07 D 401/12, C 07 D 403/14, C 07 D 403/04, A 61 K 31/505, A 61 K 31/54

# (54) 2-Pyrimidinyl-I-piperazin-Derivate, Verfahren zu ihrer Herstellung und diese enthaltende Arzneimittel.

(30) Priorität: **18.06.83 DE 3321969**

(43) Veröffentlichungstag der Anmeldung: **27.12.84 Patentblatt 84/52**

(45) Bekanntmachung des Hinweises auf die Patenterteilung: **22.11.90 Patentblatt 90/47**

(84) Benannte Vertragsstaaten: **AT BE CH DE FR GB IT LI LU NL SE**

(56) Entgegenhaltungen:
**EP-A-0 111 226**
**FR-A-2 051 556**
**FR-A-2 506 771**
**FR-A-2 518 993**
**US-A-2 672 460**

**E. SCHROEDER et al., Arzneimittelchemie I, Verlag G. Thieme, Stuttgart, 1976, S. 26**

**Die Akte enthält technische Angaben, die nach dem Eingang der Anmeldung eingereicht wurden und die nicht in dieser Patentschrift enthalten sind.**

(73) Patentinhaber: **Troponwerke GmbH & Co. KG**
**Berliner Strasse 156**
**D-5000 Köln 80 (DE)**

(72) Erfinder: **Seidel, Peter-Rudolf, Dr.**
**Alte Heide 5D**
**D-5000 Köln 90 (DE)**
Erfinder: **Horstmann, Harald, Dr.**
**Claudiusweg 19**
**D-5600 Wuppertal 1 (DE)**
Erfinder: **Traber, Jörg, Dr**
**Löwenburgstrasse 12**
**D-5204 Lohmar 21 (DE)**
Erfinder: **Dompert, Wolfgang, Dr.**
**Mühlenweg 2**
**D-5064 Rösrath-Forsbach (DE)**
Erfinder: **Glaser, Thomas, Dr.**
**Rybnikerstrasse 6**
**D-5000 Köln 80 (DE)**
Erfinder: **Schuurman, Teunis, Dr.**
**Starenweg 17**
**D-5063 Overath (DE)**

(74) Vertreter: **Adrian, Albert, Dr. et al**
**c/o BAYER AG Konzernverwaltung RP**
**Patentabteilung**
**D-5090 Leverkusen 1, Bayerwerk (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 129 128 B1

**Beschreibung**

Die vorliegende Erfindung betrifft neue substituierte 2-Pyrimidinyl-1-piperazin-Derivate der allgemeinen Formel (I), Verfahren zu ihrer Herstellung sowie diese enthaltende Arzneimittel, insbesondere das zentrale Nervensystem beeinflussende Mittel.

In US 2 672 460 werden 1-Pyrimidyl-4-phtrhalimidoylacylpiperazine mit analgetischer Wirkung beschrieben.

Es wurde gefunden, daß die neuen substituierten 2-Pyrimidinyl-1-piperazine-Derivate der allgemeinen Formel (I)

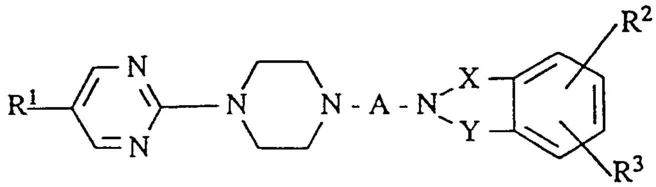

(I)

in welcher

A für eine Alkylengruppe mit 1 bis 6 Kohlenstoffatomen oder für 2-Hydroxy-n-propylen steht, wobei A nicht für die 2-Hydroxy-n-propylengruppe steht, wenn X und Y für Carbonyl und $R^1$, $R^2$ und $R^3$ für Wasserstoff stehen.

$R^1$ für Wasserstoff, Trifluormethyl oder für Alkoxy mit 1 bis 4 Kohlenstoffatomen, Trifluormethoxy oder für Hydroxy, Halogen, Cyano, Nitro, Amino, gegebenenfalls substituiert durch 1 oder 2 Methyl oder Ethyl, oder für Acetylamino oder Benzoylamino, oder für Indol-3-yl, oder für Phenyl steht, wobei der Phenylrest einfach oder mehrfach durch Methoxy, Ethoxy, Nitro, Halogen, Trifluormethyl, Amino, Cyano, Hydroxy, Methyl oder Ethyl, Acetylamino oder Benzoylamino substituiert sein kann,

$R^2$ und $R^3$ gleich oder verschieden sein können und für Wasserstoff oder Alkoxy mit 1 bis 4 Kohlenstoffatomen, oder für Phenyl, oder für Hydroxy, Halogen, Cyano, Nitro, Amino, wobei das Stickstoffatom des Aminorestes durch 1 oder 2 Methyl oder Ethyl substituiert ist, oder für Methylsulfonamide, Benzol- und Toluolsulfonamido, Acetylamino, Ethoxycarbonylamino oder für Carboxyl, Methoxy- oder Ethoxycarbonyl, oder für Phenoxycarbonylamino, oder für Carbamoyl oder Sulfamoyl steht,

X und Y gleich oder verschieden sein können und Carbonyl oder Sulfonyl bedeuten, und

X allein für Carbonyl oder Sulfonyl steht, wenn Y gleichzeitig $—CO—CH_2$ oder $—CO—N(R^4)—$ bedeutet, wobei $R^4$ für Wasserstoff, Methyl oder Phenyl steht, sowie deren Säureadditionssalze gute pharmakologische Eigenschaften aufweisen.

Die neuen erfindungsgemäßen Verbindungen der allgemeinen Formel (I) sind insbesondere ausgezeichnet zentral wirksam und weisen insbesondere eine anxiolytische, neuroleptische, antidepressive und nootrope Wirksamkeit auf.

Die erfindungsgemäßen substituierten 2-Pyrimidinyl-1-piperazin-Derivate sind durch die Formel (I) allgemein definiert. Bevorzugt sind diejenigen Verbindungen der Formel (I), in denen

A für Ethylen, n-Propylen, n-Butylen, 2-Methyl-n-propylen oder 2-Hydroxy-n-propylen steht, wobei A nicht für die 2-Hydroxy-n-propylen-gruppe steht, wenn X und Y für Carbonyl und $R^1$, $R^2$ und $R^3$ für Wasserstoff stehen,

$R^1$ für Wasserstoff oder für Methoxy, Ethoxy oder für Fluor, Iod, Cyano oder für Indol-3-yl, oder für Phenyl steht, wobei der Phenylrest einfach oder mehrfach durch Methoxy oder Ethoxy, Chlor, Fluor, Trifluormethyl, Cyano substituiert sein kann,

$R^2$ und $R^3$ gleich oder verschieden sein können und für Wassestoff, oder für Methoxy, Trifluormethoxy oder für Chlor, Fluor, Cyano, Nitro, Amino, wobei das Stickstoffatom des Aminorestes durch 1 oder 2 Methyl oder Ethyl substituiert ist, oder für Methylsulfonamido, Acetylamino, Ethoxycarbonylamino steht,

X und Y gleich oder verschieden sein können und Carbonyl oder Sulfonyl bedeuten, und

X allein für Carbonyl oder Sulfonyl steht, wenn Y gleichzeitig $—CO—CH_2—$ oder $—CO—N(R^4)—$ bedeutet, wobei $R^4$ für Wasserstoff oder Phenyl steht, sowie deren Säureadditionssalze.

Die neuen Verbindungen der allgemeinen Formel (I) zeigen überraschenderweise ausgeprägte und vorteilhafte Wirkungen auf das zentrale Nervensystem. Besonders werwähnt sei ihre Verwendung als anxiolytische, tranquillisierende, neuroleptische, antidepressive, antiamnestische und nootrope sowie lern-, leistungs- und gedächtnisverbessernde Wirkstoffe. (Antiamnestische Wirkstoffe sind Wirkstoffe, die bei Formen von Gedächnisstörungen, Gedächtnisschwäche und Errinnerungslosigkeit verwendet werden. Siehe hierzu Wörterbuch der Psychiatrie und medizinischen Psychologie, Verlag Urban und Schwarzenberg, München (1977)). Die neuen Verbindungen besitzen ferner analgetische und antiphlogistische Wirkungen, die z.B. am Carrageein-Pfotenödem bei Ratten nachgewiesen werden konnten.

Die vorliegende Erfindung betrifft weiterhin Verfahren zur Herstellung von Verbindungen der allgemeinen Formel (I), dadurch gekennzeichnet, daß man (*a*) Pyrimidinylpiperazin-Derivate der allgemeinen Formel (II)

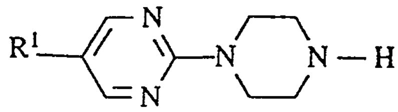

(II)

in welcher
$R^1$ die oben angegebene Bedeutung hat,
mit Verbindungen der allgemeinen Formel (III)

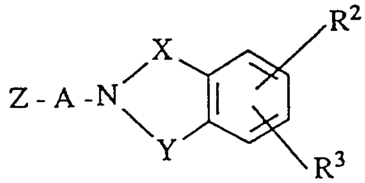

(III)

in welcher
$R^2$, $R^3$, X, Y und A die oben angegebene Bedeutung haben, und
Z eine Gruppe, Hydroxy, Chlor, Brom, Jod, $—OSO_2CH_3$, oder

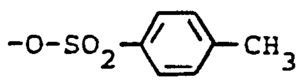

bedeutet, oder

A—Z für 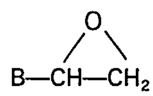

steht, wobei B dem um zwei endständige Kohlenstoffatome verkürzten Brückenglied A entspricht, in Gegenwart von inerten Lösungsmitteln bei Temperaturen zwischen 20 und 200°C, alkyliert und die so erhaltenen Verbindungen der Formel (I) gegebenenfalls anschließend in bekannter Weise in die Säureadditionssalze überführt, oder (*b*) Aminoalkylpyrimidinylpiperazin-Derivate der allgemeinen Formel (IV)

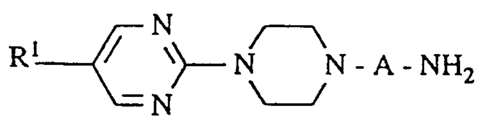

(IV)

in welcher
$R^1$ und A die oben angegebene Bedeutung haben,
mit den Anhydriden der allgemeinen Formel (V)

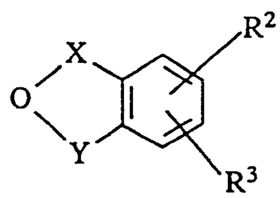

(V)

in welcher
$R^2$ und $R^3$ die oben angegebene Bedeutung, haben,
X und Y gleich oder verschieden sein können und Carbonyl oder Sulfonyl bedeuten, oder
X Carbonyl bedeutet, wenn Y gleichzeitig $—CO—CH_2—$ bedeutet,
bei erhöhter Temperatur zwischen 100 und 250°C in einem inerten organischen Lösungsmittel oder ohne Lösungsmittel in der Schmelze innerhalb des obengenannten Temperaturbereichs umsetzt, oder (*c*) Pyrimidinylpiperazin-Derivate der allgemeinen Formel (VI)

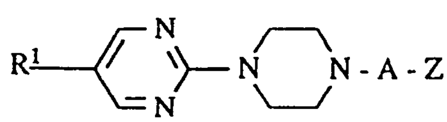

(VI)

in welcher
$R^1$, A und Z die oben angegebene Bedeutung haben,
mit Verbindungen der allgemeinen Formel (VIII)

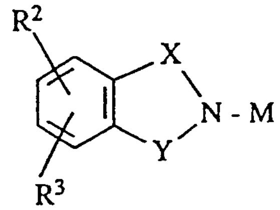

(VII)

in welcher

$R^2$, $R^3$, X und Y die oben angegebene Bedeutung haben und

M für Wasserstoff oder Natrium, Kalium oder Lithium, steht,

in Gegenwart von inerten Lösungsmitteln bei Temperaturen zwischen 20 und 180°C umsetzt, oder (*d*) Pyrimidinylpiperazin-Derivate der allgemeinen Formel (VI-a)

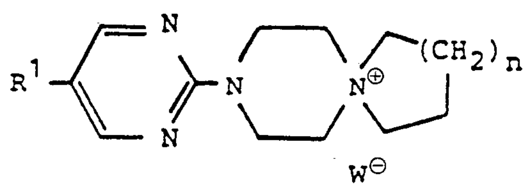

(VI-a),

in welcher

$R^1$ die oben angegebenen Bedeutung hat, und

W für Chlor, Brom oder Jod steht und

n 1 oder 2 bedeutet,

mit Verbindungen der allgemeinen Formel (VII-a)

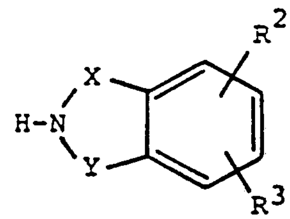

(VII-a),

in welcher

$R^2$, $R^3$, X und Y die oben angegebene Bedeutung haben,

in Gegenwart geeigneter Basen in einem inerten Lösungsmittel bei Temperaturen zwischen 20 und 180°C umsetzt.

Die vorliegende Erfindung betrifft weiterhin ein Verfahren zur Herstellung von Verbindungen der allgemeinen Formel (I)

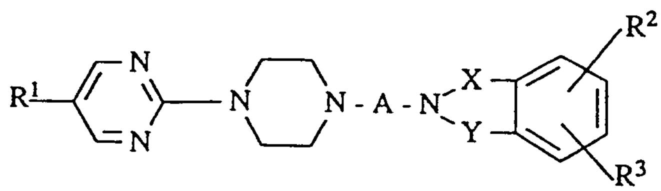

(I)

in welcher

A, $R^1$, $R^2$, $R^3$ und X die oben angegebene Bedeutung haben, und

Y für —CO—N($R^4$)— steht,

dadurch gekennzeichnet, daß man Pyrimidinylpiperazin-Derivate der allgemeinen Formel (VIII)

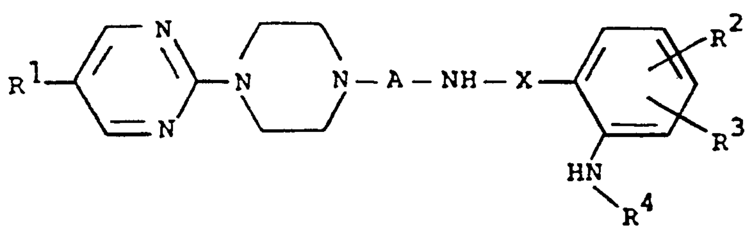

(VIII),

in welcher

A, $R^1$, $R^2$, $R^3$, $R^4$ und X die oben angegebene Bedeutung haben,

mit den Carbonylverbindungen der allgemeinen Formel (IX)

$R^5$—CO—$R^6$ (IX),

in welcher

$R^5$ Halogen, Alkoxy, Amino oder Imidazolyl und

$R^6$ Halogen, Trihalogenalkyl, Alkoxy, Aryloxy, Alkoxycarbonyloxy, Amino oder Imidazolyl bedeuten, in Gegenwart von inerten Lösungsmitteln bei Temperaturen zwischen 20 und 180°C oder ohne Lösungsmittel bei Temperaturen zwischen 50 und 200°C umsetzt.

Von besonderer Bedeutung sind Verbindungen der allgemeinen Formel (I), in welcher

A Ethylen, n-Propylen, n-Butylen oder 2-Hydroxy-n-propylen bedeutet wobei A nicht für 2-Hydroxy-n-propylen steht, wenn X und Y für Carbonyl und $R^1$, $R^2$ und $R^3$ für Wasserstoff stehen,

$R^1$ Wasserstoff, Chlor, Fluor, Iod, 4-Methoxy-phenyl, 3,4-Dimethoxyphenyl, Phenyl, 4-Chlor-phenyl, 3-(Trifluormethyl)-phenyl, 2-Chlor-6-fluor-phenyl oder Indol-3-yl bedeutet,

$R^2$ und $R^3$ gleich oder verschieden sein können und für Wasserstoff, Fluor, Chlor oder Nitro stehen,

X für Carbonyl udn Sulfonyl steht und

Y für Carbonyl, Sulfonyl, —CO—$CH_2$— oder —CO—N($R^4$)— steht, wobei

$R^4$ Wasserstoff, Phenyl oder Methyl bedeutet,

sowie deren Säureadditionssalze.

Erfindungsgemäß erhält man die neuen Verbindungen der allgemeinen Formel (I) nach den folgenden Verfahren durch

a) Umsetzung einer Verbindung der allgemeinen Formel (II) mit einer Verbindung der allgemeinen Formel (III) nach folgendem Schema:

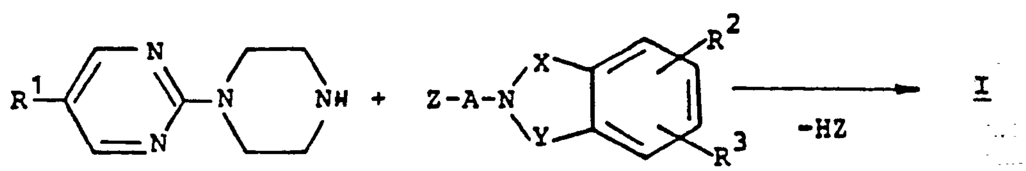


(II) (III)

in denen

A, X, Y, Z, $R^1$, $R^2$ und $R^3$ die jeweils oben angegebene Bedeutung haben.

Die Umsetzung erfolgt in Lösungsmitteln, die gegenüber den jeweiligen Reaktionspartnern inert sind. Hierzu gehören vorzugsweise Ether wie Diethylether, Diisoproplylether, Tetrahydrofuran, Dioxan, Ethylenglykoldimethylether, Ethylenglykoldiethylether; Kohlenwasserstoffe wie Ligroin, Benzol, Toluol, Xylol, Tetralin, Halogenkohlenwasserstoffe wie Chloroform, Methylenchlorid, Chlorbenzol, Dichlorbenzole; Nitrile wie Acetonitril, Propionitril; Ketone wie Aceton, Diethylketon, Methylbutylketone; Carbonsäureamide wie Dimethylformamid, Dimethylacetamid, Tetramethylharnstoff, Hexamethylphosphorsäuretriamid, N-Methylpyrrolidon, Dimethylsulfoxid; heterocyclische Basen wie Pyridin, Chinolin oder Picoline, ferner handelsübliche technische Gemische dieser Lösungsmittel.

Die Umsetzung kann in einem Überschuß der eingesetzten Verbindungen der allgemeinen Formeln (II) und/oder (III) und gegebenenfalls in Gegenwart eines säurebindenden Mittels, z.B. eines Alkoholats wie Kalium-tert.-butylat oder Natriummethylat, eines Alkalihydroxids wie Natrium- oder Kaliumhydroxid, eines Erdalkalihydroxids wie Calcium- oder Bariumhydroxid, eines Alkali- oder Erdalkalicarbonats wie Natriumcarbonat, Kaliumcarbonat, Calciumcarbonat, Natrium- oder Kaliumhydrogencarbonat, eines Alkaliamids wie Natrium- oder Kaliumamid, eines Alkalihydrids wie Natriumhydrid, tertiärer organischer Basen wie Triethylamin, N,N-Dimethylamin, Pyridine, Chinoline oder Isochinoline, oder eines Reaktionsbeschleunigers wie Kaliumjodid, je nach der Reaktionsfähigkeit des nukleophil austrauschbaren Restes zweckmäßigerweise bei Temperaturen zwischen 0 und 150°C, vorzugsweise bei Temperaturen zwischen 0 und 120°C, z.B. bei der Siedetemperatur des verwendeten Lösungsmittels, durchgeführt werden. Die Umsetzung kann jedoch auch ohne Lösungsmittel durchgeführt werden. Besonders vorteilhaft wird die Umsetzung jedoch in Gegenwart von Natriumhydrid oder Kaliumcarbonat durchgeführt.

Die Umsetzung wird normalerweise bei Atmosphärendruck durchgeführt.

Bei der Durchführung des erfindungsgemäßen Verfahrens setzt man auf 1 Mol der Verbindung der Formel (III) mindestens 1 Mol des Amins der Formel (II) und mindestens 1 Mol eines der genannten säurebindenden Mittel ein.

Zweckmäßigerweise kann die Umsetzung auch unter Inertgas, wie z.B. Stickstoff oder Argon, durchgeführt werden.

Die Aufarbeitung erfolgt zweckmäßig durch Eindampfen der Reaktionslösung, Aufnehmen des Konzentrats in einem geeigneten inerten organischen Lösungsmittel, gegebenenfalls Alkalisieren mit einer Base, z.B. Ammoniak, Natriumcarbonat oder Kaliumhydrogencarbonat, und Reinigen, gegebenenfalls mit Hilfe der Chromatographie an Kieselgel bzw. Aluminiumoxid oder anderen geeigneten Adsorbentien.

Verbindungen der allgemeinen Formel (I), in denen der Substituent $R^4$ am Stickstoffatom für Wasserstoff steht, lassen sich nach bekannten Methoden nachträglich in entsprechend substituierte Verbindungen überführen. Eine solche nachträgliche Substitution erfolgt vorzugsweise, indem man z.B. die NH-Verbindung mit Natrium, Kalium, Natriumamid, Kaliumamid, Kaliumhydroxid, Lithiumhydroxid,

Natriumhydrid oder Alkalialkoholat, wie beispielsweise Natriummethylat oder Kaliummethylat, vorzugsweise mit Natriumhybrid, in das Alkalisalz (I, $R^4$ = Na, K oder Li) umwandelt und dieses in an sich bekannter Weise mit den entsprechend substituierten Halogeniden, wie z.B. Alkylhalogeniden, vorzugsweise Methyljodid, Ethyljodid, Propylbromid, Isopropylchlorid, n-Butylbromid, insbesondere Methyljodid, umsetzt.

Die N-Alkylierung der Amidgruppe in Verbindungen der allgemeinen Formel (I) mit $R^4$ = H kann gegebenenfalls bei ausreichender Basizität dieser Gruppe auch direkt in Gegenwart geeigneter Protonenakzeptoren wie Trimethylamin, Triethylamin, N-Methylpiperidin, N,N-Dimethylanilin, N,N-Diethylanilin, heterocyclische Basen, wie Pyridin, Picoline, Kollidine, Chinolin oder Isochinolin durchgeführt werden.

Die Umsetzung kann ohne Lösemittel oder aber in Gegenwart geeigneter Lösungsvermittler durchgeführt werden. Als Lösungsvermittler kommen alle organischen Lösemittel in Frage, die gegenüber den jeweiligen Reaktionspartnern inert sind. Hierzu gehören vorzugsweise aromatische Kohlenwasserstoffe wie Benzol, Toluol, Xylol oder Tetralin, Ether wie Diethylether, Diisopropylether, Tetrahydrofuran, Dioxan, Ethylenglykoldiethylether, Nitrile wie Acetonitril, Propionitril, Carbonsäureamide wie Dimethylformamid, Dimethylacetamid, Hexamethylphosphorsäuretriamid, N-Methylpyrrolidon, Dimethylsulfoxid, heterocyclische Basen wie Pyridin, Chinolin oder Picoline, ferner handelsübliche technische Gemische dieser Lösemittel.

Die Umsetzung kann bei Normaldruck aber auch bei erhöhtem Druck durchgeführt werden, insbesondere bei niedrig siedenden Alkylhalogeniden als Reaktionspartner kann erhöhter Druck für die Umsetzung erforderlich sein.

Die Reaktionstemperaturen können in einem gewissen Bereich variiert werden. Im allgemeinen arbeitet man bei Temperaturen zwischen 0 und 200°C, vorzugsweise zwischen 20 und 150°C, insbesondere zwischen 40 und 80°C; in einzelnen Fällen genügt Raumtemperatur.

Die Aufarbeitung erfolgt dann wieder analog wie oben beschrieben.

Die als Vorprodukte einzusetzenden Piperazinderivate der allgemeinen Formel (II) sind nur teilweise bekannt und können vorzugsweise nach an sich bekannten Methoden beispielhaft entweder nach dem Reaktionsschema (*1*) hergestellt werden:

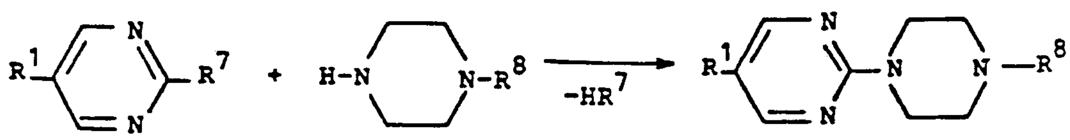


(X) (XI) (XII)

indem man Pyrimidinderivate der allgemeinen Formel (X), in welcher

$R^1$ die in der allgemeinen Formel (I) angegebene Bedeutung hat, und

$R^7$ für Halogen, insbesondere für Chlor, Brom oder Fluor, besonders bevorzugt für Chlor und Fluor, steht,

mit bekannten Piperazinderivaten der allgemeinen Formel (XI), in welcher

$R^8$ für Wasserstoff, Formyl, Alkanoyl, vorzugsweise Acetyl, Propionyl, Isopropionyl, Butyryl oder Benzoyl, Alkoxycarbonyl, vorzugsweise Methoxycarbonyl, Ethoxycarbonyl, Propoxycarbonyl oder Phenoxycarbonyl, vorzugsweise Methoxycarbonyl oder Ethoxycarbonyl, oder andere geeignete, hydrolytisch leicht abspaltbare Schutzgruppen steht,

in einem geeigneten Lösungsmittel wie Alkohole, insbesondere Ethanol, Propanol oder Butanol; Kohlenwasserstoffe wie Benzol, Toluol, Xylol, Carbonsäureamide wie Dimethylformamid, N-Methylpyrrolidon, Dimethylsulfoxid oder Ether wie Dioxan oder Tetrahydrofuran nach bekannten literaturbeschriebenen Methoden (vgl. K. L. Howard et al., J. Org. Chem. *18*, 1484 (1953)) bei Temperaturen zwischen 50 bis 150°C, vorzugsweise zwischen 70 und 120°C, umsetzt, vorteilhaft in Gegenwart eines säurebindenden Mittels, wie z.B. eines Alkalicarbonats wie Kaliumcarbonat oder einer tertiären organischen Base wie Triethylamin oder Pyridin;

oder nach dem Reaktionsschema (*2*):

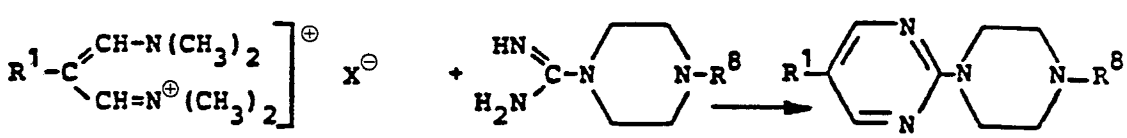

(XIII) (XIV) (XII)

indem man Verbindungen der allgemeinen Formel (XIII), in welcher

$R^1$ die oben angegebene Bedeutung hat und

$X^{\ominus}$ für Chlorid, Bromid, Sulfat, Hydrogensulfat, Perchlorat, vorzugsweise Perchlorat, steht,

mit den Piperaziderivaten der allgemeinen Formel (XIV), in welcher

$R^8$ die oben angegebene Bedeutung hat, vorzugsweise für Methoxycarbonyl oder Ethoxycarbonyl steht, nach an sich bekannter Methode (vgl. R. M. Wagner und C. Jutz, Chem. Ber. *104*, 2975 (und G. M. Coppola et al., J. Heterocycl. Chem. *11*, 51 (1974)),
in geeigneten Lösungsmitteln, wie Alkohole, insbesondere Methanol, Ethanol, Propanol, Isopropanol oder Butanol, oder Alkohol-Wasser-Gemischen bei Siedetemperatur des betreffenden Lösungsmittels umsetzt und in bekannter Weise aufarbeitet.

Die sich anschließende hydrolytische Abspaltung der Gruppe $R^8$ nach bekannten Verfahren führt zu den Verbindungen der Formel (II).

Die Verbindungen der allgemeinen Formeln (X) und (XI) sind bekannt oder werden nach an sich bekannten Verfahren erhalten.

Die Verbindungen der allgemeinen Formeln (XIII) und (XIV) sind ebenfalls bekannt oder können nach an sich bekannten Verfahren erhalten werden (vgl. z.B. J. Org. Chem. *13*, 144 (1948)).

Der dem Reaktionsschema (*2*) zugrundeliegende Reaktionsschritt, nach den Verbindungen der allgemeinen Formel (XIII) mit Piperazinderivaten der allgemeinen Formel (XIV), wobei $R^8$ die oben angegebene Bedeutung hat und besonders bevorzugt für Ethoxycarbonyl steht, zu Verbindungen der allgemeinen Formel (XII), ist ebenfalls Gegenstand der Erfindung.

Die gegebenenfalls anschließende Abspaltung der verwendeten Schutzgruppen $R^8$, wobei $R^8$ nicht für Wasserstoff steht, zu den Verbindungen der Formel (II) erfolgt vorzugsweise hydrolytisch in einem wäßrigen Lösungsmittel, z.B. in Wasser, Methanol/Wasser, Ethanol/Wasser, Isopropanol/Wasser oder Dioxan/Wasser in Gegenwart einer Säure wie Salzsäure oder Schwefelsäure oder vorzugsweise in Gegenwart einer Alkalibase wie Lithiumhydroxid, Natriumhydroxid oder Kaliumhydroxid bei Temperaturen zwischen 20 und 100°C, vorzugsweise bei der Siedetemperatur des Reaktionsgemisches. Die Reaktionszeit liegt zwischen 1 bis 24 Stunden, vorzugsweise zwischen 3 bis 12 Stunden.

Die als Vorprodukte einzusetzenden Verbindungen der allgemeinen Formel (III) werden nach an sich bekannten Methoden (vgl. z.B. H. B. Donahoe et al., J. Org. Chem. *22*, 68 (1957)) nach folgendem Reaktionsschema hergestellt:

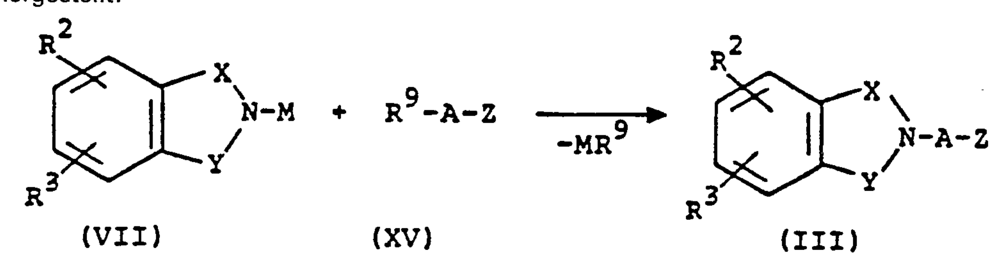


(VII) (XV) (III)

indem man geeignete Verbindungen der allgemeinen Formel (VII), in welcher
$R^2$, $R^3$, X, Y und M die oben angegebene Bedeutung haben,
mit Verbindungen der allgemeinen Formel (XV), in welcher
Z die oben angegebene Bedeutung hat und bevorzugt, für Chlor und Brom, besonders bevorzugt für Brom, steht
A die oben angegebene Bedeutung hat und besonders bevorzugt für Ethylen, n-Propylen, n-Butylen, 2-Hydroxy-n-propylen oder 2-Methyl-n-propylen steht, und
$R^9$ Halogen, bevorzugt Chlor oder Brom, besonders bevorzugt Brom, bedeutet,
unter Reaktionsbedingungen umsetzt, wie sie in der einschlägigen Literatur beschrieben sind.

Für den Fall

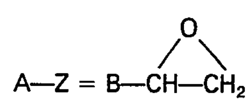

setzt man die Verbindungen der allgemeinen Formel (VII) mit Epichlorhydrin um.

Die Ausgangsprodukte werden in einem geeigneten, organischen Lösungsmittel bei Temperaturen zwischen 0 und 150°C, vorzugsweise bei Temperaturen zwischen 40 und 120°C in Gegenwart eines säurebindenden Mittels umgesetzt. Als organische Lösungsmittel sind beispielsweise Acetonitril, n-Butanol, Benzol, Toluol, Xylol, Chlorbenzol, Dioxan, Tetrahydrofuran, ganz besonders Dimethylformamid oder Dimethylsulfoxid, bevorzugt. Das bevorzugte säurebindende Mittel ist Kaliumcarbonat oder Natriumhydrid, es können jedoch auch andere anorganische oder tertiäre organische Basen verwendet werden, wie Natrium- und Kaliumhydroxid, Carbonate, Natrium- und Kaliumhydrogencarbonat oder tertiäre Amine, wie Pyridin, Chinoline oder Isochinoline.

Die intermediär entstehenden Salze der Formel (VII) mit beispielsweise M = Natrium, Kalium oder Lithium können in Substanz isoliert werden; zweckmäßigerweise werden die Salze ohne vorherige Isolierung in der Reaktionsmischung sofort weiter umgesetzt.

Die Herstellung der Ausgangssubstanzen der allgemeinen Formel (VII) wird in zahlreichen Literaturbeispielen beschrieben (vgl. z.B. H. Hettler, Advances in Heterocyclic Chemistry *15*, 234 ff (1973)).

Die vorzugsweise eingesetzten Verbindungen der allgemeinen Formel (XV) sind gleichfalls literaturbekannt.

b) Umsetzung einer Verbindung der allgemeinen Formel (IV) mit einer Verbindung der allgemeinen Formel (V) nach folgendem Reaktionsschema:

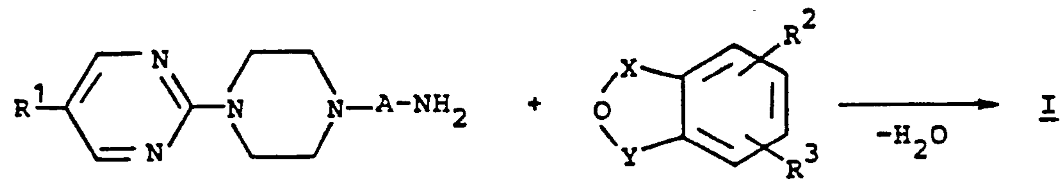


(IV) (V)

in denen

A, X, Y, $R^1$, $R^2$ und $R^3$ die oben angegebene Bedeutung haben.

Vorzugsweise wird die Umsetzung bei höheren Temperaturen in inerten organischen Lösungsmitteln oder ohne Lösungsmittel in der Schmelze der Reaktionskomponenten unter gleichzeitigem Abdestillieren des Reaktionswassers durchgeführt. Das Verfahren wird vorzugsweise durchgeführt bei Temperaturen zwischen 100 und 250°C, insbesondere in einem Bereich zwischen 115 und 170°C. Die Verwendung von Intergasatmosphäre ist vorteilhaft. Als Lösungsmittel für diese Reaktion seien beispielhaft genannt: Tetralin, Dichlorbenzol, Xylol, N-Methylpyrrolidon, Pyridin, Diethylenglykoldimethylether, wobei Pyridin besonders bevorzugt genannt sei. Die Reaktionszeiten betragen zwischen 1 und 16 Stunden, vorzugsweise zwischen 2 und 6 Stunden.

Bei der Durchführung des Verfahrens setzt man auf 1 Mol der Verbindung (IV) mindestens 1 Mol der Verbindung (V) ein.

Die Aufarbeitung erfolgt zweckmäßig in der gleichen Weise wie bei der Verfahrensvariante a) beschrieben.

Die als Vorprodukte einzusetzenden ω-Aminoalkylpiperazine der allgemeinen Formel (IV) können nach an sich bekannten Methoden, z.B. nach dem folgenden Reaktionsschema hergestellt werden:

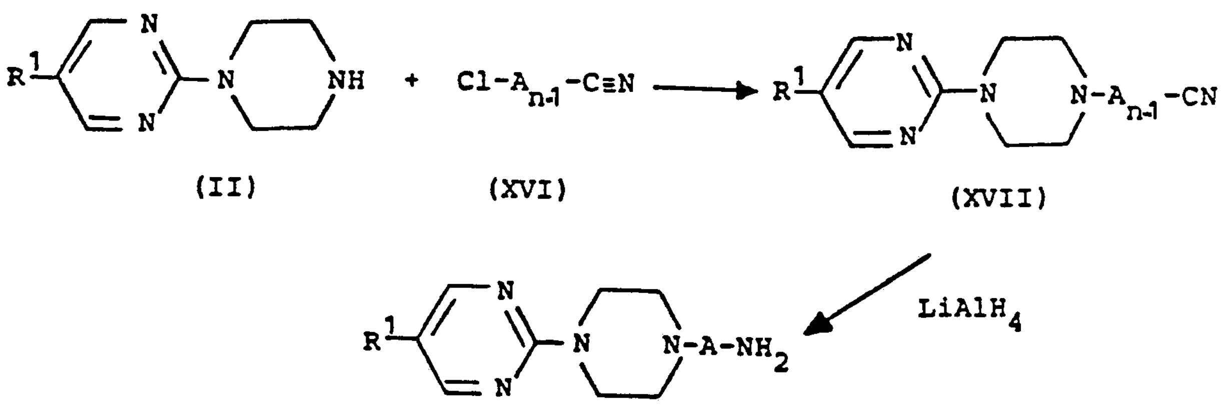


(IV)

indem man Verbindungen der allgemeinen Formel (II) mit bekannten Halogenalkylnitrilen der allgemeinen Formel (XVI), in welcher

n die Zahl der Methylengruppen in der Alkylenkette bedeutet und

A die oben angegebene Bedeutung hat, wobei die Gruppierung $—CH_2—CH(OH)—CH_2—$ ausgeklammert ist,

alkyliert und die erhaltenen Cyanverbindungen der allgemeinen Formel (XVII) zu den substituierten Piperazinen (IV) reduziert. Die Reduktion erfolgt vorzugsweise in Diethylether, Dioxan oder Tetrahydrofuran mit metallorganischen Verbindungen, vorzugsweise mit Lithiumaluminiumhydrid. Man arbeitet vorzugsweise bei den Siedetemperaturen der o.g. Lösungsmittel. Die angewandten Arbeitsweisen sind beispielhaft beschrieben in: X. -H. Wu et al., J. Med. Chem. *12*, 876 (1969) und Y. -H. Wu, J. Med. Chem. *15*, 477 (1972).

Die Verbindungen der allgemeinen Formel (IV) können nach bekannten Methoden hergestellt werden, beispielsweise nach folgendem Reaktionsschema:

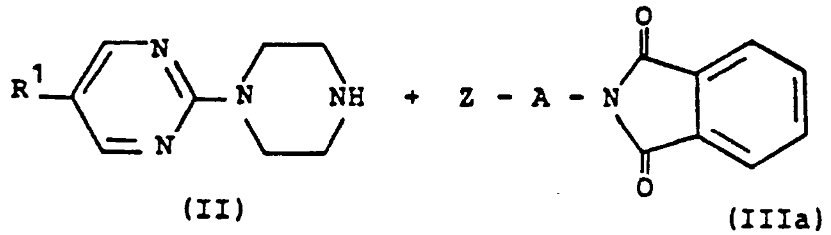

(II) (IIIa)

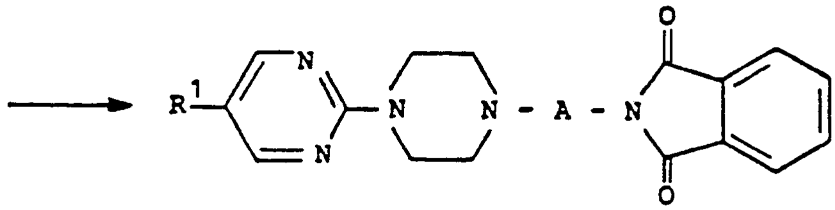

(Ib)

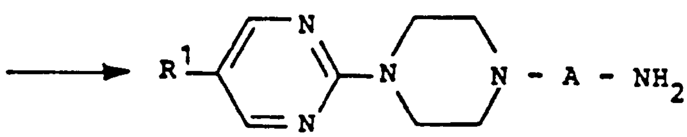

(IV)

Pyrimidylpiperazine der allgemeinen Formel (II), in welcher $R^1$ die oben angegebene Bedeutung hat, werden mit Verbindungen der allgemeinen Formel (IIIa), in welcher

A die oben angegebene Bedeutung hat und besonders bevorzugt für Ethylen, Propylen oder Butylen steht, und

Z die oben angegebene Bedeutung hat und bevorzugt für Chlor oder Brom, besonders bevorzugt für Brom, steht,

zunächst in der unter Verfahren a) ausführlich beschriebenen Weise zu Verbindungen der allgemeinen Formel (Ib) umgesetzt. Die Herstellung der Verbindungen der Formel (II) wird oben ausführlich beschrieben; die Verbindungen der Formel (IIIa) sind bekannt und sind Kaufprodukte; die Verbindungen der allgemeinen Formel (Ib) sind neu und stellen gleichzeitig spezielle Beispele der erfindungsgemäßen Verbindungen der allgemeinen Formel (I) dar.

Die Umwandlung der Verbindungen der Formel (Ib) in die neuen ω-Aminoalkylpiperazin-Derivate der allgemeinen Formel (IV) kann erfolgen, indem man nach an sich bekannter Weise eine Phthalimidspaltung durch Erhitzen der Verbindungen der Formel (Ib) in Mineralsäuren, vorzugsweise in konzentrierter Salzsäure, oder durch Erhitzen mit Hydrazinhydrat in wasserhaltigen Alkoholen, vorzugsweise in wasserhaltigem Methanol, Ethanol, Propanol oder Isopropanol, durchführt. Die Aufarbeitung der Reaktionsansätze erfolgt in der üblichen Weise (vgl. J. W. Griffin und D. H. Hey, J. Chem. Soc. *1952*, 3334).

Die Vorprodukte der allgemeinen Formel (V), in welcher

$R^2$, $R^3$, X und Y die oben angegebene Bedeutung haben, wobei vorzugsweise $R^2$ und $R^3$ gleich oder verschieden sind und für Wasserstoff oder Chlor stehen,

X und Y gleich sind und für Carbonyl stehen oder

X für Carbonyl steht, wenn Y die Gruppe $—CO—CH_2—$ bedeutet,

können nach bekannten Verfahren hergestellt werden.

c) Umsetzung einer Verbindung der allgemeinen Formel (XVIII) mit einer Verbindung der allgemeinen Formel (VII) nach folgendem Reaktionsschema:

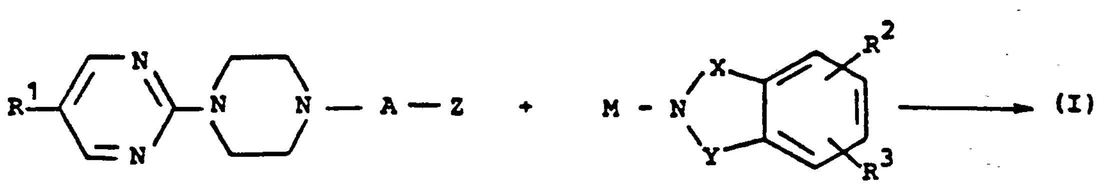

(XVIII) (VII)

in denen

A, M, X, Y, Z, $R^1$, $R^2$ und $R^3$ die oben angegebene Bedeutung haben.

Die Umsetzung wird gegebenenfalls in einem Lösungsmittel oder Lösungsmittelgemisch, z.B. in Aceton, Diethylketon, Acetonitril, Benzol, Toluol, Xylol, Dioxan, Tetrahydrofuran, Chlorbenzol, Tetramethylharnstoff, Dimethylformamid, Dimethylsulfoxid, in Alkoholen, vorzugsweise in Propanol, Butanol oder in Gemischen dieser Alkohole mit Dimethylformamid und zweckmäßigerweise je nach der Reaktionsfähigkeit des Restes Z bei Temperaturen zwischen 0 und 150°C, vorzugsweise jedoch bei der Siedetemperatur des verwendeten Lösungsmittels, durchgeführt. Die Reaktionsdauer kann 1 bis 48 Stunden, vorzugsweise 2 bis 20 Stunden, betragen, je nach Reaktionsbereitschaft des Restes Z in (XVIII).

Vorteilhaft ist die Gegenwart eines säurebindenden Mittels, wie z.B. eines Alkoholats wie Natrium- oder Kaliummethylat oder Natrium- oder Kaliumethylat, eines Alkalkihydroxids wie Natrium-, Kalium- oder Lithiumhydroxid, eines Alkalicarbonats wie Kalium- oder Natriumcarbonat, Kalium- oder Natriumhydrogencarbonat, eines Alkaliamids wie Natrium- oder Kaliumamid, eines Alkalihydrids wie Natriumhydrid oder einer tertiären organischen Base wie Triethylamin, Pyridin oder Chinolin, oder eines Reaktionsbeschleunigers wie beispielsweise Kaliumjodid.

Die als Vorprodukte eingesetzten Verbindungen der allgemeinen Formel (XVIII) können nach an sich bekannten Methoden, z.B. nach folgendem Reaktionsschema hergestellt werden:

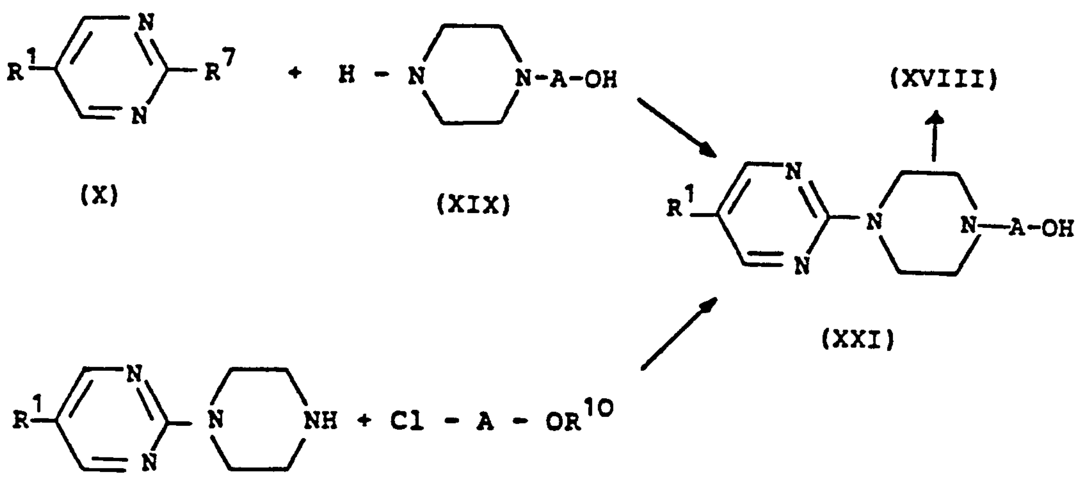

(X) (XIX) (XVIII) (XXI) (II) (XX)

indem man entweder Verbindungen der allgemeinen Formel (X), in welcher

$R^1$ die oben angegebene Bedeutung hat, im vorliegenden Falle bevorzugt für Wasserstoff oder Phenyl steht, ganz besonders bevorzugt für Wasserstoff, steht,

$R^7$ Halogen, besonders bevorzugt Chlor, bedeutet,

mit bekannten oder käuflichen Hydroxyalkylpiperazinen der allgemeinen Formel (XIX), in welcher

A die oben angegebene Bedeutung hat, im vorliegenden Fall besonders bevorzugt für n-Propylen steht, oder

indem man Verbindungen der allgemeinen Formel (II), in welcher

$R^1$ die oben angegebene Bedeutung hat, im vorliegenden Fall bevorzugt für Wasserstoff oder Phenyl steht, ganz besonders bevorzugt für Wasserstoff steht,

mit bekannten oder käuflichen Verbindungen der allgemeinen Formel (XX), in welcher

A die ben angegebene Bedeutung hat, bevorzugt für Ethylen, Propylen oder Butylen, ganz besonders für Butylen, steht, und

$R^{10}$ Wasserstoff oder Acetyl bedeutet, im vorliegenden Fall besonders für Acetyl steht,

unter bekannten Alkylierungsbedingungen zu Verbindungen der allgemeinen Formel (XXI) umsetzt, vorzugsweise ohne Lösungsmittel oder in inerten Lösungsmitteln, wie beispielsweise Aceton, Acetonitril, Benzol, Toluol, Xylol, Dioxan, Tetrahydrofuran, Chlorbenzol, Dimethylformamid oder Dimethylsulfoxid bei Temperaturen zwischen 0 und 150°C, vorzugsweise bei Temperaturen zwischen 40 und 120°C.

Vorteilhaft ist die Gegenwart eines säurebindenden Mittels, wie z.B. Kaliumcarbonat oder eine tertiäre organische Base, wie z.B. Triethylamin oder Pyridin. Die Aufarbeitung der Ansätze erfolgt in bekannter Weise.

Das Zwischenprodukt der allgemeinen Formel (XXI) wird nach geläufigen herkömmlichen Arbeitweisen aktiviert um das Zwischenprodukt (XVIII) zu bilden. Beispielsweise werden bei der Einwirkung von Thionylchlorid auf die Verbindungen der Formel (XXI) die Zwischenprodukte der Formel (XVIII) gebildet, worin Z Chlor bedeutet.

In ähnlicher Weise werden Bromide und Jodide erhalten; Tosylate und Mesylate entsprechend der Formel (XVIII) werden durch herkömmliche Laboratoriumsmethoden erhalten.

d) Umsetzung einer Verbindung der allgemeinen Formel (VI-a) mit einer Verbindung der allgemeinen Formel (VII-a) nach folgendem Reaktionsschema

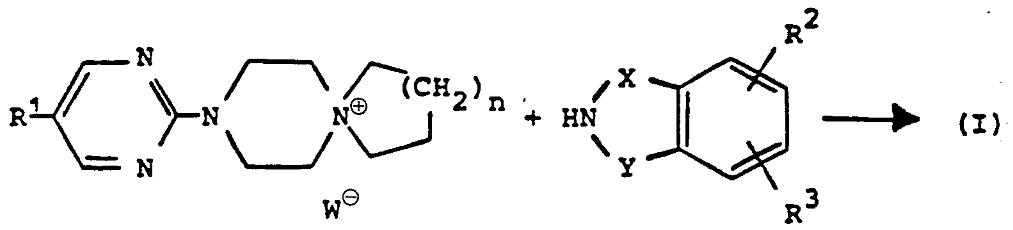

(VI-a) (VII-a)

in denen

A, W, X, Y, $R^1$, $R^2$ und $R^3$ die oben angegebene Bedeutung haben.

Die Umsetzung wird gegebenenfalls in einem Lösungsmittel z.B. Benzol, Toluol, Xylol, Chlorbenzol, Acetonitril, Tetramethylharnstoff, Dimethylformamid, Dimethylsulfoxid, Dibutylether, in Alkoholen, vorzugsweise in Propanol, n-Butanol oder Amylalkohol, besonders vorzugsweise in n-Butanol oder Dimethylformamid bei Temperaturen zwischen 20 und 180°C, vorzugsweise zwischen 80 und 160°C, besonders vorzugsweise bei der Siedetemperatur des verwendeten Lösungsmittels, durchgeführt. Vorteilhaft ist die Gegenwart eines säurebindenden Mittels, wie z.B. eines Alkoholats wie Natriummethylat oder Natriumethylat, eines Alkalihydroxids wie Natriumhydroxid oder Kalimhydroxid, eines Alkalicarbonats wie Kaliumcarbonat, eines Alkaliamids wie Natriumamid oder Kaliumamid, eines Alkalihydrids wie Natriumhydrid oder einer tertiären organischen Base wie Triethylamin oder Pyridin.

Bevorzugt werden molare Mengen der Verbindungen der allgemeinen Formel (VI-a), in welcher

$R^1$ Wasserstoff und

W Chlor oder Brom bedeutet und

n für 1 oder 2 stehen kann,

mit Verbindungen der allgemeinen Formel (VII-a), in welcher

$R^2$ und $R^3$ die oben angegebene Bedeutung haben und

X und Y für Carbonyl oder Sulfonyl stehen,

in Dimethylformamid oder n-Butanol bei Seidetemperatur der angegebenen Lösungsmittel in Gegenwart molarer Mengen Kaliumcarbonat umsetzt. Die Reaktionsdauer kann 2 bis 24 Stunden, vorzugsweise 4 bis 12 Stunden, betragen. Die Reaktion ist Stand der Technik und wird in der britischen Patentanmeldung GB 2 085 436 A beschrieben. Die oben vorzugsweise genannten Verbindungen der allgemeinen Formel (VI-a) sind bekannt; ihre Darstellung wird in der obengenannten Anmeldung beschrieben.

Die vorliegende Erfindung betrifft weiterhin ein Verfahren zur Herstellung von Verbindungen der allgemeinen Formel (Ia), in welcher

A, X, $R^1$, $R^2$ und $R^3$ die oben angegebene Bedeutung haben und

Y für —CO—N($R^4$)— steht, wobei $R^4$ vorzugsweise für Wasserstoff oder Methyl steht,

durch

e) Umsetzung einer Verbindung der allgemeinen Formel (VIII), in welcher

$R^1$, $R^2$, $R^3$, $R^4$, A und X die oben angegebene Bedeutung haben, wobei besonders $R^1$ und $R^2$ vorzugsweise für Wasserstoff, $R^4$ vorzugsweise für Wasserstoff oder Methyl steht und A insbesondere Ethylen, Propylen oder Butylen bedeutet, mit einer Verbindung der allgemeinen Formel (IX), in welcher

$R^5$ und $R^6$ die oben angegebene Bedeutung haben, wobei besonders $R^5$ und $R^6$ gleich sein können und für Chlor oder für eine Imidazolyl-(2)-Gruppe stehen oder $R^5$ Chlor bedeutet und $R^6$ für Ethoxy oder Phenoxy steht,

nach dem folgenden Reaktionsschema:

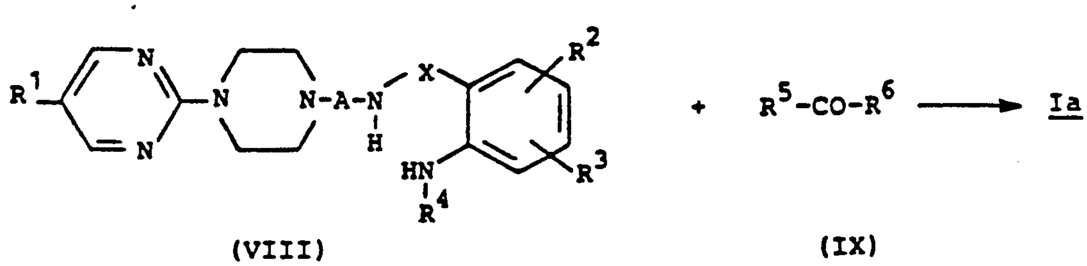

(VIII) (IX)

Die Umsetzung wird zweckmäßigerweise in einem Lösungsmittel wie beispielsweise Methylenchlorid, Benzol, Toluol, Xylol, Chlorbenzol, Dioxan, Tetrahydrofuran oder Acetonitril, vorzugsweise in Toluol oder Chlorbenzol, zweckmäßigerweise bei Temperaturen zwischen 0 und 150°C, vorzugsweise bei der Siedetemperatur zwischen 40 und 130°C, durchgeführt. Die Umsetzung kann vorteilhaft in Gegenwart geeigneter Protonenakzeptoren wie Trimethylamin, Triethylamin, N,N-Dimethylanilin, heterocyclischer Basen wie Pyridin, Picoline, Kollidine, Chinoline oder Isochinolin vorgenommen werden; vorzugsweise wird Triethylamin verwendet.

Bei der Durchführung des erfindungsgemäßen Vefahrens setzt man vorteilhaft auf 1 Mol der Verbindung (VIII) die Verbindung (IX) in einem Überschuß von 0,1 bis 2 Mol ein.

Die Aufarbeitung erfolgt zweckmäßig durch Eindampfen der Reaktionslösung, Aufnehmen des Konzentrats in einem geeigneten Lösungsmittel, z.B. Methylenchlorid, Alkalisieren mit einer geeigneten Base, z.B. Ammoniak-Lösung, und Reinigen, gegebenenfalls mit Hilfe der Chromatographie an Kieselgel oder Aluminiumoxid oder anderen geeigneten Adsorbentien.

Die als Vorprodukte einzusetzenden Verbindungen der allgemeinen Formel (VIII)

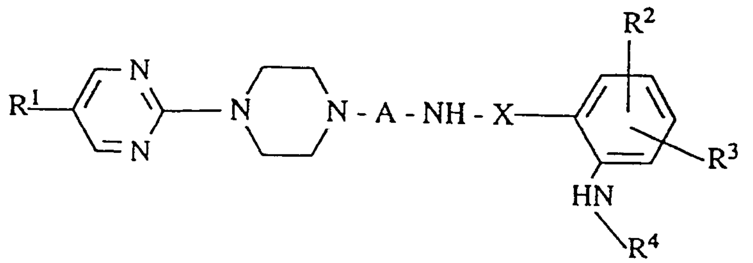

(VIII)

in welcher

$R^2$, $R^3$ und X die oben angegebene Bedeutung haben, und für

$R^1$, $R^4$ und A für die Reste stehen, die oben vorzugsweise genannt wurden,

sind neu und ebenfalls Bestandteil der Erfindung. Sie lassen sich herstellen, indem man die neuen und ebenfalls zum Bestandteil der Erfindung zählenden Nitroverbindungen der allgemeinen Formel (XXII)

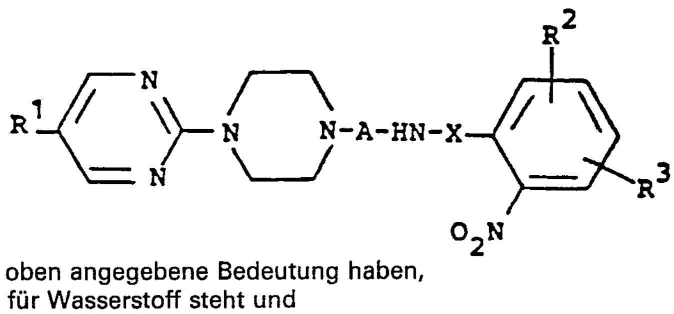

(XXII)

in welcher

$R^2$, $R^3$ und X die oben angegebene Bedeutung haben,

$R^1$ vorzugsweise für Wasserstoff steht und

A vorzugsweise für Ethylen, Propylen oder Butylen steht,

mit geeigneten Reduktionsmitteln zu den Aminoverbindungen der allgemeinen Formel (VIII) (mit $R^4$ = H) reduziert.

Zur Darstellung von (VIII) löst man die Nitroverbindung der Formel (XXII) in einem geeigneten Lösungsmittel, beispeilsweise in Alkoholen, vorzugsweise in Methanol oder Ethanol, fügt überschüssiges Hydrazinhydrat in einem Molverhältnis 1:5, vorzugsweise in einem Molverhaltnis 1:3, und einen Hydrierkatalysator, beispielsweise Palladium, Palladiumkohle oder Raney-Nickel, vorzugsweise Palladiumkohle, hinzu und erhitzt die Mischung 0,5 bis 5 Stunden auf 30 bis 100°C vorzugsweise 0,5 bis 2 Stunden auf 65 bis 80°C.

Die Aufarbeitung der Reaktionsansätze erfolgt durchweg in allgemein bekannter Art und Weise.

Die obengenannten neuen Nitroverbindungen der allgemeinen Formel (XXII) lassen sich in allgemein üblicher und bekannter Weise herstellen, indem man die neuen und zum Bestandteil der Erfindung zählenden Amine der allgemeinen Formel (IV)

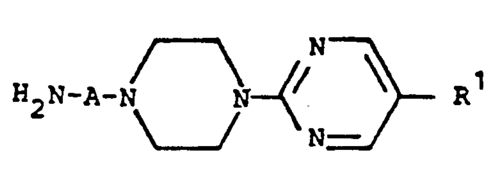

(IV)

in welcher

$R^1$ vorzugsweise Wasserstoff und

A vorzugsweise Ethylen, Propylen oder Butylen bedeutet,

mit bekannten Verbindungen der allgemeinen Formel (XXIII)

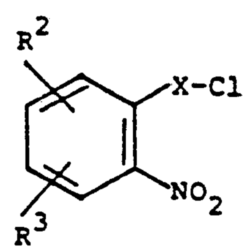

(XXIII)

in welcher
$R^2$, $R^3$ und X die oben angegebene Bedeutung, haben,
acyliert.

Die Reaktion erfolgt in einem geeigneten Lösungsmittel, vorzugsweise in Toluol, in Gegenwart eines säurebindenden Mittels, beispielsweise mit Triethylamin, Pyridin oder 10 bis 20 %iger Natron- oder Kalilauge. Die Aufarbeitung erfolgt in üblicher Art und Weise.

Die obengenannten neuen, zum Bestandteil der Erfindung gehörenden Verbindungen der allgemeinen Formel (VIII),

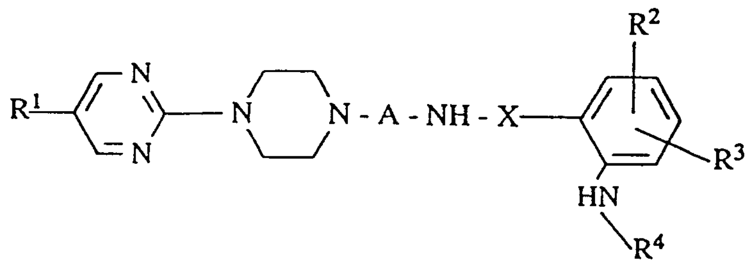

(VIII)

in welcher
$R^2$ und $R^3$ die oben angegebene Bedeutung haben,
$R^1$ vorzugsweise für Wasserstoff steht und
$R^4$ vorzugsweise Wasserstoff oder Methyl bedeutet, lassen sich für den Fall, daß
X Carbonyl bedeutet,
bevorzugt aus den leicht zugänglichen Isatosäureanhydriden der allgemeinen Formel (XXIV)

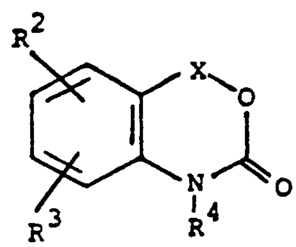

(XXIV)

in welcher
$R^2$ und $R^3$ die oben angegebene Bedeutung haben,
$R^4$ vorzugsweise für Wasserstoff oder Methyl, und
X für Carbonyl steht,
durch Umsetzung mit den Aminen der allgemeinen Formel (IV)

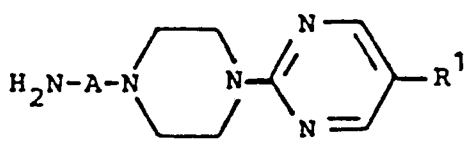

(IV)

in welcher
$R^1$ vorzugsweise Wasserstoff und
A vorzugsweise Ethylen, Propylen oder Butylen bedeutet,
herstellen. Die Reaktion ist beschrieben von T. Kappe und W. Stadlbauer in: Advances in Heterocyclic Chemistry (Ed.: A. R. Katritzky), Vol. 28, S. 127—182, Academic Press New York, 1981.

Von den oben beschriebenen Zwischenprodukten sind die Pyrimidinylpiperazin-Derivate der allgemeinen Formeln (II), (IV) und (XII) gleichfalls Gegenstand der vorliegenden Erfindung:

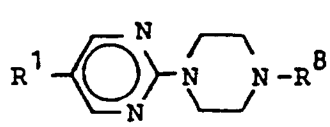

(XII)

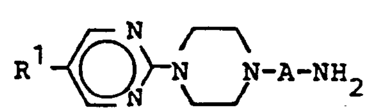

(IV)

Formel (II) entspricht Formel (XII), wenn $R^8$ Wasserstoff bedeutet.

Die obengenannten Verbindungen der allgemeinen Formeln (II), (IV) und (XII) besitzen hervorragende zentralnervöse Wirkungen, insbesondere antidepressive und anxiolytische Wirkungen; diese Verwendung in entsprechenden Arzneimitteln ist deshalb ebenfalls Bestandteil der Erfindung.

Von ganz besonderer Bedeutung sind Verbindungen der allgemeinen Formeln (II), (IV) und (XII), in denen

$R^1$ für Trifluoromethyl, Chlor, Iod, Fluor, Phenyl, 3,4-Dimethoxyphenyl, 4-Methoxyphenyl, 4-Chlorphenyl, 3-(Trifluomethyl)phenyl, 2-Chlor-6-fluorphenyl, Indol-3-yl und

$R^8$ Methoxycarbonyl, Ethoxycarbonyl, Propoxycarbonyl, Isopropoxycarbonyl, Butoxycarbonyl oder Phenoxycarbonyl bedeutet,

A für n-Propylen oder N-Butylen steht,

wobei $R^1$ nicht für Fluor steht, wenn A für n-Butylen steht und wobei $R^1$ nicht für Fluor steht, wenn $R^8$ für Ethoxycarbonyl steht.

Als neue erfindungsgemäße Verbindungen der allgemeinen Formel (II) seien bevorzugt genannt:

1-(5-Jod-2-pyrimidinyl)piperazin
1-(5-Trifluoromethyl-2-pyrimidinyl)piperazin
1-(5-(3-Indolyl)-2-pyrimidinyl)piperazin
1-(5-(4-Chlorphenyl)-2-pyrimidinyl)piperazin
1-(5-(4-Methoxyphenyl)-2-pyrimidinyl)piperazin
1-(5-(3-Trifluormethylphenyl)-2-pyrimidinyl)piperazin
1-(5-(2-Fluor-6-chlorphenyl)-2-pyrimidinyl)piperazin

Als neue erfindungsgemäße Verbindungen der allgemeinen Formel (IV) seien bevorzugt genannt:

1-(4-Aminobutyl)-4-(5-phenyl-2-pyrimidinyl)piperazin
1-(3-Aminopropyl)-4-(5-phenyl-2-pyrimidinyl)piperazin
1-(4-Aminobutyl)-4-(5-(4-chlorphenyl)-2-pyrimidinyl)piperazin
1-(3-Aminopropyl)-4-(5-(4-chlorphenyl)-2-pyrimidinyl)piperazin
1-(4-Aminobutyl)-4-(5-(4-methoxyphenyl)-2-pyrimidinyl)piperazin
1-(3-Aminopropyl)-4-(5-(4-methoxyphenyl)-2-pyrimidinyl)piperazin
1-(4-Aminobutyl)-4-(5-(3,4-dimethoxyphenyl)-2-pyrimidinyl)piperazin
1-(3-Aminopropyl)-4-(5-(3,4-dimethoxyphenyl)-2-pyrimidinyl)piperazin
1-(4-Aminobutyl)-4-(5-(3-trifluoromethylphenyl)-2-pyrimidinyl)piperazin
1-(3-Aminopropyl)-4-(5-(3-trifluormethylphenyl)-2-pyrimidinyl)piperazin
1-(4-Aminobutyl)-4-(5-(2-fluor-6-chlorphenyl)-2-pyrimidinyl)piperazin
1-(3-Aminopropyl)-4-(5-(2-fluor-6-chlorphenyl)-2-pyrimidinyl)piperazin
1-(4-Aminobutyl)-4-(5-(3-indolyl)-2-pyrimidinyl)piperazin
1-(3-Aminopropyl)-4-(5-(3-indolyl)-2-pyrimidinyl)piperazin

Als neue erfindungsgemäße Verbindungen der allgemeinen Formel (XII) seien bevorzugt genannt:

Ethyl-4-(5-chlor-2-pyrimidinyl)-1-piperazincarboxylat
Ethyl-4-(5-jod-2-pyrimidinyl)-1-piperazincarboxylat
Ethyl-4-(5-trifluormethyl-2-pyrimidinyl)-1-piperazincarboxylat
Ethyl-4-(5-phenyl-2-pyrimidinyl)-1-piperazincarboxylat
Ethyl-4-(5-(4-chlorphenyl)-2-pyrimidinyl)-1-piperazincarboxylat
Ethyl-4-(5-(4-methoxyphenyl)-2-pyrimidinyl)-1-piperazincarboxylat
Ethyl-4-(5-(3,4-dimethoxyphenyl)-2-pyrimidinyl)-1-piperazincarboxylat
Ethyl-4-(5-(2-chlor-6-fluorphenyl)-2-pyrimidinyl)-1-piperazincarboxylat
Ethyl-4-(5-(3-trifluormethylphenyl)-2-pyrimidinyl)-1-piperazincarboxylat
Ethyl-4-(5-(3-indolyl)-2-pyrimidinyl)-1-piperazincarboxylat.

Gegenstand der vorliegenden Erfindung sind ebenfalls die beschriebenen Zwischenprodukte der allgemeinen Formel (VIII):

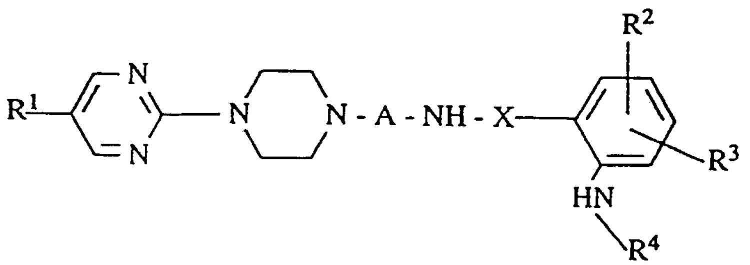

(VIII)

in welcher

A für eine Alkylengruppe mit 1 bis 6 Kohlenstoffatomen oder für 2-Hydroxy-n-propylen steht, wobei A nicht für die 2-Hydroxy-n-propylengruppe steht, wenn X und Y für Carbonyl und $R^1$, $R^2$ und $R^3$ für Wasserstoff stehen.

$R^1$ für Wasserstoff, Trifluormethyl oder für Alkoxy mit 1 bis 4 Kohlenstoffatomen, Trifluormethoxy oder für Hydroxy, Halogen, Cyano, Nitro, Amino, gegebenenfalls substituiert durch 1 oder 2 Methyl oder Ethyl, oder für Acetylamino oder Benzoylamino, oder für Indol-3-yl, oder für Phenyl steht, wobei der Phenylrest einfach oder mehrfach durch Methoxy, Ethoxy, Nitro, Halogen, Trifluormethyl; Amino, Cyano, Hydroxy, Methyl oder Ethyl, Acetylamino oder Benzoylamino substituiert sein kann,

$R^2$ und $R^3$ gleich oder verschieden sein können und für Wasserstoff oder Alkoxy mit 1 bis 4 Kohlenstoffatomen, oder für Phenyl oder für Hydroxy, Halogen, Cyano, Nitro, Amino, wobei das Stickstoffatom des Aminorestes durch 1 oder 2 Methyl oder Ethyl substituiert ist, oder für Methylsulfonamide, Benzol- und Toluolsulfonamido, Acetylamino, Ethoxycarbonylamino oder für Carboxyl, Methoxy- oder Ethoxycarbonyl, oder für Phenoxycarbonylamino, oder für Carbamoyl oder Sulfamoyl steht,

X für Carbonyl oder Sulfonyl steht, und

$R^4$ für Wasserstoff oder Phenyl steht,

und $NHR^4$ auch $NO_2$ bedeuten kann.

Die obengenannten Verbindungen der allgemeinen Formel (VIII) besitzen gute zentral-nervöse Wirkungen, insbesondere antidepressive und anxiolytische Wirkungen, ihre entsprechende Verwendung und ihre Verwendung in entsprechenden Arzneimitteln sind daher ebenfalls Bestandteil der Erfindung.

Von ganz besonderer Bedeutung sind Verbindungen der allgemeinen Formel (VIII), in welcher

$R^1$ Wasserstoff, Chlor, Fluor, Jod, Phenyl bedeutet,

$R^2$ und $R^3$ gleich oder verschieden sein können und für Wasserstoff und Chlor stehen,

$R^4$ Wasserstoff oder Methyl bedeutet,

A für n-Propylen oder n-Butylen steht,

X Carbonyl oder Sulfonyl bedeutet

und $NHR^4$ auch $NO_2$ bedeuten kann.

Als neue erfindungsgemäße Verbindungen der allgemeinen Formel (XII) seien bevorzugt genannt:

N-(3-(4-(2-Pyrimidinyl)-1-piperazinyl)propyl)-2-amino-benzoesäureamid
N-(4-(4-(2-Pyrimidinyl)-1-piperazinyl)butyl)-2-amino-benzoesäureamid
N-(3-(4-(2-Pyrimidinyl)-1-piperazinyl)propyl)-2-amino-5-chlor-benzoesäureamid
N-(4-(4-(2-Pyrimidinyl)-1-piperazinyl)butyl)-2-amino-5-chlor-benzoesäureamid
N-(3-(4-(2-Pyrimidinyl)-1-piperazinyl)propyl)-2-methyl-amino-benzoesäureamid
N-(4-(4-(2-Pyrimidinyl)-1-piperazinyl)butyl)-2-methyl-aminobenzoesäureamid
N-(4-(4-(2-Pyrimidinyl)-1-piperazinyl)butyl)-2-nitro-benzolsulfonsäureamid
N-(3-(4-(2-Pyrimidinyl)-1-piperazinyl)propyl)-2-nitro-benzolsulfonsäureamid
N-(4-(4-(2-Pyrimidinyl)-1-piperazinyl)butyl)-2-amino-benzolsulfonsäureamid
N-(3-(4-(2-Pyrimidinyl)-1-piperazinyl)propyl)-2-amino-benzolsulfonsäureamid

Ganz besonders bevorzugt bedeutet Y in Kombination mit den anderen angegebenen Resten Carbonyl, Sulfonyl, —CO—$CH_2$ oder —CO—N($R^4$), wobei die —CO-Gruppe jeweils mit dem Stickstoffatom benachbart ist und $R^4$ Wasserstoff, Phenyl oder Methyl bedeutet.

Als neue erfindungsgemäße Wirkstoffe seien im einzelnen genannt:

2-(4-(4-(2-Pyrimidinyl)-1-piperazinyl)butyl)5-fluor-1,2-benzisothiazol-3(2H)on-1,1-dioxid
2-(3-(4-(2-Pyrimidinyl)-1-piperazinyl)propyl)-1,2-benzisothiazol-3(2H)on-1,1-dioxid
2-(4-(4-(5-Fluor-2-pyrimidinyl)-1-piperazinyl)butyl)-1,2-benzisothiazol-3(2H)on-1,1-dioxid
2-(3-(4-(2-Pyrimidinyl)-1-piperazinyl)propyl)-5-fluor-1,2-benzisothiazol-3(2H)on-1,1-dioxid
2-(4-(4-(5-(3-Trifluormethylphenyl)-2-pyrimidinyl)-1-piperazinyl)butyl)-1,2-benzisothiazol-3(2H)on-1,1-dioxid
2-(4-(4-(5-Hydroxy-2-pyrimidinyl)-1-piperazinyl)butyl)-1,2-benzisothiazol-3(2H)on-1,1-dioxid
2-(4-(4-(5-Chlor-2-pyrimidinyl)-1-piperazinyl)butyl)-1,2-benzisothiazol-3(2H)on-1,1-dioxid
2-(3-(4-(5-Phenyl-2-pyrimidinyl)-1-piperazinyl)propyl)-1,2-benzisothiazol-3(2H)on-1,1-dioxid
2-(3-(4-(5-Fluor-2-pyrimidinyl)-1-piperazinyl)propyl)-1,2-benzisothiazol-3(2H)on-1,1-dioxid
2-(4-(4-(5-Methoxy-2-pyrimidinyl)-1-piperazinyl)butyl)-1,2-benzisothiazol-3(2H)on-1,1-dioxid
2-(3-(4-(5-Methoxy-2-pyrimidinyl)-1-piperazinyl)propyl)-1,2-benzisothiazol-3(2H)on-1,1-dioxid
2-(3-(4-(2-Pyrimidinyl)-1-piperazinyl)-2-hydroxypropyl)-1,2-benzisothiazol-3(2H)on-1,2-dioxid
2-(3-(4-(5-Phenyl-2-pyrimidinyl)-1-piperazinyl)-2-hydroxypropyl)-1,2-benzisothiazol-3(2H)on-1,1-dioxid
2-(3-(4-(5-Fluor-2-pyrimidinyl)-1-piperazinyl)-2-hydroxypropyl)-1,2-benzisothiazol-3(2H)on-1,1-dioxid
2-(3-(4-(5-(4-Chlorphenyl)-2-pyrimidinyl)-1-piperazinyl)-2-hydroxypropyl)-1,2-benzisothiazol-3(2H)on-1,1-dioxid
2-(3-(4-(2-Pyrimidinyl)-1-piperazinyl)propyl)-5-ethyloxycarbonylamino-1,2-benzisothiazol-3(2H)on-1,1-dioxid
2-(4-(4-(2-Pyrimidinyl)-1-piperazinyl)butyl)-1,2-benzisothiazol-3(2H)on-1,1-dioxid
2-(4-(4-(2-Pyrimidinyl)-1-piperazinyl)butyl)-5-ethyloxycarbonylamino-1,2-benzisothiazol-3(2H)on-1,1-dioxid
2-(4-(4-(2-Pyrimidinyl)-1-piperazinyl)butyl)-6-ethoxycarbonylamino-1,2-benzisothiazol-3(2H)on-1,1-dioxid
2-(3-(4-(2-Pyrimidinyl)-1-piperazinyl)propyl)-6-fluor-1,2-benzisothiazol-3(2H)on-1,1-dioxid
2-(4-(4-(2-Pyrimidinyl)-1-piperazinyl)butyl)-6-fluor-1,2-benzisothiazol-3(2H)on-1,1-dioxid
2-(3-(4-(2-Pyrimidinyl)-1-piperazinyl)propyl)-5-methylsulfonylamino-1,2-benzisothiazol-3(2H)on-1,1-dioxid
2-(4-(4-(2-Pyrimidinyl)-1-piperazinyl)butyl)-5-methylsulfonylamino-1,2-benzisothiazol-3(2H)on-1,1-dioxid
2-(4-(4-(2-Pyrimidinyl)-1-piperazinyl)butyl)-5-(4-methylphenyl)sulfonylamino-1,2-benzisothiazol-3(2H)-on-1,1-dioxid

2-(3-(4-(2-Pyrimidinyl)-1-piperazinyl)propyl)-5-(4-methylphenyl)sulfonylamino-1,2-benzisothiazol-3(2H)on-1,1-dioxid
2-(3-(4-(2-Pyrimidinyl)-1-piperazinyl)propyl)-6-methylsulfonylamino-1,2-benzisothiazol-3(2H)on-1,1-dioxid
2-(4-(4-(2-Pyrimidinyl)-1-piperazinyl)butyl)-6-methylsulfonylamino-1,2-benzisothiazol-3(2H)on-1,1-dioxid
2-(3-(4-(5-(4-Chlorphenyl)-2-pyrimidinyl)-1-piperazinyl)propyl)-1,2-benzisothiazol-3(2H)on-1,1-dioxid
2-(2-(4-(5-(4-Chlorphenyl)-2-pyrimidinyl)-1-piperazinyl)ethyl)-1,2-benzisothiazol-3(2H)on-1,1-dioxid
2-(4-(4-(2-Pyrimidinyl)-1-piperazinyl)butyl)-6-(4-methylphenyl)sulfonylamino-1,2-benzisothiazol-3(2H)-on-1,1-dioxid
2-(3-(4-(2-Pyrimidinyl)-1-piperazinyl)propyl)-6-(4-methylphenyl)sulfonylamino-1,2-benzisothiazol-3(2H)on-1,1-dioxid
2-(4-(4-(2-Pyrimidinyl)-1-piperazinyl)butyl)-5-sulfamoyl-1,2-benzisothiazol-3(2H)on-1,1-dioxid
2-(3-(4-(2-Pyrimidinyl)-1-piperazinyl)propyl)-5-sulfamoyl-1,2-benzisothiazol-3(2H)on-1,1-dioxid
2-(4-(4-(2-Pyrimidinyl)-1-piperazinyl)butyl)-6-sulfamoyl-1,2-benzisothiazol-3(2H)on-1,1-dioxid
2-(3-(4-(2-Pyrimidinyl)-1-piperazinyl)propyl)-6-sulfamoyl-1,2-benzisothiazol-3(2H)on-1,1-dioxid
2-(4-(4-(2-Pyrimidinyl)-1-piperazinyl)butyl)-6-amino-1,2-benzisothiazol-3(2H)on-1,1-dioxid
2-(4-(4-(2-Pyrimidinyl)-1-piperazinyl)butyl)-5-amino-1,2-benzisothiazol-3(2H)on-1,1-dioxid
2-(4-(4-(2-Pyrimidinyl)-1-piperazinyl)butyl)-5-nitro-1,2-benzisothiazol-3(2H)on-1,1-dioxid
2-(3-(4-(2-Pyrimidinyl)-1-piperazinyl)propyl)-6-amino-1,2-benzisothiazol-3(2H)on-1,1-dioxid
2-(3-(4-(2-Pyrimidinyl)-1-piperazinyl)propyl)-6-nitro-1,2-benzisothiazol-3(2H)on-1,1-dioxid
2-(3-(4-(2-Pyrimidinyl)-1-piperazinyl)propyl)-5-nitro-1,2-benzisothiazol-3(2H)on-1,1-dioxid
2-(3-(4-(2-Pyrimidinyl)-1-piperazinyl)propyl)-5-amino-1,2-benzisothiazol-3(2H)on-1,1-dioxid
2-(3-(4-(2-Pyrimidinyl)-1-piperazinyl)propyl)-6-acetylamino-1,2-benzisothiazol-3(2H)on-1,1-dioxid
2-(4-(4-(2-Pyrimidinyl)-1-piperazinyl)butyl)-6-acetylamino-1,2-benzisothiazol-3(2H)on-1,1-dioxid
2-(3-(4-(2-Pyrimidinyl)-1-piperazinyl)propyl)-5-acetylamino-1,2-benzisothiazol-3(2H)on-1,1-dioxid
2-(4-(4-(2-Pyrimidinyl)-1-piperazinyl)butyl)-5-acetylamino-1,1-benzisothiazol-3(2H)on-1,1-dioxid
2-(2-(4-(2-Pyrimidinyl)-1-piperazinyl)ethyl)-1,2-benzisothiazol-3(2H)on-1,1-dioxid
2-(3-(4-(2-Pyrimidinyl)-1-piperazinyl)-2-hydroxypropyl)-5-methoxy-1,2-benzisothiazol-3(2H)on-1,1-dioxid
2-(3-(4-(2-Pyrimidinyl)-1-piperazinyl)propyl)-6-methoxy-1,2-benzisothiazol-3(2H)on-1,1-dioxid
2-(4-(4-(2-Pyrimidinyl)-1-piperazinyl)butyl)-6-methoxy-1,2-benzisothiazol-3(2H)on-1,1-dioxid
2-(4-(4-(2-Pyrimidinyl)-1-piperazinyl)butyl)-5-methoxy-1,2-benzisothiazol-3(2H)on-1,1-dioxid
2-(4-(4-(5-Jod-2-pyrimidinyl)-1-piperazinyl)butyl)-1,2-benzisothiazol-3(2H)on-1,1-dioxid
2-(4-(4-(2-Pyrimidinyl)-1-piperazinyl)butyl)-6-nitro-1,2-benzisothiazol-3(2H)on-1,1-dioxid
2-(4-(4-(2-Pyrimidinyl)-1-piperazinyl)butyl)-5-chlor-1,2-benzisothiazol-3(2H)on-1,1-dioxid
2-(4-(4-(5-Phenyl-2-pyrimidinyl)-1-piperazinyl)butyl)-1,2-benzisothiazol-3(2H)on-1,1-dioxid
2-(4-(4-(5-Phenyl-2-pyrimidinyl)-1-piperazinyl)butyl)-6-chlor-1,2-benzisothiazol-3(2H)on-1,1-dioxid
2-(4-(4-(2-Pyrimidinyl)-1-piperazinyl)butyl)-isochinolin-1,3(2H,4H)dion
2-(4-(4-(5-(3,4-Dimethoxyphenyl)-2-pyrimidinyl)-1-piperazinyl)butyl)isochinolin-1,3(2H,4H)dion
2-(3-(4-(2-Pyrimidinyl)-1-piperazinyl)propyl)isochinolin-1,3(2H,4H)dion
2-(3-(4-(5-Phenyl-2-pyrimidinyl)-1-piperazinyl)propyl)isochinolin-1,3(2H,4H)dion
2-(3-(4-(2-Pyrimidinyl)-1-piperazinyl)2-hydroxypropyl)isochinolin-1,3(2H,4H)dion
2-(3-(4-(5-Phenyl-2-pyrimidinyl)-1-piperazinyl)-2-hydroxypropyl)isochinolin-1,3(2H,4H)dion
2-(2-(4-(2-Pyrimidinyl)-1-piperazinyl)ethyl)-1,3,2-benzodithiazol-1,1,3,3-tetroxid
2-(3-(4-(2-Pyrimidinyl)-1-piperazinyl)propyl)-1,3,2-benzodithiazol-1,1,3,3-tetroxid
2-(4-(4-(2-Pyrimidinyl)-1-piperazinyl)butyl)-1,3,2-benzodithiazol-1,1,3,3-tetroxid
2-(3-(4-(2-Pyrimidinyl)-1-piperazinyl)-2-hydroxypropyl)-1,3,2-benzodithiazol-1,1,3,3-tetroxid
2-(4-(4-(5-Fluor-2-pyrimidinyl)-1-piperazinyl)butyl)-1,3,2-benzodithiazol-1,1,3,3-tetroxid
2-(4-(4-(5-Phenyl-2-pyrimidinyl)-1-piperazinyl)butyl)-1,3,2-benzodithiazol-1,1,3,3-tetroxid
2-(4-(4-(5-(4-Chlorphenyl)-2-pyrimidinyl)-1-piperazinyl)butyl)-1,3,2-benzodithiazol-1,1,3,3-tetroxid
2-(4-(4-(5-(3-Trifluormethylphenyl)-2-pyrimidinyl)-1-piperazinyl)butyl)-1,3,2-benzodithiazol-1,1,3,3-tetroxid
2-(4-(4-(5-(4-Methoxyphenyl)-2-pyrimidinyl)-1-piperazinyl)butyl)-1,3,2-benzodithiazol-1,1,3,3-tetroxid
3-(3-(4-(2-Pyrimidinyl)-1-piperazinyl)propyl)-chinazolin-2,4(1H,3H)dion
3-(4-(4-(2-Pyrimidinyl)-1-piperazinyl)butyl)-chinazolin-2,4(1H,3H)dion
3-(3-(4-(2-Pyrimidinyl)-1-piperazinyl)propyl)-6-chlorchinazolin-2,4(1H,3H)dion
3-(4-(4-(2-Pyrimidinyl)-1-piperazinyl)butyl)-6-chlorchinazolin-2,4(1H,3H)dion
3-(3-(4-(2-Pyrimidinyl)-1-piperazinyl)propyl)-1-methylchinazolin-2,4(1H,3H)dion
3-(4-(4-(2-Pyrimidinyl)-1-piperazinyl)butyl)-1-methyl-6-chlorchinazolin-2,4(1H,3H)dion
3-(3-(4-(2-Pyrimidinyl)-1-piperazinyl)propyl)-7-fluorchinazolin-2,4(1H,3H)dion
3-(4-(4-(2-Pyrimidinyl)-1-piperazinyl)butyl)-7-fluor-1-methylchinazolin-2,4(1H,3H)dion
3-(3-(4-(2-Pyrimidinyl)-1-piperazinyl)propyl)-6-fluorchinazolin-2,4(1H,3H)dion
3-(4-(4-(2-Pyrimidinyl)-1-piperazinyl)butyl)-6-fluorchinazolin-2,4(1H,3H)dion
3-(3-(4-(2-Pyrimidinyl)-1-piperazinyl)-2-hydroxypropyl)-1-methylchinazolin-2,4(1H,3H)dion

2-(3-(4-(2-Pyrimidinyl)-1-piperazinyl)-2-hydroxypropyl)-6-fluor-1-methylchinazolin-2,4(1H,3H)dion
3-(3-(4-(2-Pyrimidinyl)-piperazinyl)propyl-6-methylthiochinazolin-2,4(1H,3H)dion
3-(4-(4-(2-Pyrimidinyl)-1-piperazinyl)butyl)-6-methylthiochinazolin-2,4(1H,3H)dion
3-(4-(4-(5-Phenyl-2-pyrimidinyl)-1-piperazinyl)butyl)-chinazolin-2,4(1H,3H)dion
3-(3-(4-(2-Pyrimidinyl)-1-piperazinyl)propyl)-6-nitrochinazolin-2,4(1H,3H)dion
3-(4-(4-(2-Pyrimidinyl)-1-piperazinyl)butyl)-6-nitrochinazolin-2,4(1H,3H)dion
3-(3-(4-(2-Pyrimidinyl)-1-piperazinyl)propyl)-7-nitrochinazolin-2,4(1H,3H)dion
3-(4-(4-(2-Pyrimidinyl)-1-piperazinyl)butyl)-7-nitrochinazolin-2,4(1H,3H)dion
3-(3-(4-(2-Pyrimidinyl)-1-piperazinyl)propyl)-6-aminochinazolin-2,4(1H,3H)dion
3-(4-(4-(2-Pyrimidinyl)-1-piperazinyl)butyl)-6-aminochinazolin-2,4(1H,3H)dion
3-(3-(4-(2-Pyrimidinyl)-1-piperazinyl)propyl)-7-aminochinazolin-2,4(1H,3H)dion
3-(4-(4-(2-Pyrimidinyl)-1-piperazinyl)butyl)-7-aminochinazolin-2,4(1H,3H)dion
3-(3-(4-(2-Pyrimidinyl)-1-piperazinyl)propyl)-6-acetylaminochinazolin-2,4(1H,3H)dion
3-(4-(4-(2-Pyrimidinyl)-1-piperazinyl)butyl)-6-acetylaminochinazolin-2,4(1H,3H)dion
3-(3-(4-(2-Pyrimidinyl)-1-piperazinyl)propyl)-7-acetylaminochinazolin-2,4(1H,3H)dion
3-(4-(4-(2-Pyrimidinyl)-1-piperazinyl)butyl)-7-acetylaminochinazolin-2,4(1H,3H)dion
2-(3-(4-(2-Pyrimidinyl)-1-piperazinyl)propyl)-1,2,4-benzothiadiazin-3(4H)on-1,1-dioxid
2-(3-(4-(2-Pyrimidinyl)-1-piperazinyl)propyl)-4-methyl-1,2,4-benzothiadiazin-3(4H)on-1,1-dioxid
2-(3-(4-(2-Pyrimidinyl)-1-piperazinyl)propyl)-4-ethyl-1,2,4-benzothiadiazin-3(4H)on-1,1-dioxid
2-(4-(4-(2-Pyrimidinyl)-1-piperazinyl)butyl)-4-methyl-1,2,4-benzothiadiazin-3(4H)on-1,1-dioxid
2-(4-(4-(2-Pyrimidinyl)-1-piperazinyl)butyl)-4-ethyl-1,2,4-benzothiadiazin-3(4H)on-1,1-dioxid
2-(2-(4-(2-Pyrimidinyl)-1-piperazinyl)ethyl)-1,2,4-benzothiadiazin-3(4H)on-1,1-dioxid
2-(4-(4-(2-Pyrimidinyl)-1-piperazinyl)butyl)-1,2,4-benzothiadiazin-3(4H)on-1,1-dioxid
2-(3-(4-(2-Pyrimidinyl)-1-piperazinyl)propyl)-4-phenyl-1,2,4-benzothiadiazin-3(4H)on-1,1-dioxid
2-(4-(4-(2-Pyrimidinyl)-1-piperazinyl)butyl)-4-phenyl-1,2,4-benzothiadiazin-3(4H)on-1,1-dioxid
2-(3-(4-(2-Pyrimidinyl)-1-piperazinyl)-2-hydroxypropyl)-1,2,4-benzothiadiazin-3(4H)on-1,1-dioxid
2-(3-(4-(2-Pyrimidinyl)-1-piperazinyl)propyl)-7-fluor-1,2,4-benzothiadiazin-3(4H)on-1,1-dioxid
2-(3-(4-(2-Pyrimidinyl)-1-piperazinyl)propyl)-7-fluor-4-methyl-1,2,4-benzothiadiazin-3(4H)on-1,1-dioxid
2-(3-(4-(2-Pyrimidinyl)-1-piperazinyl)propyl)-7-cyano-1,2,4-benzothiadiazin-3(4H)on-1,1-dioxid
2-(4-(4-(2-Pyrimidinyl)-1-piperazinyl)butyl)-7-cyano-1,2,4-benzothiadiazin-3(4H)on-1,1-dioxid
2-(3-(4-(2-Pyrimidinyl)-1-piperazinyl)propyl)-7-chlor-1,2,4-benzothiadiazin-3(4H)on-1,1-dioxid
2-(4-(4-(2-Pyrimidinyl)-1-piperazinyl)butyl)-7-chlor-1,2,4-benzothiadiazin-3(4H)on-1,1-dioxid
2-(3-(4-(5-Phenyl-2-pyrimidinyl)-1-piperazinyl)propyl)-7-chlor-1,2,4-benzothiadiazin-3(4H)on-1,1-dioxid
2-(3-(4-(2-Pyrimidinyl)-1-piperazinyl)-2-hydroxypropyl)-7-chlor-1,2,4-benzothiadiazin-3(4H)on-1,1-dioxid
2-(3-(4-(2-Pyrimidinyl)-1-piperazinyl)propyl)-7-chlor-4-methyl-1,2,4-benzothiadiazin-3(4H)on-1,1-dioxid
2-(3-(4-(2-Pyrimidinyl)-1-piperazinyl)propyl)-6-acetylamino-1,2,4-benzothiadiazin-3(4H)on-1,1-dioxid
2-(3-(4-(2-Pyrimidinyl)-1-piperazinyl)propyl)-6,8-dichlor-1,2,4-benzothiadiazin-3(4H)on-1,1-dioxid
2-(3-(4-(2-Pyrimidinyl)-1-piperazinyl)propyl)-6,8-dichlor-4-methyl-1,2,4-benzothiadiazin-3(4H)on-1,1-dioxid
2-(3-(4-(2-Pyrimidinyl)-1-piperazinyl)propyl)-6,7-dichlor-1,2,4-benzothiadiazin-3(4H)on-1,1-dioxid
2-(3-(4-(2-Pyrimidinyl)-1-piperazinyl)propyl)-6,7-dichlor-4-methyl-1,2,4-benzothiadiazin-3(4H)on-1,1-dioxid
2-(3-(4-(2-Pyrimidinyl)-1-piperazinyl)propyl)-5,7-dichlor-1,2,4-benzothiadiazin-3(4H)on-1,1-dioxid
2-(4-(4-(2-Pyrimidinyl)-1-piperazinyl)butyl)-5,7-dichlor-1,2,4-benzothiadiazin-3(4H)on-1,1-dioxid
2-(3-(4-(2-Pyrimidinyl)-1-piperazinyl)propyl)-7-nitro-1,2,4-benzothiadiazin-3(4H)on-1,1-dioxid
2-(4-(4-(2-Pyrimidinyl)-1-piperazinyl)butyl)-7-nitro-1,2,4-benzothiadiazin-3(4H)on-1,1-dioxid
2-(3-(4-(2-Pyrimidinyl)-1-piperazinyl)propyl)-7-nitro-4-methyl-1,2,4-benzothiadiazin-3(4H)on-1,1-dioxid
2-(3-(4-(2-Pyrimidinyl)-1-piperazinyl)propyl)-6-chlor-1,2,4-benzothiadiazin-3(4H)on-1,1-dioxid
2-(3-(4-(2-Pyrimidinyl)-1-piperazinyl)propyl)-7-amino-1,2,4-benzothiadiazin-3(4H)on-1,1-dioxid
2-(3-(4-(2-Pyrimidinyl)-1-piperazinyl)propyl)-7-amino-4-methyl-1,2,4-benzothiadiazin-3(4H)on-1,1-dioxid
2-(3-(4-(2-Pyrimidinyl)-1-piperazinyl)propyl)-7-acetylamino-4-methyl-1,2,4-benzothiadiazin-3(4H)on-1,1-dioxid
2-(3-(4-(2-Pyrimidinyl)-1-piperazinyl)propyl)-7-ethoxy-carbonylamino-4-methyl-1,2,4-benzothiadiazin-3(4H)on-1,1-dioxid
2-(4-(4-(5-Phenyl-2-pyrimidinyl)-1-piperazinyl)butyl)isoindol-1,3(2H)dion
2-(4-(4-(5-(3,4-Dimethoxyphenyl)-2-pyrimidinyl)-1-piperazinyl)butyl)isoindol-1,3(2H)dion
2-(4-(4-(5-(2-Chlor-6-fluorphenyl)-2-pyrimidinyl)-1-piperazinyl)butyl)isoindol-1,3(2H)dion
2-(4-(4-(2-Pyrimidinyl)-1-piperazinyl)butyl)isoindol-1,3(2H)dion
2-(4-(4-(5-Fluor-2-pyrimidinyl)-1-piperazinyl)butyl)isoindol-1,3(2H)dion
2-(4-(4-(5-(4-Chlorphenyl)-2-pyrimidinyl)-1-piperazinyl)butyl)isoindol-1,3(2H)dion
2-(4-(4-(5-(4-Chlorphenyl)-2-pyrimidinyl)-1-piperazinyl)butyl)isoindol-1,3(2H)dion
2-(3-(4-(5-(4-Chlorphenyl-2-pyrimidinyl)-1-piperazinyl)propyl)isoindol-1,3(2H)dion
2-(4-(4-(5-Phenyl-2-pyrimidinyl)-1-piperazinyl)butyl)-4-amino-isoindol-1,3(2H)dion

2-(4-(4-(5-(3-Indolyl)-2-pyrimidinyl)-1-piperazinyl)butyl)isoindol-1,3(2H)dion
2-(2-(4-(5-(4-Chlorphenyl-2-pyrimidinyl)-1-piperazinyl)ethyl)isoindol-1,3(2H)dion
2-(3-(4-(2-Pyrimidinyl)-1-piperazinyl)propyl)isoindol-1,3(2H)dion
2-(2-(4-(2-Pyrimidinyl)-1-piperazinyl)ethyl)isoindol-1,3(2H)dion
2-(3-(4-(5-Phenyl-2-pyrimidinyl)-1-piperazinyl)propyl)isoindol-1,3(2H)dion
2-(2-(4-(5-(Phenyl-2-pyrimidinyl)-1-piperazinyl)ethyl)isoindol-1,3(2H)dion
2-(3-(4-(2-Pyrimidinyl)-1-piperazinyl)-2-hydroxypropyl)isoindol-1,3(2H)dion
2-(3-(4-(5-(4-Chlorphenyl)-2-pyrimidinyl)-1-piperazinyl)-2-hydroxypropyl)isoindol-1,3(2H)dion
2-(3-(4-(5-(4-Methoxyphenyl)-2-pyrimidinyl)-1-piperazinyl)-2-hydroxypropyl)isoindol-1,3(2H)dion
2-(3-(4-(5-(3-Trifluormethylphenyl)-2-pyrimidinyl)-1-piperazinyl)-2-hydroxypropyl)isoindol-1,3(2H)dion
2-(4-(4-(2-Pyrimidinyl)-1-piperazinyl)butyl)-5,6-dichlor-isoindol-1,3(2H)dion
2-(4-(4-(5-Phenyl-2-pyrimidinyl)-1-piperazinyl)butyl)-5,6-dichlor-isoindol-1,3(2H)dion
2-(4-(4-(2-Pyrimidinyl)-1-piperazinyl)butyl)-5-nitro-isoindol-1,3(2H)dion
2-(4-(4-(5-Phenyl-2-pyrimidinyl)-1-piperazinyl)butyl)-5-nitro-isoindol-1,3(2H)dion
2-(4-(4-(2-Pyrimidinyl)-1-piperazinyl)butyl)-4-nitro-isoindol-1,3(2H)dion
2-(4-(4-(5-(4-Chlorphenyl)-2-pyrimidinyl)-1-piperazinyl)butyl)-4-nitro-isoindol-1,3(2H)dion
2-(4-(4-(5-Phenyl-2-pyrimidinyl)-1-piperazinyl)butyl)-5-amino-isoindol-1,3(2H)dion
2-(4-(4-(5-Phenyl-2-pyrimidinyl)-1-piperazinyl)butyl)-5-acetylamino-isoindol-1,3(2H)dion
2-(4-(4-(5-Phenyl-2-pyrimidinyl)-1-piperazinyl)butyl)-4-amino-isoindol-1,3(2H)dion
2-(4-(4-(5-(3-Indolyl)-2-pyrimidinyl)-1-piperazinyl)butyl)-isoindol-1,3(2H)dion
2-(4-(4-(5-Phenyl-2-pyrimidinyl)-1-piperazinyl)butyl)-5-methyl-isoindol-1,3(2H)dion
2-(4-(4-(5-Phenyl-2-pyrimidinyl)-1-piperazinyl)butyl)-4-methoxy-isoindol-1,3(2H)dion
2-(4-(4-(5-Phenyl-2-pyrimidinyl)-1-piperazinyl)butyl)-5-trifluormethyl-isoindol-1,3(2H)dion

Beispielsweise wurden die psychotropen Eigenschaften der erfindungsgemäßen Substanzen wie folgt untersucht:

1. Amphetamin-Potenzierung

Antidepressiv wirksame Substanzen potenzieren das Amphetamin-induzierte stereotype Verhalten bei der Ratte. Der angegebene $DE_{50}$-Wert ist die Dosis, bei der das Amphetamin-induzierte Verhalten nach Gabe von 2 mg/kg DL-Amphetaminsulfat i.v. um 50% verstärkt wird.

Lit.: J. L. Howard et al., in: Antidepressants: Neurochemical, Behavioral and Clinical Perspectives, herausgegeben von S. J. Enna et al., Raven Press, New York, S. 107—120, 1981.

Dazu seien vorzugsweise folgende Beispiele genannt:

| Substanz | $DE_{50}$ (mg/kg i.p.) |
|---|---|
| Beispiel 1 | 18 |
| 4 | 3 |
| 8 | 1 |
| 20 | 5 |

2. Tetrabenazin-Antagonismus

Antidepressiva antagonisieren die durch Tetrabenazin induzierte Ptosis bei Mäusen. Der $DE_{50}$-Wert gibt diejenige Dosis an, bei die durch Tetrabenazin (20 mg/kg i.p.) induzierte Ptosis zu 50% reduziert ist.

Lit.: J. L. Howard et al., in: Antidepressants: Neurochemical, Behavioral and Clinical Perspectives, herausgegeben von S. J. Enna et al., Raven Press, New York, S. 107—120, 1981.

Dazu seien vorzugsweise folgende Beispiele genannt:

| Substanz | $DE_{50}$ (mg/kg i.p.) |
|---|---|
| Beispiel 6 | 50 |
| 18 | 21 |

3. Behavioral Despair

Ratten zeigen — in einen mit Wasser gefüllten Glaszylinder gesetzt — zunächst hastige Schwimmbewegungen, die durch immer länger werdende Phasen der Immbolität abgelöst werden. Zahlreiche Antidepressiva unterschiedlicher chemischer Struktur verkürzen die Dauer dieser Immobilität. Der $DE_{50}$-Wert gibt diejenige Dosis einer Prüfsubstanz an, bei der die Immobilität während des Versuchszeitraums um 50% reduziert ist.

Lit.: R. D. Porsolt et al., Europ. J. Pharmacol. 47, 379—391, 1978.
Dazu seien vorzugsweise folgende Beispiele genannt:

| Substanz | $DE_{50}$ (mg/kg i.p.) |
|---|---|
| Beispiel 4 | 12 |
| 8 | 33 |
| 12 | 50 |

4. Tedeschi-Test

Anxiolytika bewirken bei Mäusen eine Reduktion des Schock-induzierten aggressiven Verhaltens. Der $DE_{50}$-Wert gibt diejenige Dosierung an, bei der das aggressive Verhalten innerhalb des Versuchszeitraums um 50% reduziert ist.

Lit.: Tedeschi et al., J. Pharmacol. Exp. Ther. 129, 28—34, 1954.
Dazu seien vorzugsweise folgende Beispiele genannt:

| Substanz | $DE_{50}$ (mg/kg i.p.) |
|---|---|
| Beispiel 4 | 20 |
| 6 | 15 |
| 7 | 5 |
| 8 | 12 |
| 12 | 16 |
| Beispiel 15 | 6 |
| 18 | 9 |

(Fortsetzung)

5. Passive avoidance Behavior

Ratten bekommen nach dem Eintritt in eine abgedunktelte Box einen Elektroschock und vermeiden daraufhinm die Box erneut zu betreten. Unter dem Einfluß von Anxiolytika betreten die Ratten die Box trotz der Schockerfahrung. Die Zeit bis zum Eintritt in die Box wird gestoppt. Angegeben wird die niedrigst wirksame Dosis (NWD) in mg/kg.

Lit.: Ader et al., Psychon. Sci. 26, 125—127, 1972.
Dazu seien vorzugsweise folgende Beispiele genannt:

| Substanz | NWD (mg/kg i.p.) |
|---|---|
| Beispiel 7 | 1 |
| 7 | 0,5 |
| 10 | 1 |
| 11 | 1 |
| 18 | 1 |

6. Serotonin-Rezeptor

Die direkte oder indirekte Beeinflussung des serotoninergen Systems durch beispielsweise anxiolytische, tranquillisierende, neuroleptische oder antidepressive Wirkstoffe ist bekannt. Erfindungsgemäße Substanzen interagieren spezifisch mit Serotonin-Rezeptoren aus Kalbs-Hippocampus.

Angegeben ist diejenige Konzentration, bei der 50% des eingesetzten $^3H$-Serotonins verdrängt wird ($K_i$-Werte).

Lit.: Peroutka, S. L. und S. H. Snyder, Molec. Pharmacol. 16, 687, 1979.

Als Beispiele seien vorzugsweise genannt:

| Substanz | $K_i$ (nM) |
|---|---|
| Beispiel 1 | 10 |
| 7 | 20 |
| 10 | 14 |
| 11 | 30 |
| 19 | 30 |
| 24 | 20 |
| 32 | 10 |
| 33 | 60 |
| 36 | 9 |
| 37 | 80 |

Die vorgenannten Wirkbeispiele sollen die Erfindung erläutern und verdeutlichen, ohne sie auf diese Beispiele einzuengen.

Zur vorliegenden Erfindung gehören pharmazeutische Zubereitungen, die neben nicht-toxischen, inerten, pharmazeutisch geeigneten Trägerstoffen eine oder mehrere der erfindungsgemäßen Verbindungen oder deren Salze enthalten oder die aus einer oder mehrerer der erfindungsgemäßen Verbindungen oder deren Salzen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten, pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzuker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quaternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opalisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositoren können neben dem oder den Wirkstoffen die üblichen wasserlöslichen oder wasserunlöslichen Trägerstoffe enthalten, z.B. Polyethylenglykole, Fette, z.B. Kakaofett, und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Läsungsvermittler und Emulgator, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylenglykol, Suspendiermittel, z.B. ethoxylierte Isostearylalkohole, Polyoxyethylensorbit- und -sorbitanester, mikrokristalline Cellulose, Aluminiummetahydroxid, Bentonid, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Sübmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer Verbindungen der Formel (I) und/oder deren Salzen auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehören auch die Verwendung der Verbindungen der Formel (I) und/oder deren Salzen sowie von pharmazeutischen Zubereitungen, die eine oder mehrere Verbindungen der Formel (I) und/oder deren Salze enthalten, in der Humanmedizin zur Verhütung, Besserung und/oder Heilung der oben aufgeführten Erkrankungen.

Die Wirkstoffe oder die pharmazeutischen Zubereitungen können vorzugsweise oral, parenteral und/ oder rectal, vorzugsweise oral parenteral, insbesondere oral und intravenös, appliziert werden.

Im allgemeinen hat es sich als vorteilhaft erwiesen, den oder die Wirkstoffe bei parenteraler (i.v. oder i.m.) Applikation in Mengen von etwa 0,01 bis etwa 10, vorzugsweise von 0,1 bis 1 mg/kg Körpergewicht je 24 Stunden und bei oraler Applikation in Mengen von etwa 0,05 bis etwa 100, vorzugsweise 0,1 bis 10 mg/ kg Körpergewicht je 24 Stunden, gegebenenfalls in Form mehrerer Einzelgaben zur Erzielung der gewünschten Ergebnisse zu verabreichen. Eine Einzelgabe enthält den oder die Wirkstoffe, vorzugsweise in Mengen von etwa 0,01 bis etwa 30, insbesondere 0,03 bis 3 mg/kg Körpergewicht.

Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der o.g. Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachman aufgrund seines Fachwissens leicht erfolgen.

Die vorliegende Erfindung soll durch die nachfolgenden Beispiele noch näher erläutert werden:

## Beispiel 1

Darstellung von (2-(4-(4-(2-Pyrimidinyl)-1-piperazinyl)butyl)-isoindol-1,3(2H)dion

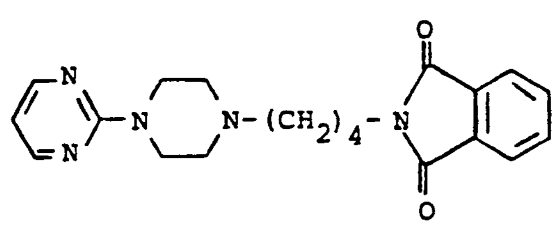

0,2 Mole 2-(4-Brombutyl)isoindol-1,3(2H)dion und 0,2 Mol 1-(2-Pyrimidyl)-piperazin werden mit 0,2 Mol $K_2CO_3$ über Nacht unter $N_2$-Atmosphäre bei 120—130°C gerührt. Danach engt man bis zur Trocke ein. Mit Wasser versetzt wird der Rückstand in Methylenchlorid aufgenommen. Nach dem Trocknen der organischen Lösung engt man ein und erhält ein Öl, das mit Cyclohexan verrieben kristallisiert.

Ausbeute: 96% der Theorie; Fp = 138°C.

Analog wurden hergestellt:

| Beispiel Nr. | Formel | Fp (°C) | Ausbeute % der Theorie |
|---|---|---|---|
| | 2-(2-(4-(2-Pyrimidinyl)-1-piperazinyl)ethyl)isoindol-1,3(2H)dion | | |
| 2 | 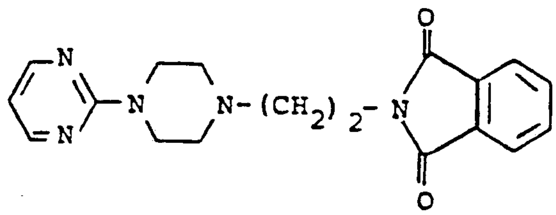 | 124° (aus Cyclohexan) | 45 |
| | 2-(3-(4-(2-Pyrimidinyl)-1-piperazinyl)propyl)isoindol-1,3(2H)dion | | |
| 3 | 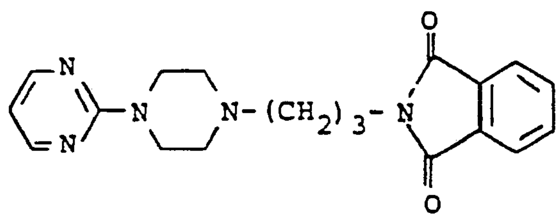 | 95° (aus Cyclohexan) | 46 |

Beispiel 4

Darstellung von 2-(4-(4-(5-Phenyl-2-pyrimidinyl)-1-piperazinyl)butyl)isoindol-1,3(2H)dion

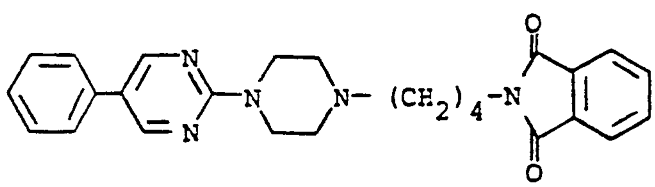

0,05 Mol 2-(4-Brombutyl)isoindol-1,3(2H)dion und 0,05 Mol 1-(5-Phenyl-2-pyrimidinyl)-piperazin werden mit 0,05 Mol $K_2CO_3$ in 80 ml Chlorbenzol unter $N_2$-Atmosphäre 8 Stunden unter Rühren auf 130°C erhitzt. Nach Erkalten wird am Rotavapor bis zur Trockne eingeengt. Der Rückstand wird mit Wasser versetzt und in Methylenchlorid aufgenommen. Die getrocknete Methylenchlorid-Lösung wird auf eine Kieselgelsäule gegeben und mit $CH_2Cl_2$/Isopropanol (10:1) eluiert. Die Substanz wird anschließend rekristallisiert.

Ausbeute: 88% der Theorie; Fp.: 126°C (aus Cyclohexan)

Analog wurden hergestellt:

| Beispiel Nr. | Formel | Fp (°C) | Ausbeute % der Theorie |
|---|---|---|---|
| | 2-(2-(4-(5-Phenyl-2-pyrimidinyl)-1-piperazinyl)ethylisoindol-1,3(2H)dion | | |
| 5 | 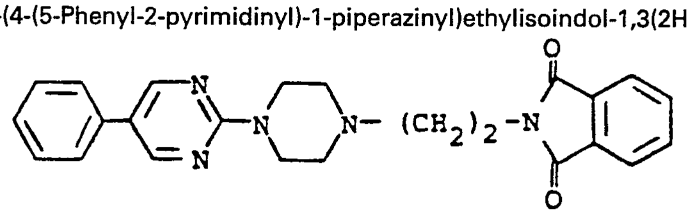 | 178° | 49 |
| | 2-(3-(4-(5-Phenyl-2-pyrimidinyl)-1-piperazinyl)propyl)isoindol-1,3(2H)dion | | |
| 6 | 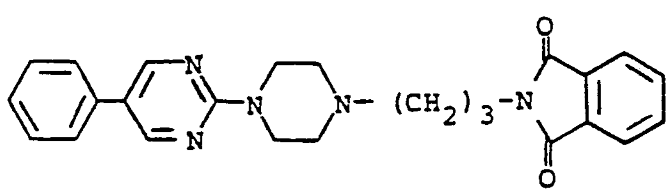 | 148° | 62 |

| Beispiel Nr. | Formel | Fp (°C) | Ausbeute % der Theorie |
|---|---|---|---|
| | 2-(4-(4-(2-Pyrimidinyl)-1-piperazinyl)butyl)-1,2-benzisothiazol-3(2H)on-1,1-dioxid | | |
| 7 | 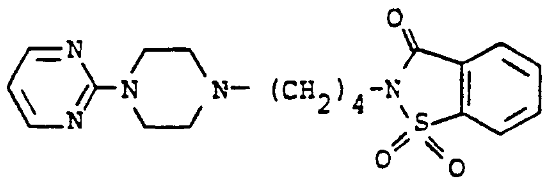 | 137—138° (aus Isopropanol) Hydrochlorid: 221—222° | 53 |
| | 2-(4-(4-(5-(2-Chlor-6-fluorphenyl)-2-pyrimidinyl)-1-piperazinyl)butyl)-isoindol-1,3(2H)dion | | |
| 8 | 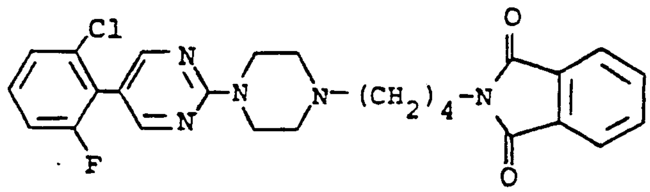 | 123—124° | 82 |
| | 2-(4-(4-(5-Phenyl-2-pyrimidinyl)-1-piperazinyl)butyl)-1,2-benzoisothiazol-3(2H)on-1,1-dioxid | | |
| 9 | 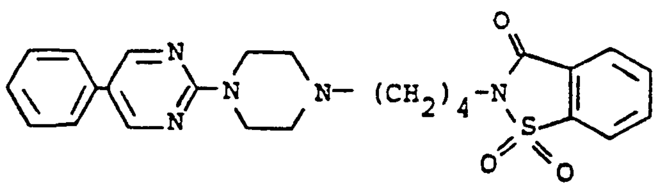 | 153° (aus Ethanol) | 63,5 |
| | 2-(4-(4-(2-Pyrimidinyl)-1-piperazinyl)butyl)-5-chlor-1,2-benzoisothiazol-3(2H)on-1,1-dioxid | | |
| 10 | 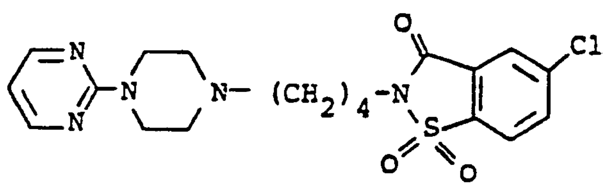 | 120—121° (aus Isopropanol) | 30 |
| | 2-(4-(4-(2-Pyrimidinyl)-1-piperazinyl)butyl)-6-chlor-1,2-benzoisothiazol-3(2H)on-1,1-dioxid | | |
| 11 | 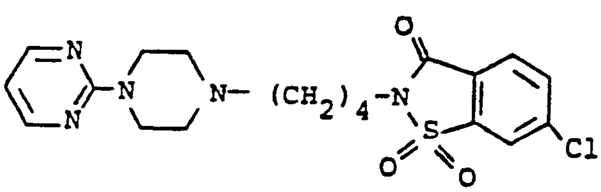 | 154—155° (aus Isopropanol) | 34 |
| | 2-(4-(4-(5-(3,4-Dimethoxyphenyl)-2-pyrimidinyl)-1-piperazinyl)butyl)isoindol-1,3(2H)dion | | |
| 12 | 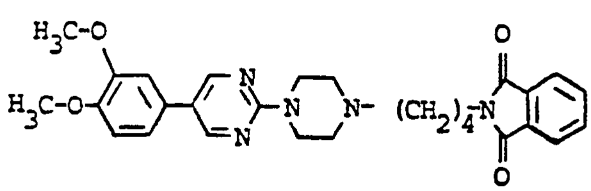 | 169—170° (aus Essigester) | 89 |
| | 2-(4-(4-(5-(4-Chlorphenyl)-2-pyrimidinyl)-1-piperazinyl)butyl)isoindol-1,3(2H)dion | | |
| 13 | 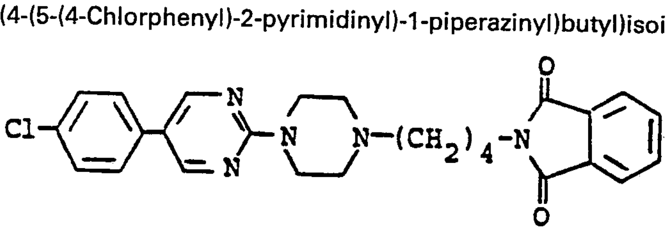 | 192—193° | 70 |

| Beispiel Nr. | Formel | Fp (°C) | Ausbeute % der Theorie |
|---|---|---|---|
| | 2-(3-(4-(5-(4-Chlorphenyl)-2-pyrimidinyl)-1-piperazinyl)propyl)isoindol-1,3(2H)dion | | |
| 14 | 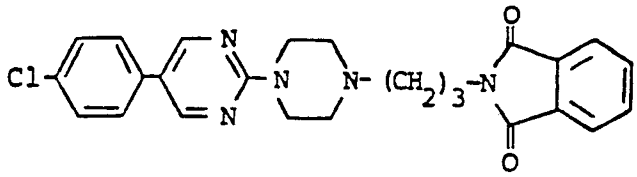 | 174—175° | 56 |
| | 2-(2-(4-(5-(4-Chlorphenyl)-2-pyrimidinyl)-1-piperazinyl)ethyl)isoindol-1,3(2H)dion | | |
| 15 | 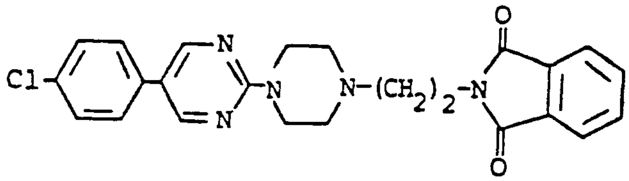 | 209—210° | 52 |
| | 2-(4-(4-(5-(3-Indolyl)-2-pyrimidinyl)-1-piperazinyl)butyl)isoindol-1,3(2H)dion | | |
| 16 | 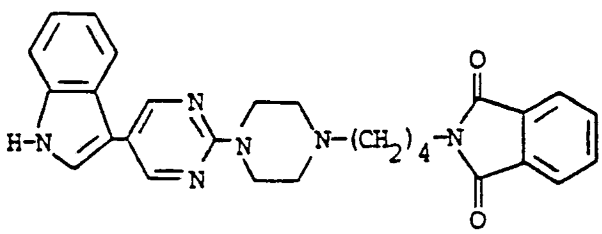 | 163—164° | 66 |

Beispiel 17

Darstellung von 2-(4-(4-(5-Phenyl-2-pyrimidinyl)-1-piperazinyl)butyl)-5,6-dichloroisoindol-1,3(2H)dion

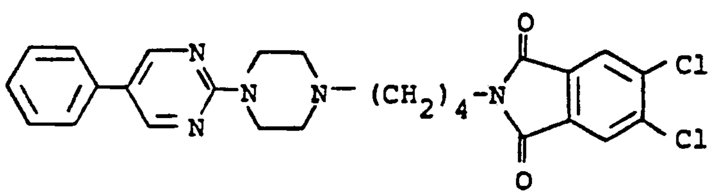

0,005 Mol 4,5-Dichlorphthalsäureanhydrid und 0,005 Mol 1-(4-Aminobutyl)-4-(5-phenyl-2-pyrimidinyl)piperazin werden in 20 ml absolutem Pyridin mehrere Stunden unter $N_2$-Atmosphäre unter Rückfluß gekocht. Nach Abkühlen filtriert man das auskristallisierte Produkt ab und wäscht mit Isopropylether.

Ausbeute: 90% der Theorie; Fp.: 206—207°C.

Beispiel 18

Darstellung von 2-(3-(4-(5-(4-Chlorphenyl)-2-pyrimidinyl)-1-piperazinyl)-2-hydroxypropyl)isoindol-1,3(2H)dion

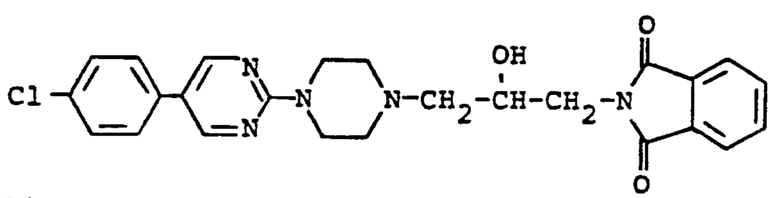

0,022 Mol 2-(2,3-Epoxypropyl)-isoindol-1,3(2H)dion, 0,02 Mol 1-(5-(4-Chlorphenyl)-2-pyrimidinyl)-piperazin und 70 ml Isopropanol werden 2 Stunden zum Sieden erhitzt. Man engt den Ansatz ein und reinigt den Rückstand durch Säulenchromatographie über Kieselgel 60. Nack dem Eluieren mit Methylenchlorid/Alkohol (96:4) wird die Verbindung mit Alkohol gerührt und abgesaugt.

F = 180—181°C.

Ausbeute: 88% der Theorie.

Beispiel 19

Darstellung von 2-(4-(4-(2-Pyrimidinyl)-1-piperazinyl)butyl)-isochinolin-1,3(2H,4H)dion

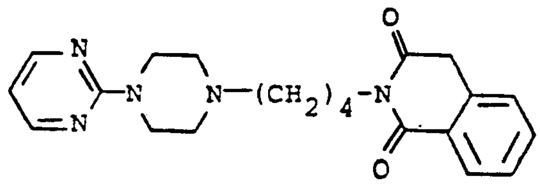

0,02 Mol 2-Benzopyran-1,3-(4H)dion und 0,02 Mol 1-(4-Aminobutyl)-4-(2-pyrimidinyl)-piperazin werden unter $N_2$-Atmosphäre und Rühren 1 Stunde auf 160—170°C erhitzt. Danach läßt man abkühlen und löst die Substanz in Methylenchlorid. Über eine Kieselgelsäule (Laufmittel: $CH_2Cl_2$/iPrOH (10:0,5)) wird die Substanz gereinigt.

Ausbeute: 33% der Theorie; Fp.: 103—104°C.

Analog wurden hergestellt:

| Beispiel Nr. | Formel | Fp (°C) | Ausbeute % d. Th. |
|---|---|---|---|
| | 2-(4-(4-(5-(3,4-Dimethoxyphenyl)-2-pyrimidinyl)-1-piperazinyl)butyl)isochinolin-1,3(2H,4H)dion | | |
| 20 | 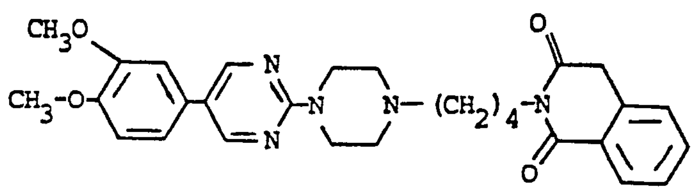 | 179—180° | 51 |
| | 2-(4-(4-(5-Phenyl-2-pyrimidinyl)-1-piperazinyl)butyl)isoindol-1,3(2H)dion | | |
| 21 | 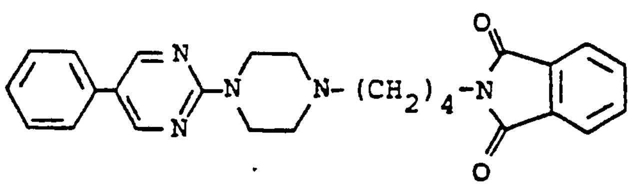 | 126° | 60 |

Beispiel 22

Darstellung von 2-(4-(4-(2-Pyrimidinyl)-1-piperazinyl)butyl)-5-fluor-1,2-benzoisothiazol-3(2H)on-1,1-dioxid

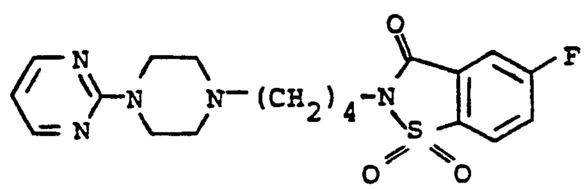

0,02 Mol 5-Fluor-2-(4-bromobutyl)-1,2-benzoisothiazol-3(2H)on-1,1-dioxid und 0,02 Mol 1-(2-Pyrimidinyl)-piperazin werden mit 0,02 Mol $K_2CO_3$ in 150 ml absolutem DMF 1 Stunde bei 100°C gerührt. Danach engt man ein. Mit Wasser versetzt, wird die organische Substanz in Methylenchlorid aufgenommen. Die getrocknete $CH_2Cl_2$-Phase wird auf eine Kieselgelsäule gegeben und mit $CH_2Cl_2/CH_3OH$ (95:5) eluiert.

Ausbeute: 34% der Theorie; Fp.: 138—139°C.

| Beispiel Nr. | Formel | Fp (°C) | Ausbeute % d. Th. |
|---|---|---|---|
| 23 | 2-(4-(4-(5-Iodo-2-pyrimidinyl)-1-piperazinyl)butyl)-1,2-benzoisothiazol-3(2H)on-1,1-dioxid 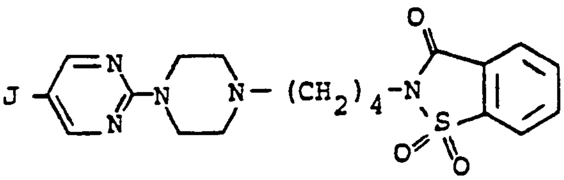 | 124—125° | 40 |
| 24 | 2-(4-(4-(2-Pyrimidinyl)-1-piperazinyl)butyl)-6-nitro-1,2-benzoisothiazol-3(2H)on-1,1-dioxid 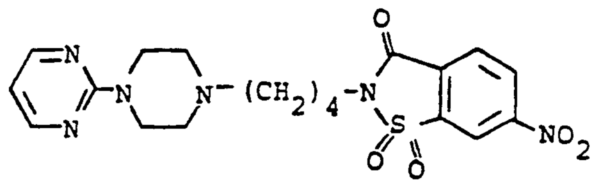 | 168° | 45 |
| 25 | 2-(3-(4-(2-Pyrimidinyl)-1-piperazinyl)propyl)-1,2-benzoisothiazol-3(2H)on-1,1-dioxid 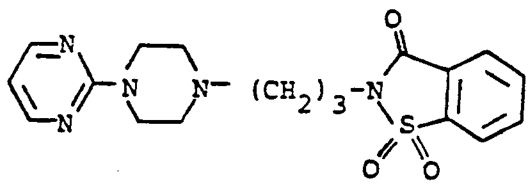 | 131° | 50 |
| 26 | 2-(4-(4-(5-Fluor-2-pyrimidinyl)-1-piperazinyl)butyl)-1,2-benzoisothiazol-3(2H)on-1,1-dioxid 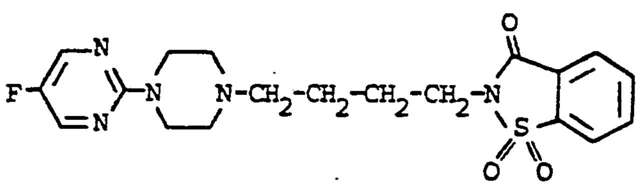 | 124—125° | 40 |
| 27 | 2-(4-(4-(5-(3-Trifluormethylphenyl)-2-pyrimidinyl)-1-piperazinyl)butyl)-1,2-benzoisothiazol-3(2H)on-1,1-dioxid 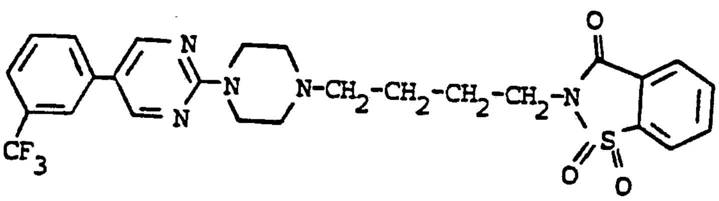 | 144—145° | 40 |

Beispiel 28

Darstellung von 3-(4-(4-(2-Pyrimidinyl)-1-piperazinyl)butyl)-chinazolin-2,4(1H,3H)dion

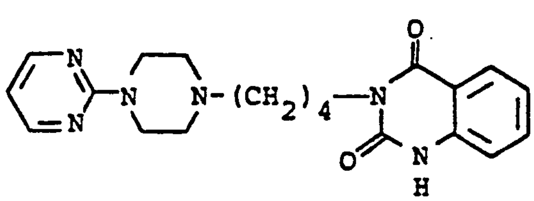

0,02 Mol N-(4-(2-Pyrimidinyl)-1-piperazinyl)butyl)-2-aminobenzoesäureamid, 0,04 Mol Triethylamin und 80 ml absolutes Toluol werden unter Rühren mit einer Lösung von 0,028 Mol Phosgen in 20 ml absolutem Toluol versetzt. Nach Abklingen der exothermen Reaktion wird noch 2 h gerührt. Man engt ein

und schüttelt mit Methylenchlorid und verdünntem wäßrigem Ammoniak. Die organische Phase wird vom Lösungsmittel befreit und der Rückstand umkristallisiert.

Fp: 187—188°C (aus Essigester)

Ausbeute: 59% der Theorie

Analog wurden dargestellt:

| Beispiel Nr. | Formel | Fp (°C) | Ausbeute % d.Th. |
|---|---|---|---|
| | 3-(3-(4-(2-Pyrimidinyl)-1-piperazinyl)propyl)chinazolin-2,4(1H,3H)dion | | |
| 29 | 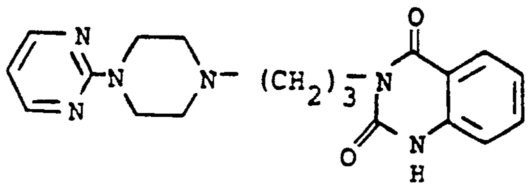 | 177—178°C (aus Essigester) | 74 |
| | 3-(4-(4-(2-Pyrimidinyl)-1-piperazinyl)butyl)-6-chlorchinazolin-2,4(1H,3H)dion | | |
| 30 | 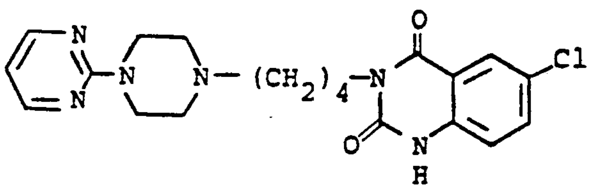 | 181—182° (aus Essigester) | 44 |
| | 3-(3-(4-(2-Pyrimidinyl)-1-piperazinyl)propyl)-6-chlorchinazolin-2,4(1H,3H)dion | | |
| 31 | 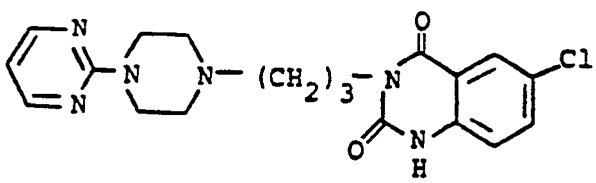 | 245—246° (aus DMF) | 76 |
| | 2-(3-(4-(2-Pyrimidinyl)-1-piperazinyl)propyl)1,2,4-benzothiadiazin-3(4H)on-1,1-dioxid | | |
| 32 | 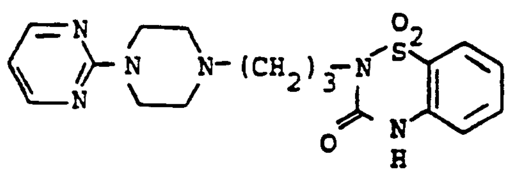 | 182,4° (aus Essigester) | 93 |
| | 2-(4-(4-(2-Pyrimidinyl)-1-piperazinyl)butyl)-1,2,4-benzothiadiazin-3(4H)on-1,1-dioxid | | |
| 33 | 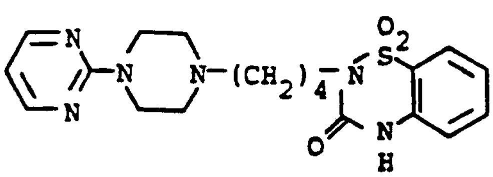 | 131,6° (aus Isopropanol) | 83 |

Beispiel 34

Darstellung von 3-(3-(4-(2-Pyrimidinyl)-1-piperazinyl)propyl)-1-methylchinazolin-2,4(1H,3H)dion

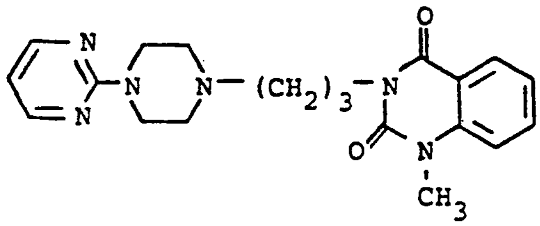

0,02 Mol 3-(3-(4-(2-Pyrimidinyl)-1-piperazinyl)-propyl)-chinazolin-2,4(1H,3H)dion werden in 100 ml absolutem DMF gegeben, 0,02 Mol Natriumhydrid (55—60%ige Dispersion) zugesetzt und 1 Stunde gerührt. Nach Zusatz von 0,02 Mol Methyliodid weitere 3 Stunden gerührt, in 300 ml Wasser gegossen und mit Methylenchlorid augeschüttelt. Man engt die organische Phase ein und reinigt den Rückstand durch Säulenchromatographie über Kieselgel 60. Nach dem Eluieren mit Methylenchlorid/Methanol (95:5) wird die Verbindung umkristallisiert.

Fp: 158—159° (aus Essigester),

Ausbeute: 77% der Theorie

Analog wurden dargestellt:

| Beispiel Nr. | Formel | Fp (°C) | Ausbeute % d.Th. |
|---|---|---|---|
| | 3-(4-(4-(2-Pyrimidinyl)-1-piperazinyl)butyl)-6-chlor-1-methyl-chinazolin-2,4(1H,3H)dion | | |
| 35 | 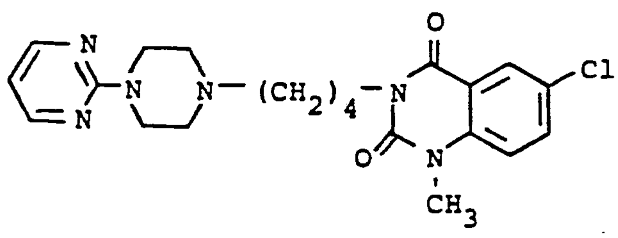 | 148—149° (aus Essigester) | 76 |
| | 2-(3-(4-(2-Pyrimidinyl)-1-piperazinyl)propyl)-4-methyl-1,2,4-benzothiadiazin-3(4H)on-1,1-dioxid | | |
| 36 | 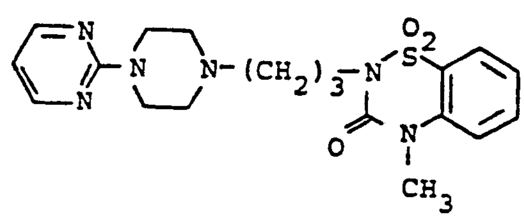 | 121,7° (aus Isopropanol) | 58 |
| | 2-(4-(4-(2-Pyrimidinyl)-1-piperazinyl)butyl)-4-methyl-1,2,4-benzothiadiazin-3(4H)on-1,1-dioxid | | |
| 37 | 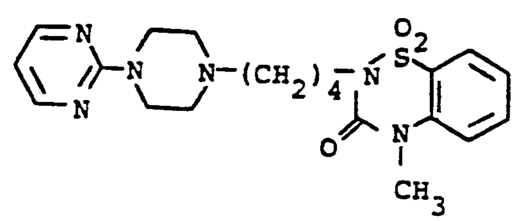 | 131,6° (aus Isopropanol) | 70 |

Beispiel 38

Darstellung von 2-(4-(4-(2-Pyrimidinyl)-1-piperazinyl)butyl)-7-chlor-1,2,4-benzothiadiazin-3(4H)on-1,1-dioxid

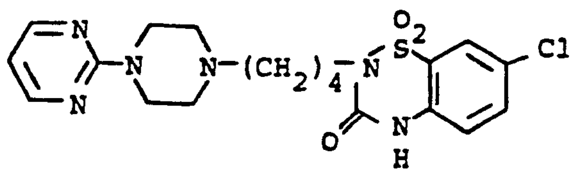

0,01 Mol 7-Chlor-2-(4-brombutyl)-1,2,4-benzothiadiazin-3(4H)on-1,1-dioxid werden in 50 ml DMF gelöst, 0,013 Mol 1-(2-Pyrimidinyl)-piperazin zugegeben und 1 Stunde auf 100°C erwärmt. Danach wird eingeengt, und über Kieselgel 60 gereinigt. Laufmittel: $CH_2Cl_2$/MeOH (95:5).

Ausbeute: 43% der Theorie, Fp.: 129°C

Beispiel 39

Darstellung von N-(3-(4-(2-Pyrimidinyl)-1-piperazinyl)propyl)-2-aminobenzoesäureamid

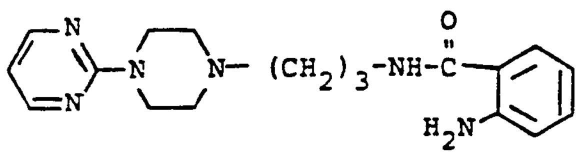

0,02 Mol 1-(3-Aminopropyl)-4-(2-pyrimidinyl)-piperazin werden in 100 ml absolutem Dimethylformamid gelöst, 0,02 Mol Isatosäureanhydrid zugesetzt und 1 Stunde gerührt. Man verdünnt mit 50 ml Wasser, schüttelt mit Essigester und engt die organische Phase ein. Gereinigt wird durch Säulenchromatographie über Kieselgel 60. Nach dem Eluieren mit Methylenchlorid/Methanol 93:7 läßt sich das erhaltene Öl aus Essigester mit Isopropylether kristallisieren.

Fp: 79—80°C,

Ausbeute: 80% der Theorie

Analog wurden dargestellt:

| Beispiel Nr. | Formel | Fp (°C) | Ausbeute % d.Th. |
|---|---|---|---|
| | N-(4-(4-(2-Pyrimidinyl)-1-piperazinyl)butyl)-2-aminobenzoesäureamid | | |
| 40 | 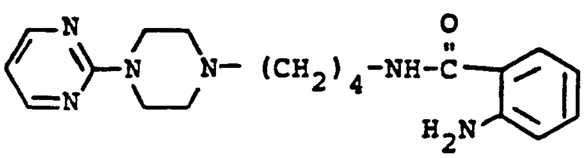 | 113—114° (aus Essigester/Isopropylether) | 80 |
| | N-(3-(4-(2-Pyrimidinyl)-1-piperazinyl)propyl)-2-amino-5-chlor-benzoesäureamid | | |
| 41 | 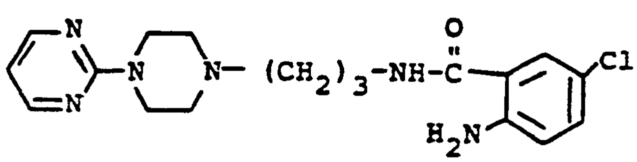 | 119—120° (aus Essigester/Isopropylether) | 76 |
| | N-(4-(4-(2-Pyrimidinyl)-1-piperazinyl)butyl)-2-amino-5-chlor-benzoesäureamid | | |
| 42 | 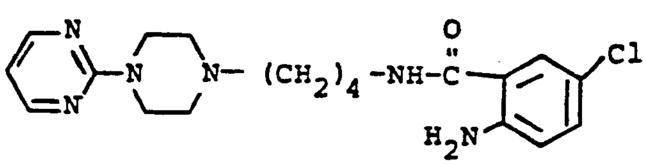 | 118—119° (aus Essigester) | 63 |

| Beispiel Nr. | Formel | Fp (°C) | Ausbeute % d.Th. |
|---|---|---|---|
| | N-(3-(4-(2-Pyrimidinyl)-1-piperazinyl)propyl)-2-methylaminobenzoesäureamid | | |
| 43 | 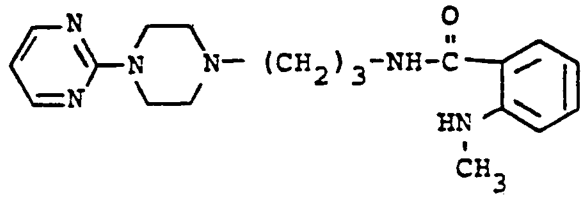 | 102—103° (aus Essigester/ Isopropylether) | 77 |
| | N-(4-(4-(2-Pyrimidinyl)-1-piperazinyl)butyl)-2-methylaminobenzoesäureamid | | |
| 44 | 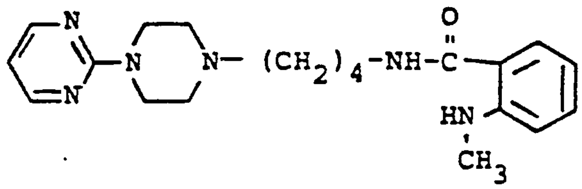 | 214—215° (Dihydrochlorid) | 65 |

Beispiel 45

Darstellung von N-(4-(4-(2-Pyrimidinyl)-1-piperazinyl)butyl)-2-nitro-benzolsulfonsäureamid

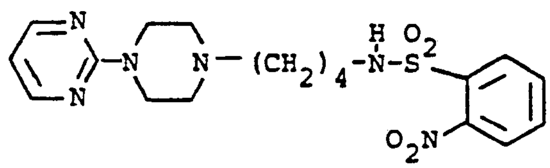

0,1 Mol 1-(4-Aminobutyl)-4-(2-pyrimidinyl)piperazin werden in 150 ml Toluol gelöst, mit 100 ml 20%iger NaOH versetzt und unter starkem Rühren und Eiskühlung 0,1 Mol 2-Nitro-benzolsulfonylchlorid, gelöst in 150 ml Toluol, innerhalb 20 Minuten zugetropft.

2 Stunden bei Raumtemperatur gerührt, mit 2 n HCl angesäuert und vom Toluol abgetrennt. Die wäßrige Phase wird ammoniakalisch gestellt, mit Essigester ausgeschüttelt und die organische Phase eingeengt. Rückstand aus Essigester/Isopropylether kristallisiert.

Ausbeute: 77% der Theorie, Fp.: 102,3°C

Analog wurden dargestellt:

| Beispiel Nr. | Formel | Fp (°C) | Ausbeute % d.Th. |
|---|---|---|---|
| | N-(3-(4-(2-Pyrimidinyl)-1-piperazinyl)propyl)-2-nitrobenzolsulfonsäureamid | | |
| 46 | 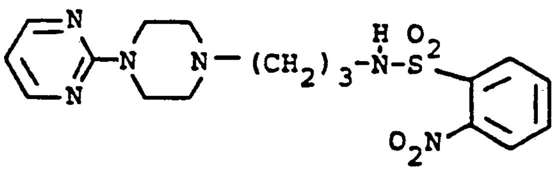 | 121,7° | 78 |

Beispiel 47

Darstellung von N-(4-(4-(2-Pyrimidinyl)-1-piperazinyl)butyl)-2-aminobenzosulfonsäureamid

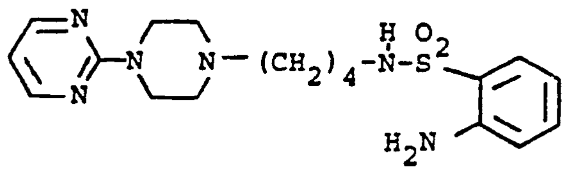

0,06 Mol N-(4-(4-(2-Pyrimidinyl)-1-piperazinyl)butyl)-2-nitrobenzolsulfonsäureamid werden in 500 ml MeOH gelöst, 2,4 g Pd-C, 5%ig, zugegeben, zum Sieden erhitzt und 0,17 Mol Hydrazinhydrat in 50 ml MeOH innerhalb 15 Minuten zugetropft. Nach weiteren 30 Minuten Sieden wird abfiltriert, eingeengt, der Rückstand in Diethylether aufgenommen, mit Wasser gewaschen, wieder eingeengt und aus Isopropanol/Isopropylether kristallisiert.

Ausbeute: 86% der Theorie; Fp.: 82°C

Analog wurde dargestellt:

| Beispiel Nr. | Formel | Fp (°C) | Ausbeute % d.Th. |
|---|---|---|---|
| | N-(3-(4-(2-Pyrimidinyl)-1-piperazinyl)propyl)-2-aminobenzolsulfonsäureamid | | |
| 48 | 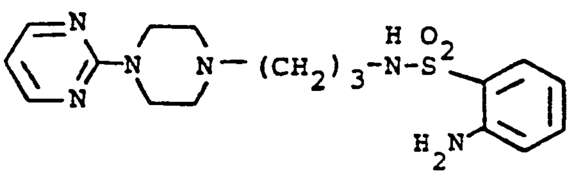 | 61,4° | 86 |

Beispiel 49

Darstellung von 1-(4-Aminobutyl)-4-(5-phenyl)-21-pyrimidinyl)-piperazin

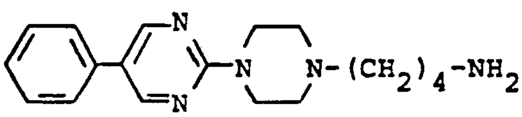

0,005 Mol 2-(4-(4-(5-Phenyl-2-pyrimidinyl)-1-piperazinyl(butyl)isoindol-1,3(2H)dion und 0,005 Mol Hydrazinhydrat werden in 25 ml Ethanol und 0,6 ml $H_2O$ bei 60 bis 70°C unter $N_2$-Atmosphäre 7 Stunden gerührt. Nach dem Abkühlen wird mit 7,5 ml 2n HCl angesäuert und mit 100 ml $H_2O$ verdünnt. Unlösliches weißes Produkt (Hydrazid) wird abfiltriert und die Lösung eingeengt. Der zurückbleibende Festkörper wird in Methylenchlorid/2n NaOH geschüttelt und extrahiert. Nach dem Trocknen der organischen Phase wird eingeengt. Der halbkristalline Rückstand wird mit Isopropylether verrieben und kristallisiert. Aus Cyclohexan wird rekristallisiert.

Ausbeute: 59% der Theorie; Fp.: 66—67°C,

Dihydrochlorid: Fp >250°C (Zers.)

Analog wurden hergestellt:

| Beispiel Nr. | Formel | Fp (°C) | Ausbeute % d.Th. |
|---|---|---|---|
| | 1-(4-Aminobutyl)-4-(5-(2-fluor-6-chlorphenyl)-2-pyrimidinyl)-piperazinhydrochlorid | | |
| 50 | 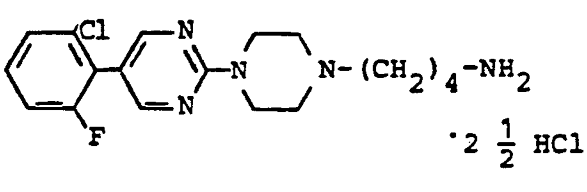 | 275° (Zers.) | 93 |

| Beispiel Nr. | Formel | Fp (°C) | Ausbeute % d. Th. |
|---|---|---|---|
| | 1-(4-Aminobutyl)-4-(5-(3,4-dimethoxyphenyl)-2-pyrimidinyl)piperazin | | |
| 51 | 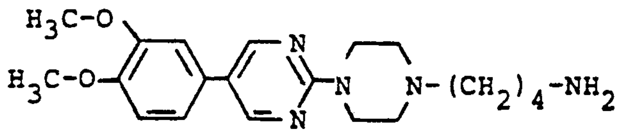 | 94—95°<br>·2HCl: 273—275° (Zers.) | 79 |
| | 1-(3-Aminopropyl)-4-(5-(4-chlorphenyl)-2-pyrimidinyl)piperazin | | |
| 52 | 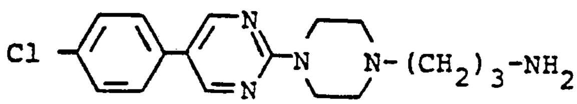 | 103—104° | 80 |
| | 1-(4-Aminobutyl)-4-(5-(3-indolyl)-2-pyrimidinyl)piperazin | | |
| 53 | 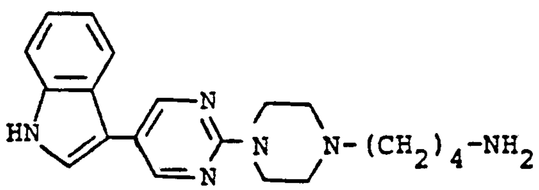 | 137—138° | 67 |

## Beispiel 54

Darstellung von Ethyl-4-(5-phenyl-2-pyrimidinyl)-1-piperazincarbxylat

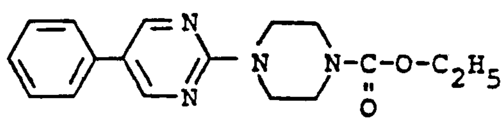

0,1 Mol Ethyl-4-(aminoiminomethyl)-1-piperazincarboxylat und 0,1 Mol N-(3-Dimethylamino-2-(phenyl)-2-propenyliden)-N-methylmethaniminiumperchlorat werden in 400 ml $CH_3OH$ mit 50 ml 2 n $NaOCH_3$-Lösung versetzt. Nach einer halben Stunde werden weitere 50 ml 2 n $NaOCH_3$-Lösung zugegeben. Dann wird 3 Stunden unter Rückfluß gekocht. Danach engt man bis zur Trockne ein, versetzt mit Wasser und nimmt die organische Phase in $CH_2Cl_2$ auf. Nach Einengen wird der Rückstand kristallisiert.

Ausbeute: 59% der Theorie; Fp.: 123°C (aus Isopropanol).

Analog wurden hergestellt:

| Beispiel Nr. | Formel | Fp (°C) | Ausbeute % d.Th. |
|---|---|---|---|
| | Ethyl-4-(5-(2-chlor-6-fluorphenyl)-2-pyrimidinyl)-1-piperazincarboxylat | | |
| 55 | 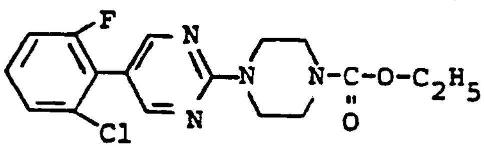 | 106—107° | 46 |
| | Ethyl-4-(3,4-dimethoxyphenyl)-2-pyrimidinyl)-1-piperazincarboxylat | | |
| 56 | 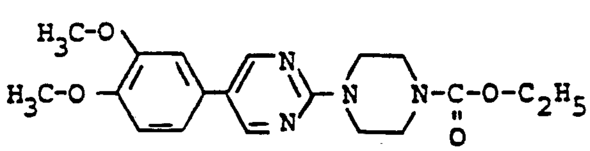 | 165—166° (aus Ethanol) | 66 |

| Beispiel Nr. | Formel | Fp (°C) | Ausbeute % d.Th. |
|---|---|---|---|
| | Ethyl-4-(5-(4-Chlorphenyl)-2-pyrimidinyl)-1-piperazincarboxylat | | |
| 57 | 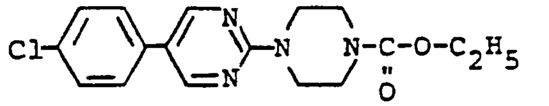 | 133—134° (aus Ethanol) | 60 |
| | Ethyl-4-(5-(3-indolyl)-2-pyrimidinyl)-1-piperazincarboxylat | | |
| 58 | 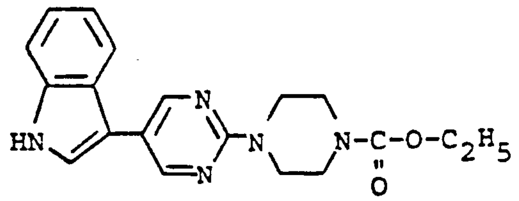 | 155—156° (aus Isopropanol) | 55 |
| | Ethyl-4-(5-(3-trifluorophenyl)-2-pyrimidinyl)-1-piperazincarboxylat | | |
| 59 | 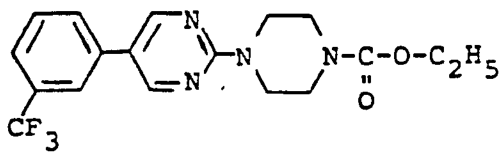 | 123—124° | 55 |

Beispiel 60

Darstellung von 1-(5-Phenyl-2-pyrimidinyl)-piperazin

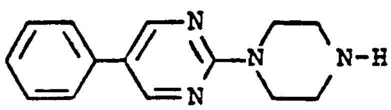

0,04 Mol Ethyl-1-(5-phenyl-2-pyrimidinyl)-4-piperazincarboxylat werden mit 0,78 Mol KOH in 400 ml $C_2H_5OH$ und 40 ml $H_2O$ under $N_2$-Schutzgas 15 Stunden unter Rückfluß gekocht. Der Ansazt wird bis zur Trockne eingeengt, mit Wasser versetzt und der Rückstand in $CH_2Cl_2$ aufgenommen. Nach dem Waschen mit Wasser wird die organische Phase abgetrennt und zur Trockne eingedampft, wobei das Amin kristallin anfällt.

Ausbeute: 90% der Theorie; Fp.: 107—108°C

Analog wurden dargestellt:

| Beispiel Nr. | Formel | Fp (°C) | Ausbeute % d.Th. |
|---|---|---|---|
| | 1-(5-(2-Fluor-6-chlorphenyl)-2-pyrimidinyl)piperazin | | |
| 61 | 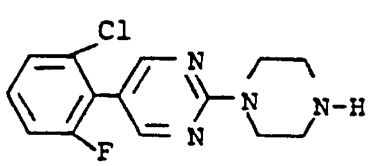 | 87° | 79 |
| | 1-(5-(3,4-Dimethoxyphenyl)-2-pyrimidinyl)piperazin | | |
| 62 | 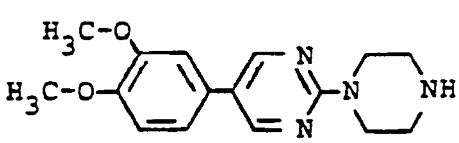 | 140—141° (aus Essigester) Dihydrochlorid: 298—300° | 85 |

| Beispiel Nr. | Formel | Fp (°C) | Ausbeute % d.Th. |
|---|---|---|---|
| | 1-(5-(3-Trifluorphenyl)-2-pyrimidinyl)piperazin | | |
| 63 | 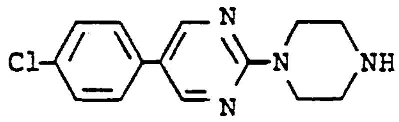 | 157—158° (aus Ethanol) | 83 |
| | 1-(5-(3-Indolyl)-2-pyrimidinyl)piperazin | | |
| 64 | 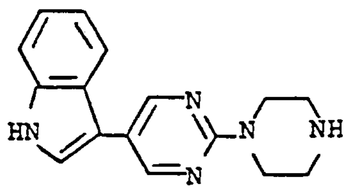 | 202—202° (aus Isopropanol) | 53 |
| | 1-(5-(4-Chlorphenyl)-2-pyrimidinyl) piperazin | | |
| 65 | 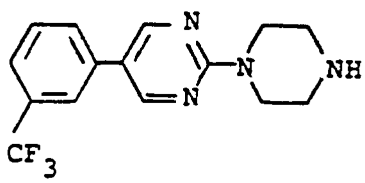 | 98—99° | 79 |

Beispiel 66

Darstellung von 1-(5-Jod-2-pyrimidinyl)-piperazin

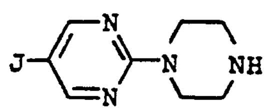

0,02 Mol 2-Chlor-5-jodpyrimidin, 0,06 Mol Piperazin und 100 ml absolutes Dimethylformamid werden 45 Minuten auf 100° erhitzt und dann eingeengt. Der kristalline Rückstand wird mit Kaliumbicarbonatlösung und Methylenchlorid geschüttelt. Man reinigt die in der Methylenchloridphase enthaltene Substanz durch Säulenchromatographie über Kieselgel 60. Das durch Eluieren mit Methylenchlorid/Methanol (7:3) erhaltene Amin wird mit Isopropylether verrührt und abgesaugt.

Fp: 135—136°C

Ausbeute: 72% der Theorie.

Analog wurden dargestellt:

1-(5-Fluor-2-pyrimidinyl)piperazin

67 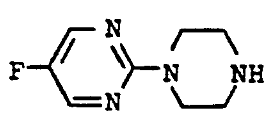

Fp = 39—40°C,

Ausbeute: 32% der Theorie.

Beispiel 68

Darstellung von 2-(4-Brombutyl)-5-chlor-1,2-benzoisothiazol-3(2H)on-1,1-dioxid

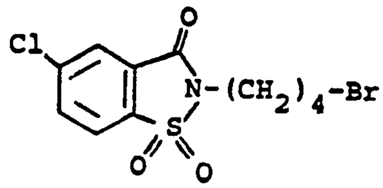

0,05 mol 5-Chlor-1,2-benzoisothiazol-3(2H)on-1,1-dioxid werden zu einer Suspension von 0,05 Mol NaH in 100 ml DMF gegeben. Danach werden 0,2 Mol 1,4-Dihrombutan zugegeben. Bei 100°C läßt man den Ansatz 1 Stunde rühren. Der Ansazt wird eingeengt und der Rückstand über Kieselgel 60 gereinigt, wobei zunächst mit Cyclohexan, dann mit Methylenchlorid/Methanol (98:2) eluiert wird.

Ausbeute: 65% der Theorie; Fp.: 72—73°C (aus Isopropanol).

Analog wurden hergestellt:

| Beispiel Nr. | Formel | Fp (°C) | Ausbeute % d.Th. |
|---|---|---|---|
| | 2-(4-Brombutyl)-6-chlor-1,2-benzoisothiazol-3(2H)on-1,1-dioxid | | |
| 69 | 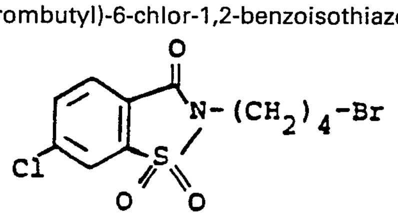 | 107° | 73 |
| | 2-(4-Brombutyl)-5-fluor-1,2-benzoisothiazol-3(2H)on-1,1-dioxid | | |
| 70 | 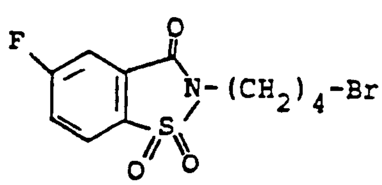 | 75° | 54 |
| | 2-(4-Brombutyl)-6-fluor-1,2-benzoisothiazol-3(2H)on-1,1-dioxid | | |
| 71 | 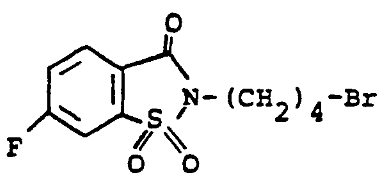 | 72—73° | 78 |
| | 2-(4-Brombutyl)-6-nitro-1,2-benzoisothiazol-3(2H)on-1,1-dioxid | | |
| 72 | 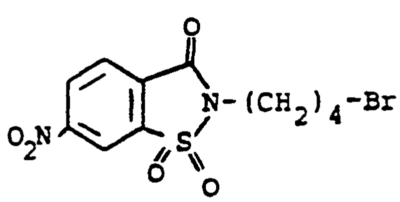 | 120—121° | 66 |
| | 2-(4-Brombutyl)-7-chlor-1,2,4-benzothiadiazin-3(4H)on-1,1-dioxid | | |
| 73 | 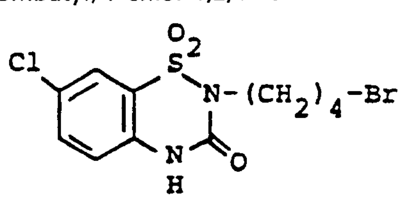 | 147,2° (aus Isopropylether) | 15 |

Beispiel 74

Darstellung von 2-(4-(4-(2-Pyrimidinyl)-1-piperazinyl)butyl)-6-amino-1,2-benzoisothiazol-3(2H)on-1,1-dioxid

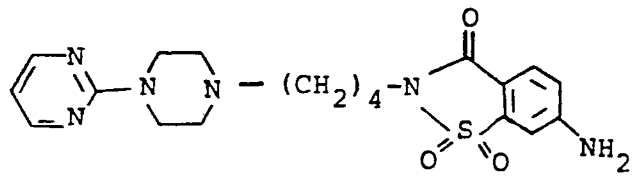

0,03 mol 2-(4-(4-(2-Pyrimidinyl)-1-piperazinyl)butyl)-6-nitro-1,2-benzoisothiazol-3(2H)on-1,1-dioxid werden in 50 ml konzentrierter Salzsäure gelöst und mit einer Lösung von 0,13 Mol $SnCl_2 \cdot 2\,H_2O$ in 100 ml konzentrierter Salzsäure versetzt. Nach Beendigung der oxothermen Reaktion wird 30 Minuten nachgerührt. Man gibt den Ansatz auf Eis, filtriert und wäscht das Kristallisat mit Wasser. Nach Behandlung mit verdünnter Natronlauge schüttelt man die Base mit Methylenchlorid aus, dampft das Lösungsmittel ab und erhält das Produkt als farbloses Kristallisat.

Beispiel 75

Darstellung von 2-(4-(4-(2-Pyrimidinyl)-1-piperazinyl)butyl)-6-acetamido-1,2-benzoisothiazol-3(2H)on-1,1-dioxid

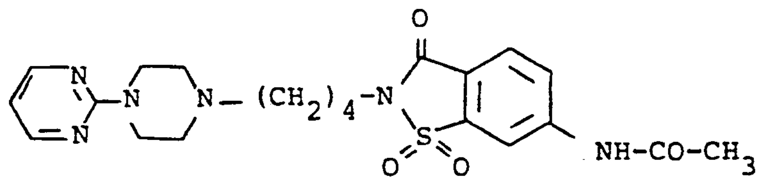

0,06 Mol 2-(4-(4-(2-Pyrimidinyl)-1-piperazinyl)-butyl)-6-amino-1,2-benzoisothiazol-3(2H)on-1,1-dioxid werden mit 200 ml Essigsäureanhydrid versetzt und 12 Stunden bei Raumtemperatur gerührt. Danach engt man bis zur Trockne ein, versetzt mit Isopropylether und rührt 1 Stunde. Anschließend wird filtriert und das farblose Kirstallisat im Vakuum getrocknet.

Ausbeute: 91% Theorie; Fp. 162°C.

Beispiel 76

Darstellung von 2-(4-(4-(2-Pyrimidinyl)-1-piperazinyl)butyl)-6-ethoxycarbonylamino-1,2-benzoisothiazol-3(2H)on-1,1-dioxid

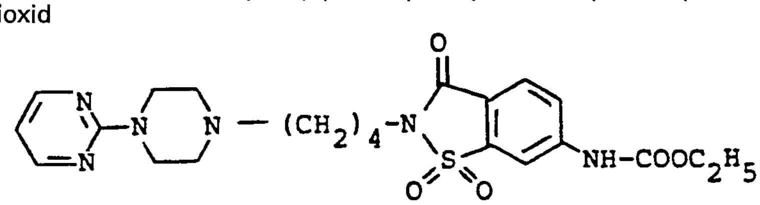

0,01 Mol 2-(4-(4-(2-Pyrimidinyl)-1-piperazinyl)butyl-6-amino-1,2-benzoisothiazol-3(2H)on-1,1-dioxid werden in 30 ml absolutem Pyridin gelöst und unter Eiskühlung mit 0,02 Mol Chlorameisensäurealkylester versetzt. Anschließend läßt man den Ansatz auf Raumtemperatur kommen und rührt 12 Stunden nach. Man dampft das Lösungsmittel ab, versetzt mit Eiswasser und schüttelt mehrmals mit Methylenchlorid aus. Nach abdampfen des Lösungsmittels erhält man ein farbloses Kristallisat.

Ausbeute: 53% der Theorie; Fp. 160°C.

Beispiel 77

Darstellung von 2-(4-(4-(2-Pyrimidinyl)-1-piperazinyl)butyl)-6-bis(methansulfonyl)amino-1,2-benzoisothiazol-3(2H)on-1,1-dioxid

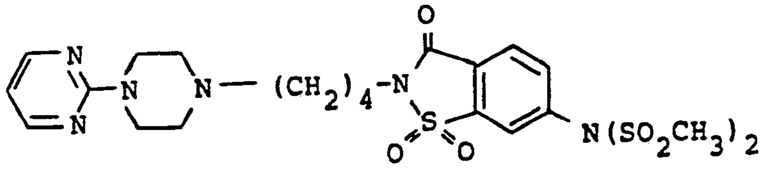

0,01 Mol 2-(4-(4-(2-Pyrimidinyl)-1-piperazinyl)butyl)-6-amino-1,2-benzoisothiazol-3(2H)-1,1-dioxid werden in 50 ml absolutem Pyridin gelöst und unter Eiskühlung mit 0,023 Mol Methansulfonsäurechlorid versetzt. Anschließend läßt man den Ansatz auf Raumteperatur kommen und rührt 12 Stunden nach. Man dampft das Lösungsmittel ab, versetzt mit verdünnter Natronlauge und schüttelt mehrmals mit Methylenchlorid aus. Zur Reinigung chromatographiert man an Kieselgel und eluiert mit $CH_2Cl_2/CH_3OH$ (10:1).

Ausbeute: 23% der Theorie; Fp.: 167°C (Zers.).

Beispiel 78

Darstellung von 2-(3-(4-(2-Pyrimidinyl)-1-piperazinyl)propyl-5-fluor-1,2-benzoisothiazol-3(2H)on-1,1-dioxid

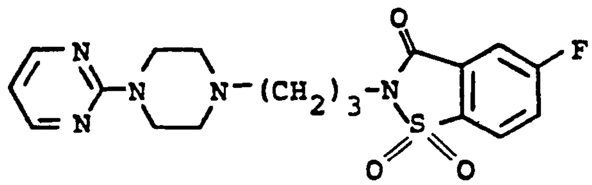

0,03 Mol 5-Fluor-2-(3-brompropyl)-1,2-benzoisothiazol-3(2H)on-1,1-dioxid und 0,03 Mol 1-(2-Pyrimidinyl)piperazin werden mit 0,03 Mol $K_2CO_3$ in 100 ml absolutem DMF 2 Stunden bei 50 bis 60°C gerührt. Danach eingt man ein, versetzt den Rückstand mit Wasser und extrahiert die Base mit Methylenchlorid. Zur Reinigung wird an Kieselgel chromatographiert; Elutionsmittel ist $CH_2Cl_2/CH_3OH$ (98:2), Umkristallisation erfolgt aus Toluol.

Ausbeute: 20% der Theorie; Fp.: 117 bis 118°C.

Analog wurden hergestellt:

| Beispiel Nr. | Formel | Fp (°C) | Ausbeute % d.Th. |
|---|---|---|---|
| | 2-(3-(4-(5-(4-Chlorphenyl)-2-pyrimidinyl)-1-piperazinyl)propyl)-1,2-benzoisothiazol-3(2H)on-1,1-dioxid | | |
| 79 | 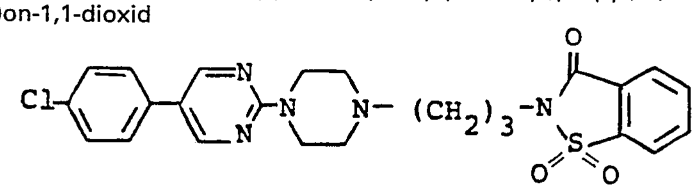 | 185° | 52 |
| | 2-(2-(4-(5-(4-Chlorphenyl)-(2-pyrimidinyl)-1-piperazinyl)ethyl)-1,2-benzoisothiazol-3(2H)on-1,1-dioxid | | |
| 80 | 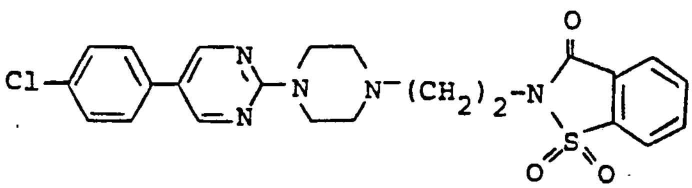 | 186° | 10 |

In Analogie zu Beispiel 18 wurden hergestellt:

| Beispiel Nr. | Formel | Fp (°C) | Ausbeute % d.Th. |
|---|---|---|---|
| | 2-(3-(4-(2-Pyrimidinyl)-1-piperazinyl)-2-hydroxypropyl)-1,2-benzoisothiazol-3(2H)on-1,1-dioxid | | |
| 81 | 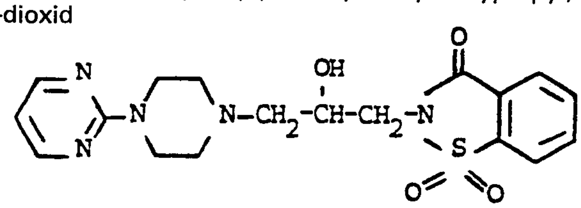 | 108° | 42 |
| | 2-(3-(4-(5-(4-Chlorphenyl)-2-pyrimidinyl)-1-piperazinyl)-2-hydroxypropyl)-1,2-benzo-isothiazol-3(2H)on-1,1-dioxid | | |
| 82 | 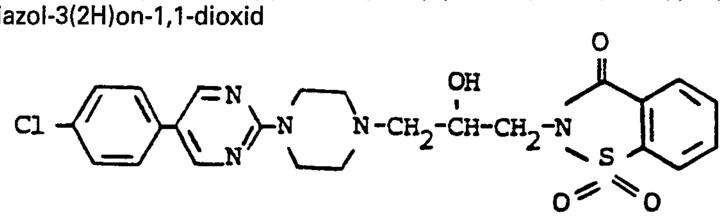 | 159° | 28 |

Beispiel 83

Darstellung von 2-(4-(4-(2-Pyrimidinyl)-1-piperazinyl)butyl)-1,3,2-benzodithiazol-1,1,3,3-tetroxid

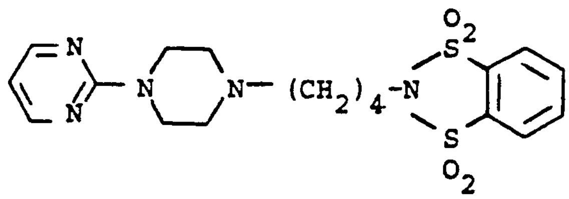

0,028 Mol 2-(4-Brombutyl)-1,3,2-benzodithiazol-1,1,3,3-tetroxid und 0,056 Mol 1-(2-Pyrimidinyl)-piperazin werden in 125 ml absolutem DMF 6 Stunden bei 25°C gerührt. Man kühlt dann auf 0°C ab, tropft unter Rühren 150 ml Wasser hinzu und saugt die auskristallisierte Substanz ab. Zur Reinigung wird das Produkt am Kieselgel chromatographiert; Elutionsmittel ist Essigsäureethylester.

Ausbeute: 31% der Theorie; Fp.: 178°C.

Beispiel 84

Darstellung von 2-(4-Brombutyl)-1,3,2-benzodithiazol-1,1,3,3-tetroxid

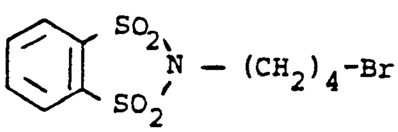

0,13 Mol 1,2-Benzoldisulfonylchlorid in 720 ml absolutem Toluol werden unter Rühren bei 25°C tropfenweise mit einer Lösung von 0,65 Mol Ammoniak in 1050 ml absolutem Ethanol versetzt. Es wird noch eine Stunde nachgerührt, eine geringe Trübung durch Filtration über eine Kieselgurschicht entfernt und das Filtrat zur Trockne eingedampft. Man er hält das Ammoniumsalz des 1,3,2-Benzodithiazol-1,1,3,3-tetroxid.

Ausbeute: 91% der Theorie; Fp.: 246 bis 247°C.

0,06 Mol des Ammoniumsalzes und 0,25 Mol 1,4-Dibrombutan werden in 130 ml absolutem DMF 5 Stunden bei 130 bis 140°C gerührt. Nach dem Erkalten wird am Rotationsverdampfer eingeengt und restliches 1,4-Dibrombutan durch Wasserdampfdestillation entfernt. Das Reaktionsprodukt wird in Methylenchlorid aufgenommen, an Kieselgel säulenchromatographisch (Elutionsmittel $CH_2Cl_2$/Cyclohexan (7:3)) gereinigt und zuletzt aus Isopropanol umkristallisiert.

Ausbeute: 35% der Theorie; Fp.: 82 bis 83°C.

Beispiel 85

Darstellung von 2-(3-(4-(2-Pyrimidinyl)-1-piperazinyl)propyl)-7-methoxy-1,2,4-benzothiadiazin-3(4H)on-1,1-dioxid

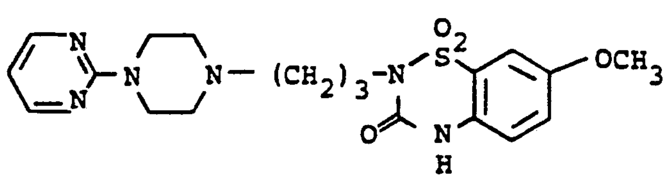

0,01 Mol 2-(3-Brompropyl)-7-methoxy-1,2,4-benzothiadiazin-3(4H)on-1,1-dioxid, 0,012 Mol 1-(2-Pyrimidinyl)-piperazin und 0,01 Mol Triethylamin werden in 30 ml DMF gelöst und unter Rühren 1,5 Stunden auf 60°C erwärmt. Danach werden 15 ml Wasser zugesetzt und die Reaktionsmischung auf 0°C abgekühlt. Die hierbei auskristallisierende Substanz wird abgesaugt, mit Isopropanol verrührt, erneut abgesaugt und im Vakuum getrocknet.

Ausbeute: 53% der Theorie; Fp.: 178°C.

Analog wurden hergestellt:

| Beispiel Nr. | Formel | Fp (°C) | Ausbeute % d.Th. |
|---|---|---|---|
| 86 | 2-(3-(4-(2-Pyrimidinyl)-1-piperazinyl)propyl)-4-phenyl-1,2,4-benzothiadiazin-3(4H)on-1,1-dioxid 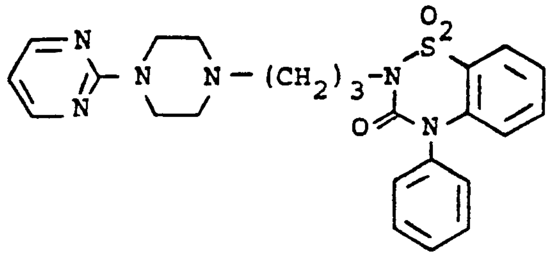 | 129° | 84 |
| 87 | 2-(4-(4-(2-Pyrimidinyl)-1-piperazinyl)butyl)-4-phenyl-1,2,4-benzothiadiazin-3(4H)on-1,1-dioxid 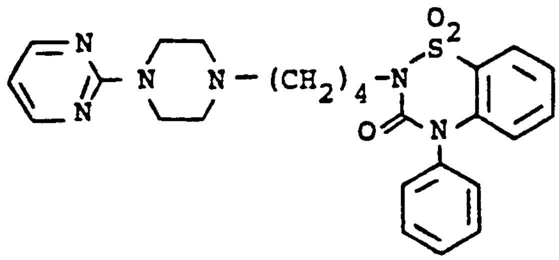 | 227° | 76 |
| 88 | 2-(3-(4-(2-Pyrimidinyl)-1-piperazinyl)propyl)-7-chlor-1,2,4-benzothiadiazin-3(4H)on-1,1-dioxid 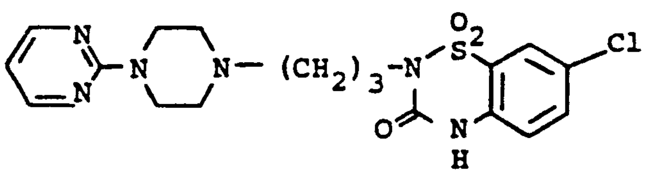 | 208° | 55 |

Beispiel 89

Darstellung von 2-(3-(4-(2-Pyrimidinyl)-1-piperazinyl)propyl)-4-methyl-7-methoxy-1,2,4-benzothiadiazin-3(4H)on-1,1-dioxid

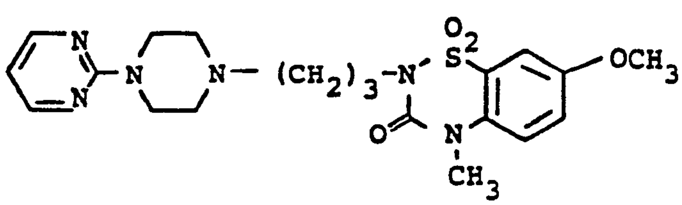

0,028 Mol 2-(3-(4-(2-Pyrimidinyl)-1-piperazinyl)propyl)-7-methoxy-1,2,4-benzothiadiazin-3(4H)on-1,1-dioxid werden in 150 ml absolutem DMF gelöst, die Lösung mit 0,028 Mol Natriumhydrid versetzt und 0,25 Stunden bei Raumtemperatur gerührt. Danach werden 0,028 Mol Methyliodid zugesetzt und 2 weitere Stunden bie 25°C gerührt. Durch tropfenweise Zugabe von insgesamt 18 ml Wasser scheidet sich das Produkt kristallin aus. Zur Reinigung kristallisiert man aus Isopropanol um.

Ausbeut: 40% der Theorie; Fp.: 146°C.

Analog wurden hergestellt:

| Beispiel Nr. | Formel | Fp (°C) | Ausbeute % d.Th. |
|---|---|---|---|
| | 2-(3-(4-(2-Pyrimidinyl)-1-piperazinyl)propyl)-7-chlor-4-methyl-1,2,4-benzothiadiazin-3(4H)on-1,1-dioxid | | |
| 90 | 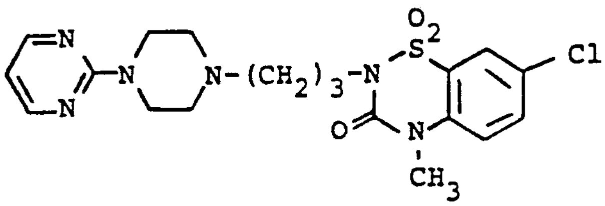 | 128° | 72 |

Beispiel 91

Darstellung von 2-(3-Brompropyl)-7-methoxy-1,2,4-benzothiadiazin-3(4H)on-1,1-dioxid

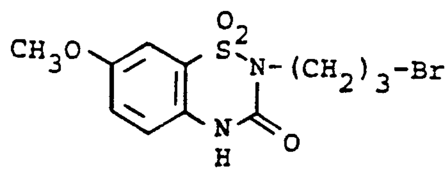

0,03 Mol 7-Methoxy-1,2,4-benzothiadiazin-3(4H)on-1,1-dioxid werden in 50 ml absolutem DMF gelöst und mit 0,03 Mol Natriumhydrid versetzt. Nach 15 Minuten war die Natriumsalzbildung beendet. Man tropft anschließend 0,06 Mol 1,3-Dibrompropan hinzu und erwärmt 2 Stunden auf 80°C. Danach wird das Lösungsmittel abdestilliert und der Rückstand durch Säulenchromatographie an Kieselgel gereinigt. Als Elutionsmittel wird ein $CH_2Cl_2$($CH_3OH$-Gemisch (98:2) verwendet.

Ausbeute: 46% der Theorie; Fp.: 166°C.

Analog wurden hergestellt:

| Beispiel Nr. | Formel | Fp (°C) | Ausbeute % d.Th. |
|---|---|---|---|
| | 2-(3-Brompropyl)-4-phenyl-1,2,4-benzothiadiazin-3(4H)-on-1,1-dioxid | | |
| 92 | 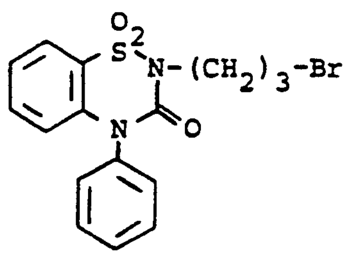 | 138° | 48 |
| | 2-(4-Brombutyl)-4-phenyl-1,2,4-benzothiadiazin-3(4H)on-1,1-dioxid | | |
| 93 | 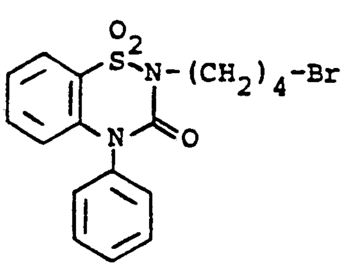 | 93° | 51 |

Beispiel 94

Darstellung von N-(4-(4-(2-Pyrimidinyl)-1-piperazinyl)butyl)-2-(acetylamino)benzolsulfonsäureamid

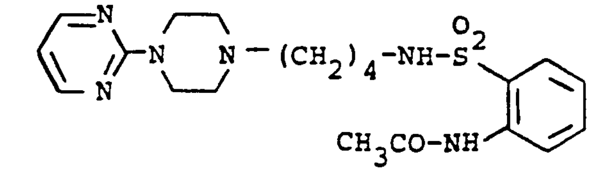

0,02 Mol N-(4-(4-(2-Pyrimidinyl)-1-piperazinyl)butyl)-2-aminobenzolsulfonsäureamid und 0,5 Mol Essigsäureanhydrid werden 2 Stunden bei Raumtemperatur gerührt. Man dampft zur Trockene ein, übergießt den Rückstand mit konzentrierter wäßriger Ammoniaklösung und schüttelt die Base mit Methylenchlorid aus. Zur Reinigung wird an Kieselgel chromatographiert; Elutionsmittel ist $CH_2Cl_2/CH_3OH$ (9:1). Die Substanz wird aus Isopropanol umkristallisiert.

Ausbeute: 37% der Theorie; Fp.: 99°C.

Beispiel 95

Darstellung von N-Acetyl-N-(4-(4-(2-Pyrimidinyl)-1-piperazinyl)butyl)-2-(acetylamino) benzolsulfonsäureamid

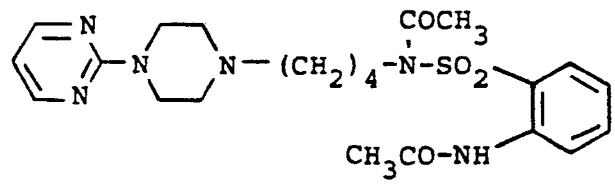

0,01 Mol N-(4-(4-(2-Pyrimidinyl)-1-piperazinyl)butyl)-2-aminobenzolsulfonsäureamid werden in 0,25 Mol Essigsäureanhydrid gelöst und 3 Stunden auf 100°C erhitzt. Man dampft zur Trockene ein, übergießt den Rückstand mit konzentrierter wäßriger Ammoniaklösung und schüttelt die Base mit Methylenchlorid aus. Zur Reinigung wird aus Isopropanol umkristallisiert.

Ausbeute: 47% der Theorie; Fp.: 152°C.

Beispiel 96

Darstellung von 1-(3-Nitrophenyl)-3-(3-(4-(2-Pyrimidinyl)-1-piperazinyl)-propyl)chinazolin-2,4(1H,3H)dion

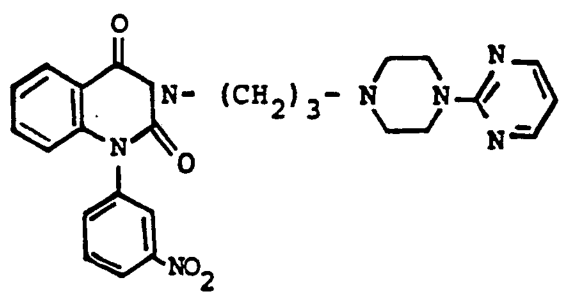

0.0874 Mol 2-(3-Nitrophenylamino)-benzoylchlorid in 650 ml trockenem Dioxan werden mit 0.096 Mol 1-(3-Aminopropyl)-4-(2-pyrimidinyl)-piperazin versetzt und 1 Stunde unter Rückfluß erhitzt. Die beim Abkühlen ausgefallenen Kristalle werden abfiltriert, in Wasser gelöst, die wässrige Phase mit Kaliumhydrogencarbonat versetzt und mit Dichlormethan extrahiert. Die organische Phase wird über Natriumsulfat getrocknet, eingedampft und der Trockenrückstand mit Diisopropylether und Petrolether ausgerührt.

Ausbeute an substituiertem Glutamid: 44% d. Th.; Fp 101—03°C.

0.0076 Mol des vorstehend beschriebenen Amids werden in 75 ml trockenem Tetrahydrofuran suspendiert, mit 0,0152 Mol Natriumhydrid und nach beendeter Wasserstoffentwicklung mit 0,0228 Mol Carbonyldiimidazol versetzt und das breiige Reaktionsgemisch 1 Stunde bei Raumtemperatur gerührt. Man hydrolysiert das Reaktionsgemisch durch Zugabe von Wasser, extrahiert das Produkt mit Dichlormethan, kristallisiert den nach Verdampfen des Lösungsmittels verbleibenden Rückstand aus Dichlormethan/Petrolether um und trocknet das kristalline Produkt 3 Tage bei 70°C i. Vakuum.

Ausbeute: 67,5% d. Th., Fp 100—115°C.

**Patentansprüche**

1. 2-Pyrimidinyl-1-piperazin-Derivate der allgemeinen Formel

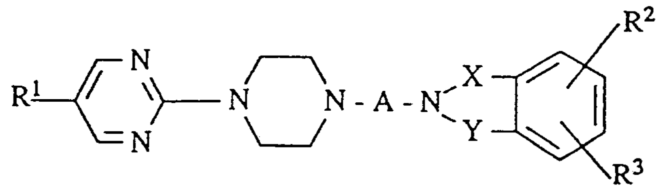

(I)

worin

A für eine Alkylengruppe mit 1 bis 6 Kohlenstoffatomen oder für 2-Hydroxy-n-propylen steht, wobei A nicht für die 2-Hydroxy-n-propylengruppe steht, wenn X und Y für Carbonyl und $R^1$, $R^2$ und $R^3$ für Wasserstoff stehen.

$R^1$ für Wasserstoff, Trifluormethyl oder für Alkoxy mit 1 bis 4 Kohlenstoffatomen, Trifluormethoxy oder für Hydroxy, Halogen, Cyano, Nitro, Amino, gegebenenfalls substituiert durch 1 oder 2 Methyl oder Ethyl, oder für Acetylamino oder Benzoylamino, oder für Indol-3-yl, oder für Phenyl steht, wobei der Phenylrest einfach oder mehrfach durch Methoxy, Ethoxy, Nitro, Halogen, Trifluormethyl, Amino, Cyano, Hydroxy, Methyl oder Ethyl, Acetylamino oder Benzoylamino substituiert sein kann,

$R^2$ und $R^3$ gleich oder verschieden sein können und für Wasserstoff oder Alkoxy mit 1 bis 4 Kohlenstoffatomen, oder für Phenyl, oder für Phenyl, oder für Hydroxy, Halogen, Cyano, Nitro, Amino, wobei das Stickstoffatom des Aminorestes durch 1 oder 2 Methyl oder Ethyl substituiert ist, oder für Methylsulfonamide, Benzol- und Toluolsulfonamido, Acetylamino, Ethoxycarbonylamino Carboxyl, Methoxy- oder Ethoxycarbonyl, oder für Phenoxycarbonylamino, oder für Carbamoyl oder Sulfamoyl steht,

X und Y gleich oder verschieden sein können und Carbonyl oder Sulfonyl bedeuten, und

X allein für Carbonyl oder Sulfonyl steht, wenn Y gleichzeitig $—CO—CH_2$ oder $—CO—N(R^4)—$ bedeutet, wobei $R^4$ für Wasserstoff, Methyl oder Phenyl steht, sowie deren Säureadditionssalze.

2. 2-Pyrimidinyl-1-piperazin-Derivate der allgemeinen Formel nach Anspruch 1, in welcher

A für Ethylen, n-Propylen, n-Butylen, 2-Methyl-n-propylen oder 2-Hydroxy-n-propylen steht, wobei A nicht für die 2-Hydroxy-n-propylen-gruppe steht, wenn X und Y für Carbonyl und $R^1$, $R^2$ und $R^3$ für Wasserstoff stehen,

$R^1$ für Wasserstoff oder für Methoxy, Ethoxy oder für Fluor, Iod, Cyano oder für Indol-3-yl, oder für Phenyl steht, wobei der Phenylrest einfach oder mehrfach durch Methoxy oder Ethoxy, Chlor, Fluor, Trifluormethyl, Cyano substituiert sein kann,

$R^2$ und $R^3$ gleich oder verschieden sein können und für Wasserstoff, oder für Methoxy, Trifluormethoxy oder für Chlor, Fluor, Cyano, Nitro, Amino, wobei das Stickstoffatom des Aminorestes durch 1 oder 2 Methyl oder Ethyl substituiert ist, oder für Methylsulfonamido, Acetylamino, Ethoxycarbonylamino steht,

X und Y gleich oder verschieden sein können und Carbonyl oder Sulfonyl bedeuten, und

X allein für Carbonyl oder Sulfonyl steht, wenn Y gleichzeitig $—CO—CH_2—$ oder $—CO—N(R^4)—$ bedeutet, wobei $R^4$ für Wasserstoff oder Phenyl steht, sowie deren Säureadditionssalze.

3. 2-Pyrimidinyl-1-piperazin-Derivate der allgemeinen Formel nach Anspruch 1, in welcher

A Ethylen, n-Propylen, n-Butylen oder 2-Hydroxy-n-propylen bedeutet wobei A nich für 2-Hydroxy-n-propylen steht, wenn X und Y für Carbonyl und $R^1$, $R^2$ und $R^3$ für Wasserstoff stehen,

$R^1$ Wasserstoff, Chlor, Fluor, Iod, 4-Methoxy-phenyl, 3,4-Dimethoxyphenyl, Phenyl, 4-Chlor-phenyl, 3-(Trifluormethyl)-phenyl, 2-Chlor-6-fluor-phenyl oder Indol-3-yl bedeutet,

$R^2$ und $R^3$ gleich oder verschieden sein können und für Wasserstoff, Fluor, Chlor oder Nitro stehen,

X für Carbonyl und Sulfonyl steht und

Y für Carbonyl, Sulfonyl, $—CO—CH_2—$ oder $—CO—N(R^4)—$ steht, wobei $R^4$ Wasserstoff, Phenyl oder Methyl bedeutet, sowie deren Säureadditionssalze.

4. 2-(4-(4-(2-Pyrimidinyl)-1-piperazinyl)butyl)-1,2-benzoisothiazol-(2H)on-1,1-dioxid

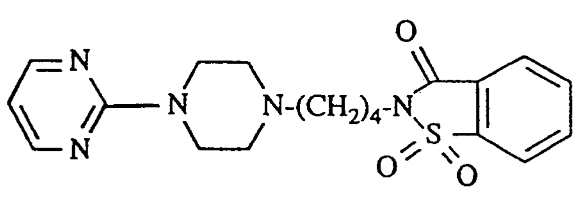

sowie das entsprechende Hydrochlorid.

5. 2-(3-(4-(2-Pyrimidinyl)-1-piperazinyl)propyl)-1,1-benzoisothiazol-3(2H)on-1,1-dioxid

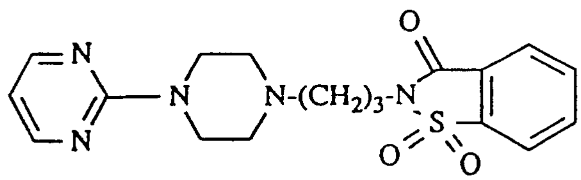

sowie das entsprechende Hydrochlorid.

6. Verfahren zur Herstellung von 2-Pyrimidinyl-1-piperazin-Derivaten der allgemeinen Formel

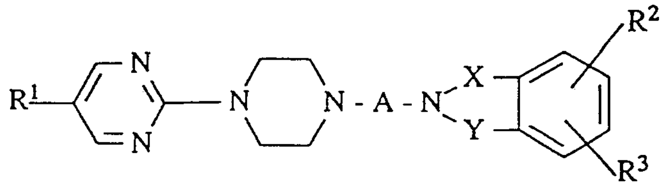

(I)

worin

A für eine Alkylengruppe mit 1 bis 6 Kohlenstoffatomen oder für 2-Hydroxy-n-propylen steht, wobei A nicht für die 2-Hydroxy-n-propylengruppe steht, wenn X und Y für Carbonyl und $R^1$, $R^2$ und $R^3$ für Wasserstoff stehen.

$R^1$ für Wasserstoff, Trifluormethyl oder für Alkoxy mit 1 bis 4 Kohlenstoffatomen, Trifluormethoxy oder für Hydroxy, Halogen, Cyano, Nitro, Amino, gegebenenfalls substituiert durch 1 oder 2 Methyl oder Ethyl, oder für Acetylamino oder Benzoylamino, oder für Indol-3-yl, oder für Phenyl steht, wobei der Phenylrest einfach oder mehrfach durch Methoxy, Ethoxy, Nitro, Halogen, Trifluormethyl, Amino, Cyano, Hydroxy, Methyl oder Ethyl, Acetylamino oder Benzoylamino substituiert sein kann,

$R^2$ und $R^3$ gleich oder verschieden sein können und für Wasserstoff oder Alkoxy mit 1 bis 4 Kohlenstoffatomen, oder für Phenyl oder für Hydroxy, Halogen, Cyano, Nitro, Amino, wobei das Stickstoffatom des Aminorestes durch 1 oder 2 Methyl oder Ethyl substituiert ist, oder für Methylsulfonamide, Benzol- und Toluolsulfonamido, Acetylamino, Ethoxycarbonylamino oder für Carboxyl, Methoxy- oder Ethoxycarbonyl, oder für Phenoxycarbonylamino, oder für Carbamoyl oder Sulfamoyl steht,

X und Y gleich oder verschieden sein können und Carbonyl oder Sulfonyl bedeuten, und

X allein für Carbonyl oder Sulfonyl steht, wenn Y gleichzeitig —CO—$CH_2$ oder —CO—N($R^4$)— bedeutet, wobei $R^4$ für Wasserstoff, Methyl oder Phenyl steht, sowie deren Säureadditionssalze, dadurch gekennzeichnet, daß man

a) Pyrimidinylpiperazin-Derivate der allgemeinen Formel

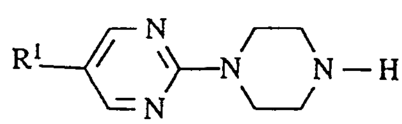

(II)

in welcher

$R^1$ die obengenannte Bedeutung hat,

mit Verbindungen der allgemeinen Formel

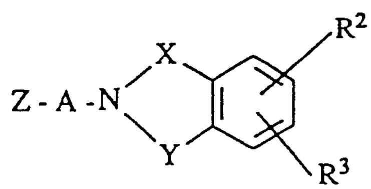

(III)

in welcher

$R^2$, $R^3$, X und Y die obengenannte Bedeutung haben und

Z für Hydroxy, Chlor, Brom, Iod, Methylsulfonyloxy oder 4-Tolylsulfonyloxy bedeutet, oder die Gruppe A—Z für

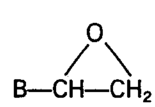

steht wobei

B für ein um zwei endständige C-Atome verkürztes Brückengleid A steht, in Gegenwart von inerten Lösungsmitteln bei Temperaturen zwischen 20 und 200°C alkyliert oder

b) Aminoalkylpyrimidinylpiperazin-Derivate der allgemeinen Formel

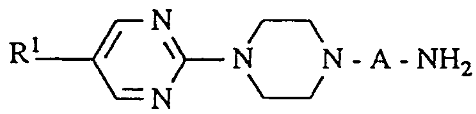

(IV)

in welcher

$R^1$ und A die obengenannte Beduetung haben, mit Anhydriden der allgemeinen Formel

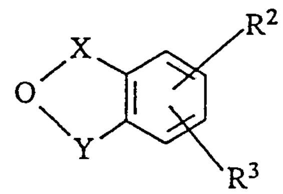

(V)

in welcher

$R^2$, $R^3$, X und Y die obengenannte Bedeutung haben, gleich oder verschieden sein können und Carbonyl oder Sulfonyl bedeuten, oder

X Carbonyl bedeutet, wenn Y gleichzeitig —CO—$CH_2$— bedeutet, bei einer Temperatur zwischen 100 und 250°C in einem inerten Lösungsmittel oder ohne Lösungsmittel in der Schmelze umsetzt, oder

c) Pyrimidinylpiperazin-Derivate der allgemeinen Formel

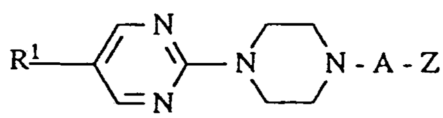

(VI)

in welcher

$R^1$, A und Z die obengenannte Bedeutung haben, mit Verbindungen der allgemeinen Formel

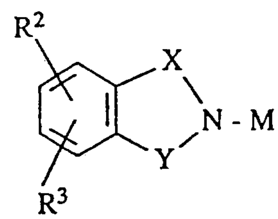

(VII)

in welcher

$R^2$, $R^3$, X und Y die obengenannte Bedeutung haben und

M für Wasserstoff oder ein Metallatom steht, in Gegenwart von inerten Lösungsmitteln bei Temperaturen zwischen 20 und 180°C umsetzt oder (d) Pyrimidinyl-piperazin-Derivate der allgemeinen Formel (VI-a)

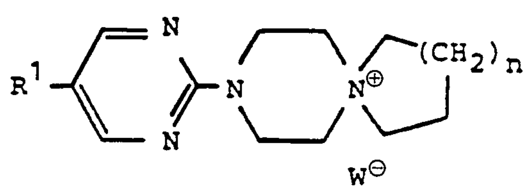

(VI-a),

in welcher

$R^1$ die oben angegebene Bedeutung hat, und

W für Chlor, Brom oder Jod steht und

n 1 oder 2 bedeutet,

mit Verbindungen der allgemeinen Formel (VII-a)

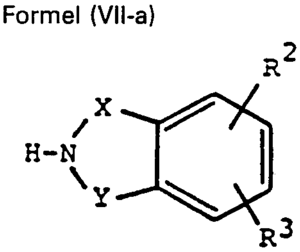

(VII-a),

in welcher

$R^1$, $R^2$ X und Y die oben angegebene Bedeutung haben, in Gegenwart von Basen in einem inerten Lösemittel bei Temperaturen zwischen 20 und 180°C umsetzt, und im Fall der Herstellung der Salze durch Zusatz von Säuren in das entsprechende Salz überführt.

7. Verfahren zur Herstellung von 2-Pyrimidinyl-1-piperazin-Derivaten der allgemeinen Formel

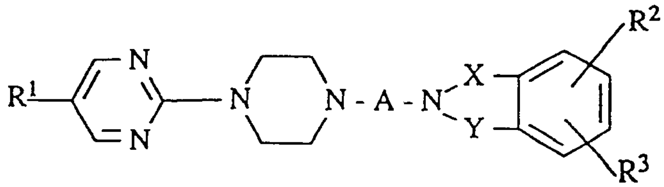

(I)

worin

A für eine Alkylengruppe mit 1 bis 6 Kohlenstoffatomen oder für 2-Hydroxy-n-propylen steht, wobei A nicht für die 2-Hydroxy-n-propylengruppe steht, wenn X und Y für Carbonyl und $R^1$, $R^2$ und $R^3$ für Wasserstoff stehen,

$R^1$ für Wasserstoff, Trifluormethyl oder für Alkoxy mit 1 bis 4 Kohlenstoffatomen, Trifluormethoxy oder für Hydroxy, Halogen, Cyano, Nitro, Amino, gegebenenfalls substituiert durch 1 oder 2 Methyl oder Ethyl, oder für Acetylamino oder Benzoylamino, oder für Indol-3-yl, oder für Phenyl steht, wobei der Phenylrest einfach oder mehrfach durch Methoxy, Ethoxy, Nitro, Halogen, Trifluormethyl, Amino, Cyano, Hydroxy, Methyl oder Ethyl, Acetylamino oder Benzoylamino substituiert sein kann,

$R^2$ und $R^3$ gleich oder verschieden sein können und für Wasserstoff oder für Alkoxy mit 1 bis 4 Kohlenstoffatomen, oder für Phenyl, oder für Hydroxy, Halogen, Cyano, Nitro, Amino, wobei das Stickstoffatom des Aminorestes durch 1 oder 2 Methyl oder Ethyl substituiert ist, oder für Methylsulfonamide, Benzol- und Toluolsulfonamido, Acetylamino, Ethoxycarbonylamino oder für Carboxyl, Methoxy- oder Ethoxycarbonyl, oder für Phenoxycarbonylamino, oder für Carbamoyl oder Sulfamoyl steht,

X Carbonyl oder Sulfonyl bedeuten, und

Y für den Rest

$$-\overset{\overset{\displaystyle O}{\|}}{C}-N(R^4)- \text{ steht}$$

wobei

$R^4$ Wasserstoff, Methyl oder Phenyl bedeutet, dadurch gekennzeichnet, daß man Pyrimidinylpiperazin-Derivate der allgemeinen Formel

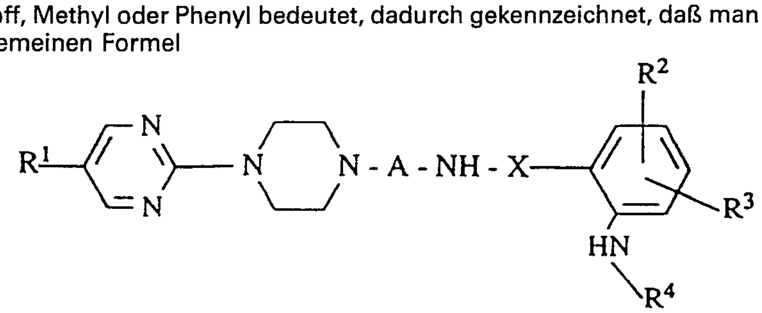

(VIII)

in der

$R^1$, $R^2$, $R^3$, $R^4$, X und A die obengenannte Bedeutung haben mit Carbonylverbindungen der allgemeinen Formel

$$R^5—CO—R^6 \qquad (IX)$$

worin

$R^5$ Halogen, Alkoxy, Amino oder Imidazolyl und

$R^6$ Halogen, Trihalogenalkyl, Alkoxy, Aryloxy, Alkoxycarbonyloxy, Amino oder Imidazolyl bedeuten, in Gegenwart von inerten Lösungsmitteln bei Temperaturen zwischen 20 und 180°C oder ohne Lösungsmittel bei Temperaturen zwischen 50 und 200°C umsetzt, und im Fall der Herstellung der Salze durch Zusatz von Säuren in das entsprechende Salz überführt.

8. Arzneimittel, enthaltend mindestens ein 2-Pyrimidinylpiperazin-Derivat nach Anspruch 1.

9. Verwendung von 2-Pyrimidinyl-piperazin-Derivaten nach Anspruch 1 zur Herstellung von Arzneimitteln.

10. Verwendung von 2-Pyrimidinyl-piperazin-Derivaten nach Anspruch 1 zur Herstellung von Arzneimitteln mit Wirkung auf das zentrale Nervensystem.

11. Verwendung von 2-Pyrimidinyl-piperazin-Derivaten nach Anspruch 1 zur Herstellung von Anxiolytika, Tranquilizern, Neuroleptika, Antidepressiva, Antiamnestika und Nootropika sowie lern-, leistungs- und gedächtnisverbessernden Mitteln.

12. Piperazin-Derivate der allgemeinen Formel

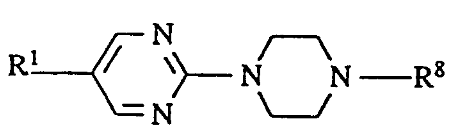

(XII)

in welcher

$R^1$ für Chlor, Fluor, Iod, Triofluormethyl, Phenyl, 3,4-Dimethoxyphenyl, 4-Methoxyphenyl, 4-Chlorphenyl, 3-Trifluormethylphenyl, 2-Chlor-6-fluor-phenyl, Indol-3-yl steht und

$R^8$ Methoxycarbonyl, Ethoxycarbonyl, Propoxycarbonyl, Isopropoxycarbonyl, Butoxycarbonyl oder Phenoxycarbonyl bedeutet, wobei $R^1$ nicht für Fluor steht, wenn $R^8$ für Ethoxycarbonyl steht.

13. ω-Aminoalkylpiperazine der allgemeinen Formel

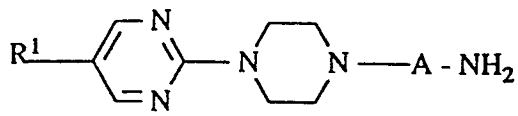

(IV)

in welcher

$R^1$ für Trifluormethyl, Chlor, Iod, Fluor, Phenyl, 3,4-Dimethoxyphenyl 4-Methoxyphenyl, 4-Chlorphenyl, 3-(Trifluormethyl)phenyl, 2-Chlor-6-fluorphenyl, Indol-3-yl und

A für n-Propylen oder n-Butylen steht, wobei $R^1$ nicht für Fluor steht, wenn A für n-Butylen steht.

14. Verbindungen der allgemeinen Formel

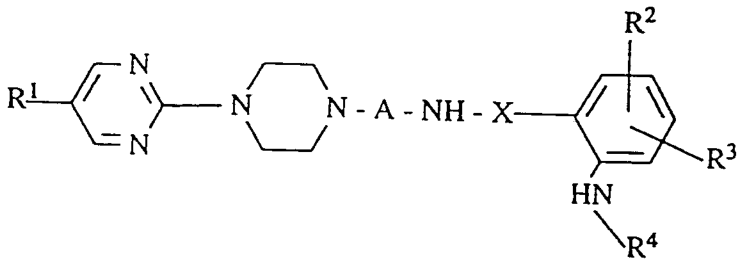

(VIII)

worin

A für eine Alkylengruppe mit 1 bis 6 Kohlenstoffatomen oder für 2-Hydroxy-n-propylen steht, wobei A nicht für die 2-Hydroxy-n-propylengruppe steht, wenn X und Y für Carbonyl und $R^1$, $R^2$ und $R^3$ für Wasserstoff stehen,

$R^1$ für Wasserstoff, Trifluormethyl oder für Alkoxy mit 1 bis 4 Kohlenstoffatomen, Trifluormethoxy oder für Hydroxy, Halogen, Cyano, Nitro, Amino, gegebenenfalls substituiert durch 1 oder 2 Methyl oder Ethyl, oder für Acetylamino oder Benzoylamino, oder für Indol-3-yl, oder für Phenyl steht, wobei der Phenylrest einfach oder mehrfach durch Methoxy, Ethoxy, Nitro, Halogen, Trifluormethyl; Amino, Cyano, Hydroxy, Methyl oder Ethyl, Acetylamino oder Benzoylamino substituiert sein kann,

$R^2$ und $R^3$ gleich oder verschieden sein können und für Wasserstoff oder Alkoxy mit 1 bis 4 Kohlenstoffatomen, oder für Phenyl, oder für Hydroxy, Halogen, Cyano, Nitro, Amino, wobei das Stickstoffatom des Aminorestes durch 1 oder 2 Methyl oder Ethyl substituiert ist, oder für Methylsulfonamide, Benzol- und Toluolsulfonamido, Acetylamino, Ethoxycarbonylamino, oder für Carboxyl, Methoxy- oder Ethoxycarbonyl, oder für Phenoxycarbonylamino, oder für Carbamoyl oder Sulfamoyl steht,

X für Carbonyl oder Sulfonyl steht, und

$R^4$ für Wasserstoff oder Phenyl steht.

15. Verfahren zur Herstellung von Piperazin-Derivaten der Formel

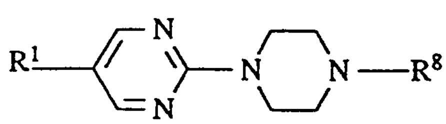

(XII)

in welcher

$R^1$ für Chlor, Fluor, Iod, Triofluormethyl, Phenyl, 3,4-Dimethoxyphenyl, 4-Methoxyphenyl, 4-Chlorphenyl, 3-Trifluormethylphenyl, 2-Chlor-6-fluor-phenyl, Indol-3-yl steht und

$R^8$ Methoxycarbonyl, Ethoxycarbonyl, Propoxycarbonyl, Isopropoxycarbonyl, Butoxycarbonyl oder Phenoxycarbonyl bedeutet, wobei $R^1$ nicht für Fluor steht, wenn $R^8$ für Ethoxycarbonyl steht, dadurch gekennzeichnet, daß man

a) Pyrimidin-Derivate der allgemeinen Formel

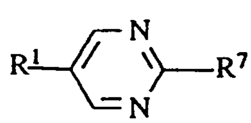

(X)

in welcher

$R^1$ die oben angegebene Bedeutung hat und

$R^7$ für Chlor oder Fluor steht, mit Piperazin-Derivaten der Formel

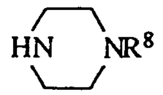

(XI)

in welcher

$R^8$ für Methoxycarbonyl oder Ethoxycarbonyl steht, in einem geeigneten Lösemittel bei Temperaturen von 50 bis 150°C, vorteilhaft in Gegenwart eines säurebindenden Mittels umsetzt oder indem man

b) Verbindungen der Formel

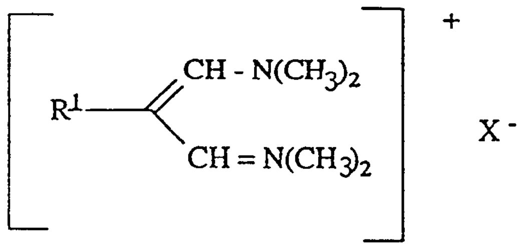

(XIII)

in welcher

$R^1$ die oben angegebene Bedeutung hat und

$X^-$ für Perchlorat steht mit Piperazinderivten der allgemeinen Formel

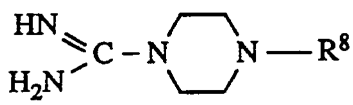

(XIV)

in welcher

$R^8$ die obenangegebene Bedeutung hat, in geeigneten Lösungsmitteln, bei deren Siedetemperatur umsetzt und in bekannter Weise aufarbeitet.

16. Verfahren zur Herstellung von ω-Aminoalkylpiperazinen der allgemeinen Formel

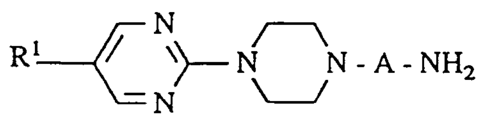

(IV)

in welcher

$R^1$ für Trifluormethyl, Chlor, Iod, Fluor, Phenyl, 3,4-Dimethoxyphenyl 4-Methoxyphenyl, 4-Chlorphenyl, 3-(Trifluormethyl)phenyl, 2-Chlor-6-fluorphenyl, Indol-3-yl und

A für n-Propylen oder n-Butylen steht, wobei $R^1$ nicht für Fluor steht, wenn A für n-Butylen steht, dadurch gekennzeichnet, daß man Pyrimidinyl-1-piperazine der allgemeinen Formel

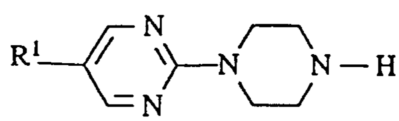

(II)

in welcher

$R^1$ die oben angegebene Bedeutung hat, mit Halogenalkylnitrilen der allgemeinen Formel

$$Cl—(A)_{n-1}—CN \quad (XVI)$$

wobei

n die Anzahl der Methylengruppen der Kette A bedeutet und

A die oben angegebene Bedeutung hat, alkyliert und die erhaltenen Cyanoverbindungen reduziert.

17. Verfahren zur Herstellung der Verbindung der allgemeinen Formel

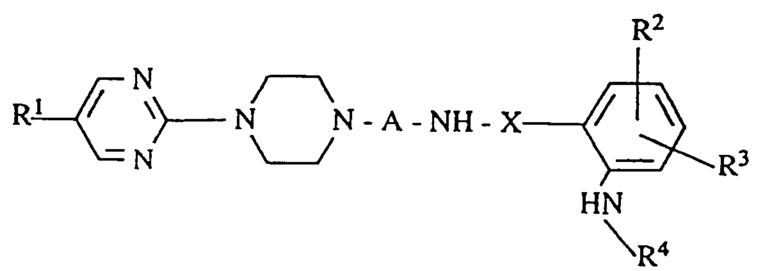

(VIII)

worin

A für eine Alkylengruppe mit 1 bis 6 Kohlenstoffatomen oder für 2-Hydroxy-n-propylen steht, wobei A

nicht für die 2-Hydroxy-n-propylengruppe steht, wenn X und Y für Carbonyl und $R^1$, $R^2$ und $R^3$ für Wasserstoff stehen,

$R^1$ für Wasserstoff, Trifluormethyl oder für Alkoxy mit 1 bis 4 Kohlenstoffatomen, Trifluormethoxy oder für Hydroxy, Halogen, Cyano, Nitro, Amino, gegebenenfalls substituiert durch 1 oder 2 Methyl oder Ethyl, oder für Acetylamino oder Benzoylamino, oder für Indol-3-yl, oder für Phenyl steht, wobei der Phenylrest einfach oder mehrfach durch Methoxy, Ethoxy, Nitro, Halogen, Trifluormethyl; Amino, Cyano, Hydroxy, Methyl oder Ethyl, Acetylamino oder Benzoylamino substituiert sein kann,

$R^2$ und $R^3$ gleich oder verschieden sein können und für Wasserstoff oder Alkoxy mit 1 bis 4 Kohlenstoffatomen, oder für Phenyl, oder für Hydroxy, Halogen, Cyano, Nitro, Amino, wobei das Stickstoffatom des Aminorestes durch 1 oder 2 Methyl oder Ethyl substituiert ist, oder für Methylsulfonamide, Benzol- und Toluolsulfonamido, Acetylamino, Ethoxycarbonylamino oder für Carboxyl, Methoxy- oder Ethoxycarbonyl, oder für Phenoxycarbonylamino, oder für Carbamoyl oder Sulfamoyl steht,

X für Carbonyl oder Sulfonyl steht, und

$R^4$ für Wasserstoff oder Phenyl steht, dadurch gekennzeichnet, daß man Nitroverbindungen der allgemeinen Formel

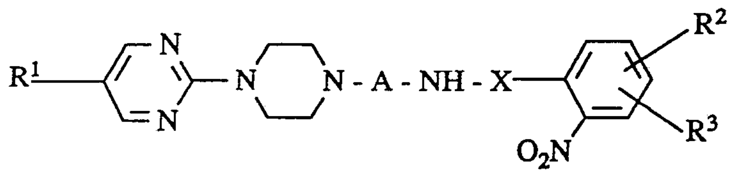

(XXII)

in welcher

$R^1$, $R^2$, $R^3$, X und A die oben angegebene Bedeutung haben, mit geeigneten Reduktionsmitteln reduziert.

18. 2-Pyrimidinyl-1-piperazin-Derivate nach Anspruch 1 zur Behandlung von Krankheiten.

**Revendications**

1. Dérivés de 2-pyrimidinyl-1-pipérazine de formule générale

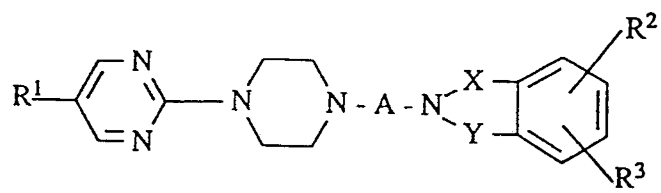

(I)

dans laquelle

A représente un groupe alkylène ayant 1 à 6 atomes de carbone ou un groupe 2-hydroxy-n-propylène, A n'étant pas le groupe 2-hydroxy-n-propylène lorsque X et Y sont des groupes carbonyle et $R^1$, $R^2$ et $R^3$ sont de l'hydrogène,

$R^1$ représente l'hydrogène, un groupe trifluorométhyle ou un groupe alkoxy ayant 1 à 4 atomes de carbone, trifluorométhoxy, ou un groupe hydroxy, un halogène, un groupe cyano, nitro, amino, éventuellement substitué par 1 ou 2 radicaux méthyle ou éthyle, ou un groupe acétylamino ou benzoylamino, ou un groupe indole-3-yle, ou un reste phényle, le reste phényle pouvant être substitué une ou plusieurs fois par un radical méthoxy, éthoxy, nitro, halogéno, trifluorométhyle, amino, cyano, hydroxy, méthyle ou éthyle, acétylamino ou benzoylamino,

$R^2$ et $R^3$ peuvent être identiques ou différents et représentent l'hydrogène ou un groupe alkoxy ayant 1 à 4 atomes de carbone, ou un groupe phényle, ou un groupe hydroxy, un halogène, un groupe cyano, nitro, amino, l'atome d'azote du reste amino étant substitué par 1 ou 2 radicaux méthyle ou éthyle, ou un groupe méthylsulfonamido, benzène- et toluènesulfonamido, acétylamino, éthoxycarbonylamino ou un groupe carboxyle, méthoxycarbonyle ou éthoxycarbonyle, ou un groupe phénoxycarbonylamino, ou un groupe carbamoyle ou sulfamoyle,

X et Y peuvent être identiques ou différents et représentent des groupes carbonyle ou sulfonyle, et

X seul représente un groupe carbonyle ou sulfonyle, lorsque Y représente en même temps un groupe $—CO—CH_2$ ou $—CO—N(R^4)—$, $R^4$ représentant l'hydrogène ou un groupe méthyle ou phényle, ainsi que leurs sels d'addition d'acides.

2. Dérivés de 2-pyrimidinyl-1-pipérazine de formule générale suivant la revendication 1, dans laquelle

A est un groupe éthylène, n-propylène, n-butylène, 2-méthyl-n-propylène ou 2-hydroxy-n-propylène, A ne représentant pas le groupe, 2-hydroxy-n-propylène lorsque X et Y sont des groupes carbonyle et $R^1$, $R^2$ et $R^3$ sont de l'hydrogène,

$R^1$ représente l'hydrogène ou un groupe méthoxy, éthoxy ou le fluor, l'iode, un groupe cyano ou un groupe indole-3-yle, ou un reste phényle, le reste phényle pouvant porter un ou plusieurs substituants méthoxy ou éthoxy, chloro, fluoro, trifluorométhyle, cyano,

$R^2$ et $R^3$ peuvent être identiques ou différents et représentent l'hydrogène ou un groupe méthoxy, trifluorométhoxy ou le chlore, le fluor, un groupe cyano, nitro, amino, l'atome d'azote du reste amino étant substitué par 1 ou 2 radicaux méthyle ou éthyle, ou un groupe méthylsulfonamido, acétylamino, éthoxycarbonylamino,

X et Y peuvent être identiques ou différents et représentent des groupes carbonyle ou sulfonyle et

X représente seul un groupe carbonyle ou sulfonyle, lorsque Y représente en même temps un groupe $—CO—CH_2$ ou $—CO—N(R^4)—$, où

$R^4$ est l'hydrogène ou un groupe phényle ainsi que leurs sels d'addition d'acides.

3. Dérivés de 2-pyrimidinyl-1-pipérazine de formule générale suivant la revendication 1, dans laquelle

A est un groupe éthylène, n-propylène, n-butylène ou 2-hydroxy-n-propylène, A ne représentant pas le groupe 2-hydroxy-n-propylène lorsque X et Y sont des groupes carbonyle et $R^1$, $R^2$ et $R^3$ sont de l'hydrogène,

$R^1$ représente l'hydrogène, le chlore, le fluor, l'iode, un groupe 4-méthoxyphényle, 3,4-diméthoxyphényle, phényle, 4-chlorophényle, 3-(trifluorométhyl)phényle, 2-chloro-6-fluorophényle ou indole-3-yle,

$R^2$ et $R^3$ peuvent être identiques ou différents et représentent l'hydrogène, le fluor, le chlore ou un groupe nitro,

X représente les groupes carbonyle et sulfonyle et

Y est un groupe carbonyle, sulfonyle, $—CO—CH_2—$ ou $—CO—N(R^4)—$,

$R^4$ représente l'hydrogène ou un radical phényle ou méthyle, ainsi que leurs sels d'addition d'acides.

4. Le 1,1-dioxyde de 2-(4-(4-(2-pyrimidinyl)-1-pipérazinyl)butyl)-1,2-benzoisothiazole-(2H)-one

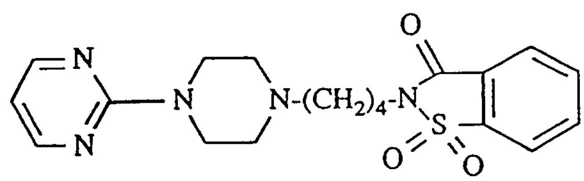

ainsi que le chlorhydrate correspondant.

5. Le 1,1-dioxyde de 2-(3-(2-pyrimidinyl)-1-pipérazinyl)propyl)-1,1-benzoisothiazole-3(2H)one

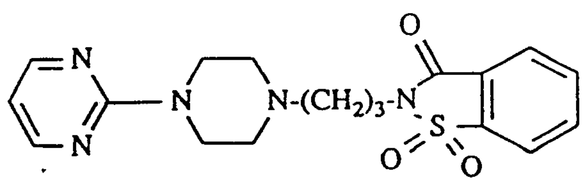

ainsi que le chlorhydrate correspondant.

6. Procédé de production de dérivés de 2-pyrimidinyl-1-pipérazine de formule générale

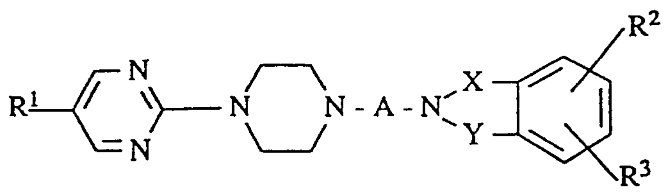

(I)

dans laquelle

A représente un groupe alkylène ayant 1 à 6 atomes de carbone ou un groupe 2-hydroxy-n-propylène, A n'étant pas le groupe 2-hydroxy-n-propylène lorsque X et Y sont des groupes carbonyle et $R^1$, $R^2$ et $R^3$ sont de l'hydrogène,

$R^1$ représente l'hydrogène, un groupe trifluorométhyle ou un groupe alkoxy ayant 1 à 4 atomes de carbone, trifluorométhoxy, ou un groupe hydroxy, un halogène, un groupe cyano, nitro, amino, éventuellement substitué par 1 ou 2 radicaux méthyle ou éthyle, ou un groupe acétylamino ou benzoylamino, ou un groupe indole-3-yle, ou un reste phényle, le reste phényle pouvant être substitué une ou plusieurs fois par un radical méthoxy, éthoxy, nitro, halogéno, trifluorométhyle, amino, cyano, hydroxy, méthyle ou éthyle, acétylamino ou benzoylamino,

$R^2$ et $R^3$ peuvent être identiques ou différents et représentent l'hydrogène ou un groupe alkoxy ayant 1 à 4 atomes de carbone, ou un groupe phényle, ou un groupe hydroxy, un halogène, un groupe cyano, nitro, amino, l'atome d'azote du reste amino étant substitué par 1 ou 2 radicaux méthyle ou éthyle, ou un groupe méthylsulfonamido, benzène- et toluènesulfonamido, acétylamino, éthoxycarbonylamino ou un groupe carboxyle, méthoxycarbonyle ou éthoxycarbonyle, ou un groupe phénoxycarbonylamino, ou un groupe carbamoyle ou sulfamoyle,

X et Y peuvent être identiques ou différents et représentent des groupes carbonyle ou sulfonyle, et
X seul représente un groupe carbonyle ou sulfonyle, lorsque
Y représente en même temps un groupe —CO—$CH_2$ ou —CO—N($R^4$)—, $R^4$ représentant l'hydrogène ou un groupe méthyle ou phényle, ainsi que leurs sels d'addition d'acides, caractérisé en ce que
a) On alkyle des dérivés de pyrimidinylpipérazine de formule générale

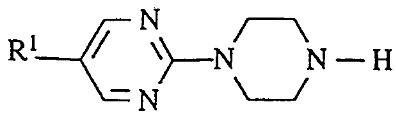
(II)

dans laquelle
$R^1$ a la définition donnée ci-dessus avec des composés de formule générale

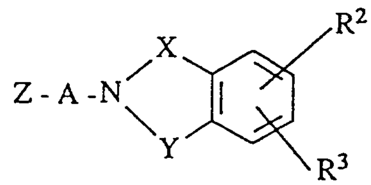
(III)

dans laquelle
$R^2$, $R^3$, X et Y ont la définition donnée ci-dessus et
Z est un groupe hydroxy, du chlore, du brome, de l'iode, un groupe méthylsulfonyloxy ou 4-tolylsulfonyloxy, ou bien le groupe
A—Z représente un groupe

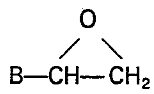

B représentant un pont A raccourci de deux atomes terminaux de carbone, en présence de solvants inertes à des températures comprises entre 20 et 200°C, ou
b) On fait réagir des dérivés d'aminoalkylpyrimidinylpipérazine de formule générale

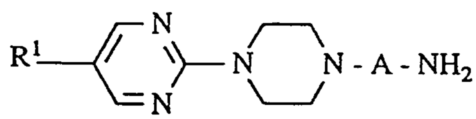
(IV)

dans laquelle
$R^1$ et A ont la définition indiquée ci-dessus, avec des anhydrides de formule générale

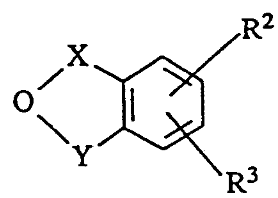
(V)

dans laquelle
$R^2$ et $R^3$ ont la définition indiquée ci-dessus,
X et Y peuvent être identiques ou différents et désignent des groupes carbonyle ou sulfonyle, ou bien
X est un groupe carbonyle lorsque Y est en même temps un groupe —CO—$CH_2$—, à une température de 100 à 250°C dans un solvant inerte ou à l'état fondu, en l'absence de solvant, ou bien
c) On fait réagir des dérivés de pyrimidinylpipérazine de formule générale

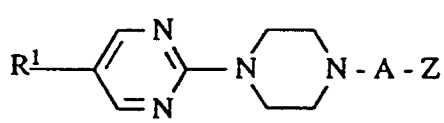
(VI)

dans laquelle
$R^1$, A et Z ont la définition indiquée ci-dessus, avec des composés de formule générale

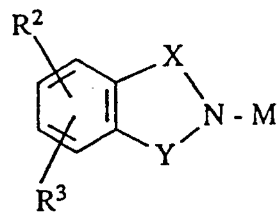

(VII)

dans laquelle

$R^2$, $R^3$, X et Y ont la définition indiquée ci-dessus et

M représente l'hydrogène ou un atome de métal, en présence de solvants inertes, à des températures comprises entre 20 et 180°C, ou bien (*d*) on fait réagir des dérivés de pyrimidinylpipérazine de formule générale (VI-a)

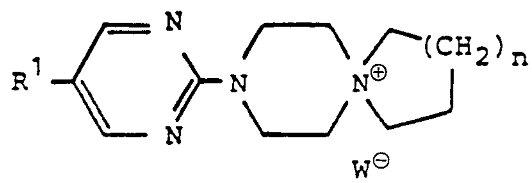

(VI-a),

dans laquelle

$R^1$ a la définition indiquée ci-dessus,

W représente le chlore, le brome ou l'iode et

n a la valeur 1 ou 2, avec des composés de formule générale (VII-a)

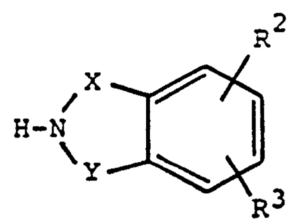

(VII-a),

dans laquelle

$R^1$, $R^2$, X et Y ont la définition indiquée ci-dessus, en présence de bases dans un solvant inerte, à des températures comprises entre 20 et 180°C, et dans le cas de la préparation des sels, on transforme le produit par addition d'acides en le sel correspondant.

7. Procédé de préparation de dérivés de 2-pyrimidinyl-1-pipérazine de formule générale

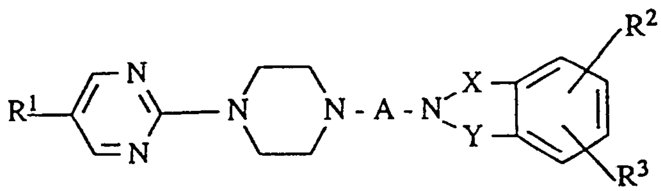

(I)

dans laquelle

A est un groupe alkylène ayant 1 à 6 atomes de carbone ou un groupe 2-hydroxy-n-propylène, A n'étant pas le groupe 2-hydroxy-n-propylène lorsque X et Y sont des groupes carbonyle et $R^1$, $R^2$ et $R^3$ sont de l'hydrogène,

$R^1$ représente l'hydrogène, un groupe trifluorométhyle ou un groupe alkoxy ayant 1 à 4 atomes de carbone, trifluorométhoxy ou un groupe hydroxy, un halogène, un groupe cyano, nitro, amino, éventuellement substitué par 1 ou 2 radicaux méthyle ou éthyle, ou un groupe acétylamino ou benzoylamino ou un groupe indole-3-yle ou un reste phényle, qui peut être substitué une ou plusieurs fois par un radical méthoxy, éthoxy, nitro, halogéno, trifluorométhyle, amino, cyano, hydroxy, méthyle ou éthyle, acétylamino ou benzoylamino,

$R^2$ et $R^3$ peuvent être identiques ou différents et représentent l'hydrogène ou un groupe alkoxy ayant 1 à 4 atomes de carbone, ou

un groupe phényle ou un groupe hydroxy, un halogène, un groupe cyano, nitro, amino, l'atome d'azote du reste amino étant substitué par 1 ou 2 radicaux méthyle ou éthyle, ou un groupe méthylsulfonamido, benzène- et toluènesulfonamido, acétylamino, éthoxycarbonylamino, ou un groupe carboxyle, méthoxy- ou éthoxycarbonyle ou un groupe phénoxycarbonylamino ou un groupe carbamoyle ou sulfamoyle,

X est un groupe carbonyle ou sulfonyle et

Y est le reste de formule

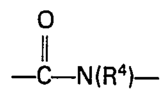

dans laquelle

$R^4$ est l'hydrogène ou un groupe méthyle ou phényle, caractérisé en ce qu'on fait réagir des dérivés de pyrimidinylpipérazine de formule générale

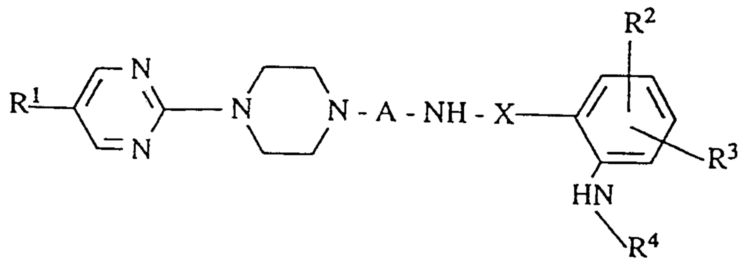

(VIII)

dans laquelle

$R^1$, $R^2$, $R^3$, $R^4$, X et A ont la définition indiquée ci-dessus, avec des composés carbonyliques de formule générale

$$R^5—CO—R^6 \qquad (IX)$$

dans laquelle

$R^5$ est un halogène, un groupe alkoxy, amino ou imidazolyle et

$R^6$ est un halogène, un groupe trihalogénalkyle, alkoxy, aryloxy, alkoxycarbonyloxy, amino ou imidazolyle, en présence de solvants inertes, à des températures comprises entre 20 et 180°C ou en l'absence de solvants à des températures comprises entre 50 et 200°C, et dans le cas de la préparation des sels, on transforme le produit en le sel correspondant par addition d'acides.

8. Médicament contenant au moins un dérivé de 2-pyrimidinylpipérazine suivant la revendication 1.

9. Utilisation de dérivés de 2-pyrimidinylpipérazine suivant la revendication 1 pour la préparation de médicaments.

10. Utilisation de dérivés de 2-pyrimidinylpipérazine suivant la revendication 1 pour la préparation de médicaments agissant sur le système nerveux central.

11. Utilisation de dérivés de 2-pyrimidinylpipérazine suivant la revendication 1 pour la préparation d'anxiolytiques, de tranquillisants, de neuroleptiques, d'anti-dépresseurs, d'anti-amnésiques et de nootropes ainsi que de compositions améliorant la capacité et la performance intellectuelle et la mémoire.

12. Dérivés de pipérazine de formule générale

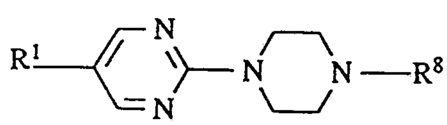

(XII)

dans laquelle

$R^1$ représente le chlore, le fluor, l'iode, un groupe trifluorométhyle, phényle, 3,4-diméthoxyphényle, 4-méthoxyphényle, 4-chlorophényle, 3-trifluorométhylphényle, 2-chloro-6-fluorophényle, indole-3-yle et

$R^8$ est un groupe méthoxycarbonyle, éthoxycarbonyle, propoxycarbonyle, isopropoxycarbonyle, butoxycarbonyle ou phénoxycarbonyle,

$R^1$ ne représentant pas le fluor lorsque $R^8$ est un groupe éthoxycarbonyle.

13. ω-aminoalkylpipérazines de formule générale

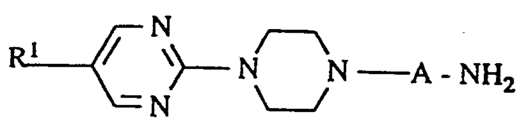

(IV)

dans laquelle

$R^1$ est un groupe trifluorométhyle, le chlore, l'iode, le fluor, un groupe phényle, 3,4-diméthoxyphényle, 4-méthoxyphényle, 4-chlorophényle, 3-(trifluorométhyl)phényle, 2-chloro-6-fluorophényle, indole-3-yle et

A est un groupe n-propylène ou n-butylène,

$R^1$ ne représentant pas le fluor lorsque A est un groupe n-butylène.

14. Composés de formule générale

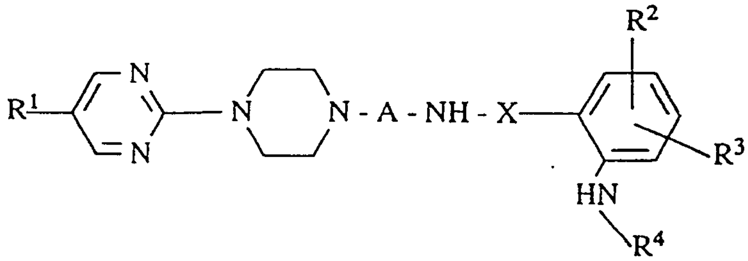

(VIII)

dans laquelle

A est un groupe alkylène ayant 1 à 6 atomes de carbone ou un groupe 2-hydroxy-n-propylène, A n'étant pas le groupe 2-hydroxy-n-propylène lorsque X et Y sont des groupes carbonyle et $R^1$, $R^2$ et $R^3$ sont de l'hydrogène,

$R^1$ représente l'hydrogène, un groupe trifluorométhyle ou un groupe alkoxy ayant 1 à 4 atomes de carbone, trifluorométhoxy ou un groupe hydroxy, un halogène, un groupe cyano, nitro, amino, éventuellement substitué par 1 ou 2 radicaux méthyle ou éthyle, ou un groupe acétylamino ou benzoylamino, ou un groupe indole-3-yle ou un reste phényle, qui peut être substitué une ou plusieurs fois par un radical méthoxy, éthoxy, nitro, halogéno, trifluorométhyle; amino, cyano, hydroxy, méthyle ou éthyle, acétylamino ou benzoylamino,

$R^2$ et $R^3$ peuvent être identiques ou différents et représentent l'hydrogène ou un groupe alkoxy ayant 1 à 4 atomes de carbone, ou un groupe phényle ou un groupe hydroxy, un halogène, un groupe cyano, nitro, amino, l'atome d'azote du reste amino étant substitué par 1 ou 2 radicaux méthyle ou éthyle, ou un groupe méthylsulfonamido, benzène- et toluènesulfonamido, acétylamino, éthylènecarbonylamino ou un groupe carboxyle, méthoxy- ou éthoxycarbonyle ou un groupe phénoxycarbonylamino ou un groupe carbamoyle ou sulfamoyle,

X est un groupe carbonyle ou sulfonyle, et

$R^4$ est l'hydrogène ou un groupe phényle.

15. Procédé de préparation de dérivés de pipérazine de formule

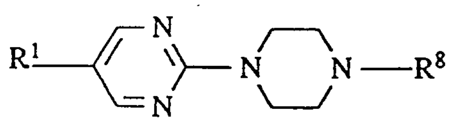

(XII)

dans laquelle

$R^1$ représente le chlore, le fluor, l'iode, un groupe trifluorométhyle, phényle, 3,4-diméthoxyphényle, 4-méthoxyphényle, 4-chlorophényle, 3-trifluorométhylphényle, 2-chloro-6-fluorophényle, indole-3-yle et

$R^8$ est un groupe méthoxycarbonyle, éthoxycarbonyle, propoxycarbonyle, isopropoxycarbonyle, butoxycarbonyle ou phénoxycarbonyle,

$R^1$ ne représentant pas le fluor lorsque $R^8$ est un groupe éthoxycarbonyle, caractérisé en ce que

a) On fait réagir des dérivés de pyrimidine de formule générale

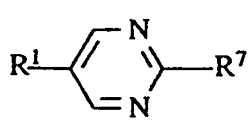

(X)

dans laquelle

$R^1$ a la définition indiquée ci-dessus, et

$R^7$ est le chlore ou le fluor, avec des dérivés de pipérazine de formule

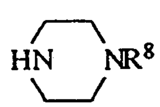

(XI)

dans laquelle

$R^8$ est un groupe méthoxycarbonyle ou éthoxycarbonyle, dans un solvant approprié à des températures de 50 à 150°C, de préférence en présence d'un accepteur d'acide, ou bien

b) On fait réagir des composés de formule

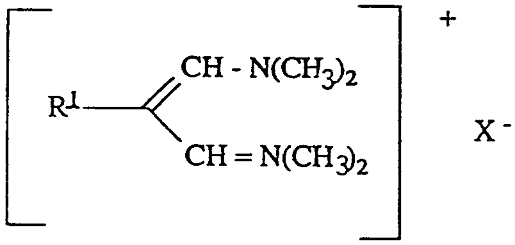

(XIII)

dans laquelle

$R^1$ a la définition indiquée ci-dessus et

$X^-$ est l'ion perchlorate avec des dérivés de pipérazine de formule générale

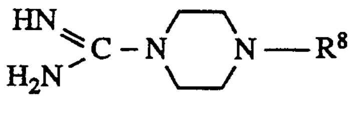

(XIV)

dans laquelle

$R^8$ a la définition indiquée ci-dessus, dans des solvants appropriés, à leurs températures d'ébullition et on traite le produit d'une manière connue

16. Procédé de préparation d' ω-aminoalkylpipérazines de formule générale

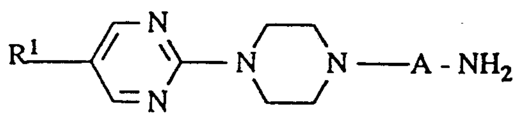

(IV)

dans laquelle

$R^1$ est un groupe trifluorométhyle, le chlore, l'iode, le fluor, un groupe phényle, 3,4-diméthoxyphényle, 4-méthoxyphényle, 4-chlorophényle, 3-(trifluorométhyl)phényle, 2-chloro-6-fluorophényle, indole-3-yle et

A est un groupe n-propylène ou n-butylène,

$R^1$ ne représentant pas le fluor lorsque A est un groupe n-butylène, caractérisé en ce qu'on alkyle des pyrimidinyl-1-pipérazines de formule générale

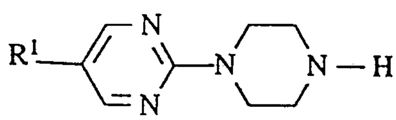

(II)

dans laquelle $R^1$ a la définition indiquée ci-dessus

avec des halogénalkylnitriles de formule générale

$Cl\text{-}(A)_{n-1}\text{—}CN$ (XVI)

dans laquelle

n est le nombre de groupes méthylène de la chaîne A et

A a la définition indiquée ci-dessus et on réduit les composés cyano obtenus.

17. Procédé de préparation des composés de formule générale

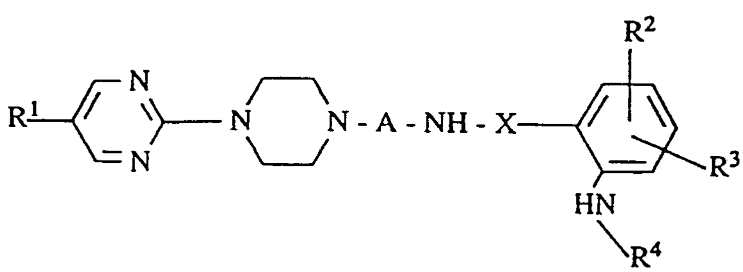

(VIII)

dans laquelle

A est un groupe alkylène ayant 1 à 6 atomes de carbone ou un groupe 2-hydroxy-n-propylène, A n'étant pas le groupe 2-hydroxy-n-propylène lorsque X et Y sont des groupes carbonyle et $R^1$, $R^2$ et $R^3$ sont de l'hydrogène,

$R^1$ représente l'hydrogène, un groupe trifluorométhyle ou un groupe alkoxy ayant 1 à 4 atomes de carbone, trifluorométhoxy ou un groupe hydroxy, un halogène, un groupe cyano, nitro, amino, éventuellement substitué par 1 ou 2 radicaux méthyle ou éthyle, ou un groupe acétylamino ou benzoylamino, ou un groupe indole-3-yle, ou un reste phényle, qui peut être substitué une ou plusieurs fois

par un radical méthoxy, éthoxy, nitro, halogéno, trifluorométhyle; amino, cyano, hydroxy, méthyle ou éthyle, acétylamino ou benzoylamino,

$R^2$ et $R^3$ peuvent être identiques ou différents et représentent l'hydrogène ou un groupe alkoxy ayant 1 à 4 atomes de carbone, ou un groupe phényle ou un groupe hydroxy, un halogéne, un groupe cyano, nitro, amino, l'atome d'azote du reste amino étant substitué par 1 ou 2 radicaux méthyle ou éthyle, ou un groupe méthylsulfonamido, benzène- et toluènesulfonamido, acétylamino, éthylènecarbonylamino ou un groupe carboxyle, méthoxy- ou éthoxycarbonyle, ou un groupe phénoxycarbonylamino ou un groupe carbamoyle ou sulfamoyle,

X est un groupe carbonyle ou sulfonyle, et

$R^4$ est l'hydrogène ou un groupe phényle, caractérisé en ce qu'on réduit des composés nitrés de formule générale

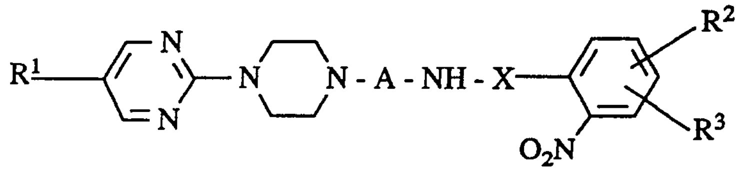

(XXII)

dans laquelle

$R^1$, $R^2$, $R^3$, X et A ont la définition indiquée ci-dessus, avec des agents réducteurs appropriés.

18. Dérivés de 2-pyrimidinyl-1-pipérazine suivant la revendication 1, destinés au traitement de maladies.

**Claims**

1. 2-Pyrimidinyl-1-piperazine derivatives of the general formula

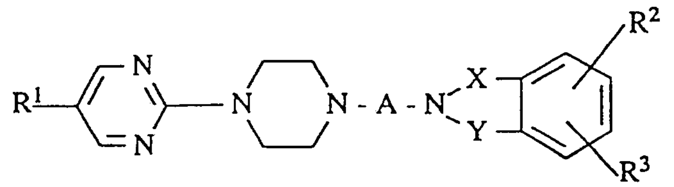

(I)

in which

A represents an alkylene group having 1 to 6 carbon atoms or represents 2-hydroxy-n-propylene, but A does not represent the 2-hydroxy-n-propylene group when X and Y represent carbonyl and $R^1$, $R^2$ and $R^3$ represent hydrogen,

$R^1$ represents hydrogen, trifluoromethyl or represents alkoxy having 1 to 4 carbon atoms, trifluoromethoxy or represents hydroxyl, halogen, cyano, nitro, amino, optionally substituted by 1 or 2 methyl or ethyl, or represents acetylamino or benzoylamino or represents indol-3-yl, or represents phenyl, it being possible for the phenyl radical to be monosubstituted or polysubstituted by methoxy, ethoxy, nitro, halogen, trifluoromethyl, amino, cyano, hydroxyl, methyl or ethyl, acetylamino or benzoylamino,

$R^2$ and $R^3$, which can be identical or different, represent hydrogen or alkoxy having 1 to 4 carbon atoms, or represents phenyl, or represent hydroxyl, halogen, cyano, nitro, amino, in which case the nitrogen atom of the amino radical is substituted of the amino radical is substituted by 1 or 2 methyl or ethyl, or represent methylsulphonamido, benzene- and toluenesulphonamido, acetylamino, ethoxycarbonylamino or represent carboxyl, methoxy- or ethoxycarbonyl, or represent phenoxycarbonylamino, or represent carbamoyl or sulphamoyl,

X and Y, which can be identical or different, denote carbonyl or sulphonyl, and

X alone represents carbonyl or sulphonyl when Y at the same time denotes —CO—$CH_2$ or —CO—N($R^4$)—, where $R^4$ represents hydrogen, methyl or phenyl and the acid addition salts thereof.

2. 2-Pyrimidinyl-1-piperazine derivatives of the general formula according to Claim 1, in which

A represents ethylene, n-propylene, n-butylene, 2-methyl-n-propylene or 2-hydroxy-n-propylene, but A does not represent the 2-hydroxy-n-propylene group when X and Y represent carbonyl and $R^1$, $R^2$ and $R^3$ represent hydrogen,

$R^1$ represents hydrogen or represents methoxy, ethoxy or represents fluorine, iodine, cyano or represents indol-3-yl, or represents phenyl, it being possible for the phenyl radical to be monosubstituted or polysubstituted by methoxy or ethoxy, chlorine, fluorine, trifluoromethyl, cyano,

$R^2$ and $R^3$, which can be identical or different, represent hydrogen, or represent methoxy, trifluoromethoxy or represent chlorine, fluorine, cyano, nitro, amino, in which case the nitrogen atom of the amino radical is substituted by 1 or 2 methyl or ethyl, or represent methylsulphonamido, acetylamino, ethoxycarbonylamino,

X and Y, which can be identical or different, denote carbonyl or sulphonyl, and

X alone represents carbonyl or sulphonyl when Y at the same time denotes —CO—$CH_2$— or —CO—N($R^4$)—,

$R^4$ represents hydrogen or phenyl and the acid additions salts thereof.

3. 2-Pyrimidinyl-1-piperazine derivatives of the general formula according to Claim 1, in which

A denotes ethylene, n-propylene, n-butylene or 2-hydroxy-n-propylene but A does not represent 2-hydroxy-n-propylene when X and Y represent carbonyl and $R^1$, $R^2$ and $R^3$ represent hydrogen,

$R^1$ denotes hydrogen, chlorine, fluorione, iodine, 4-methoxy-phenyl, 3,4-dimethoxyphenyl, phenyl, 4-chloro-phenyl, 3-(trifluoromethyl)-phenyl, 2-chloro-6-fluoro-phenyl or indol-3-yl,

$R^2$ and $R^3$ which can be identical or different, represent hydrogen, fluorine, chlorine or nitro,

X represents carbonyl and sulphonyl, and

Y represents carbonyl, sulphonyl, $—CO—CH_2—$ or $—CO—N(R^4)—$, where $R^4$ denotes hydrogen, phenyl or methyl, and the acid addition salts thereof.

4. 2-(4-(4-(2-Pyrimidinyl)-1-piperazinyl)butyl)-1,2-benzoisothiazol-3(2H)-one, 1,1-dioxide

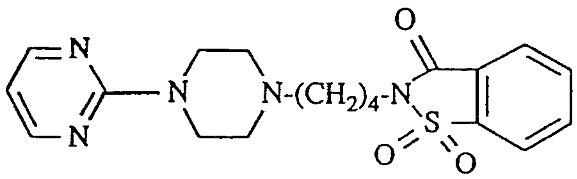

and the corresponding hydrochloride.

5. 2-(3-(4-(2-Pyrimidinyl)-1-piperazinyl)propyl)-1,2-benzoisothiazol-3(2H)one 1,1-dioxide

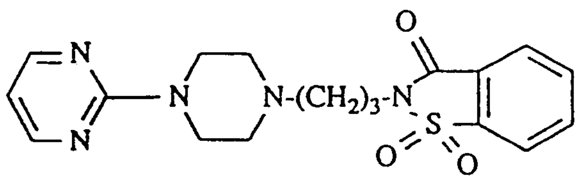

and the corresponding hydrochloride.

6. Process for the preparation of 2-pyrimidinyl-1-piperazine derivatives of the general formula

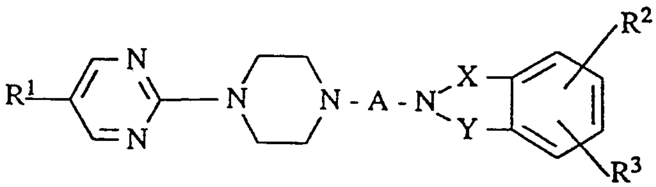

(I)

in which

A represents an alkylene group having 1 to 6 carbon atoms or represents 2-hydroxy-n-propylene, but A does not represent the 2-hydroxy-n-propylene group when X and Y represent carbonyl and $R^1$, $R^2$ and $R^3$ represent hydrogen,

$R^1$ represents hydrogen, trifluoromethyl or represents alkoxy having 1 to 4 carbon atoms, trifluoromethoxy or represents hydroxyl, halogen, cyano, nitro, amino, optionally substituted by 1 or 2 methyl or ethyl, or represents acetylamino or benzoylamino or represents indol-3-yl, or represents phenyl, it being possible for the phenyl radical to be monosubstituted or polysubstituted by methoxy, ethoxy, nitro, halogen, trifluoromethyl, amino, cyano, hydroxyl, methyl or ethyl, acetylamino or benzoylamino,

$R^2$ and $R^3$, which can be identical or different, represent hydrogen or alkoxy having 1 to 4 carbon atoms, or represent phenyl, or represent hydroxyl, halogen, cyano, nitro, amino, in which case the nitrogen atom of the amino radical is substituted by 1 or 2 methyl or ethyl, or represent methylsulphonamido, benzene- and toluenesulphonamido, acetylamino, ethoxycarbonylamino or represent carboxyl, methoxy- or ethoxycarbonyl, or represent phenoxycarbonylamino, or represent carbamoyl or sulphamoyl,

X and Y, which can be identical or different, denote carbonyl or sulphonyl, and

X alone represents carbonyl or sulphonyl when Y at the same time denotes $—CO—CH_2$ or $—CO—N(R^4)—$, where $R^4$ represents hydrogen, methyl or phenyl and the acid addition salts thereof, characterized in that

a) pyrimidinylpiperazine derivatives of the general formula

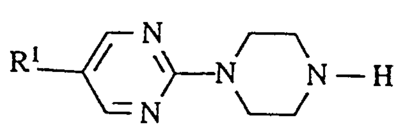

(II)

in which

$R^1$ has the abovementioned meaning, are alkylated with compounds of the general formula

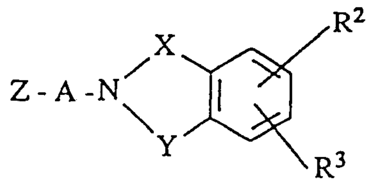

(III)

in which

$R^2$, $R^3$, X and Y have the abovementioned meaning, and

Z represents hydroxyl, chlorine, bromine, iodine, methylsulphonyloxy or 4-tolylsulphonyloxy, or the group

A—Z represents

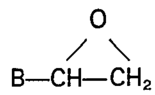

where

B represents a bridging member A shortened by two terminal C atoms, in the presence of inert solvents at temperatures between 20 and 200°C or

b) aminoalkylpyrimidinylpiperazine derivatives of the general formula

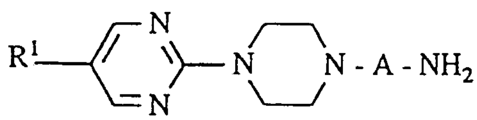

(IV)

in which

$R^1$ and A have the abovementioned meaning, are reacted with anhydrides of the general formula

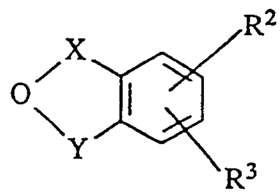

(V)

in which

$R^2$, $R^3$, X and Y have the abovementioned meaning, can be identical or different, and denote carbonyl or sulphonyl, or

X denotes carbonyl when Y at the same time denotes $-CO-CH_2-$, at a temperature between 100 and 250°C in an inert solvent or without solvent in the melt, or

c) pyrimidinylpiperazine derivatives of the general formula

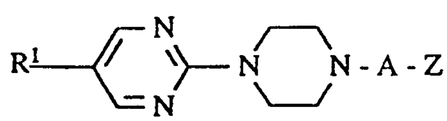

(VI)

in which

$R^1$, A and Z have the abovementioned meaning, are reacted with compounds of the general formula

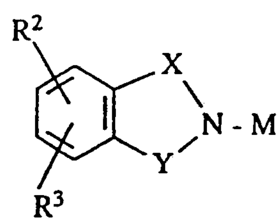

(VII)

in which

$R^2$, $R^3$, X and Y have the abovementioned meaning, and

M represents hydrogen or a metal atom, in the presence of inert solvents at temperatures between 20 and 180°C, or

d) pyrimidinyl-piperazine derivatives of the general formula (VI-a)

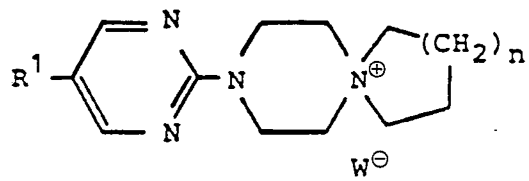

(VI-a),

in which

$R^1$ has the abovementioned meaning, and

W represents chlorine, bromine or iodine, and

n denotes 1 or 2, are reacted with compounds of the general formula (VII-a)

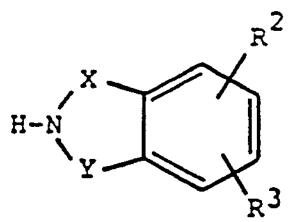

(VII-a),

in which

$R^1$, $R^2$, X and Y have the abovementioned meaning, in the presence of bases in an inert solvent at temperatures between 20 and 180°C, and, in the case of the preparation of the salts, acids are added to convert into the corresponding salt.

7. Process for the preparation of 2-pyrimidinyl-1-piperazine derivatives of the general formula

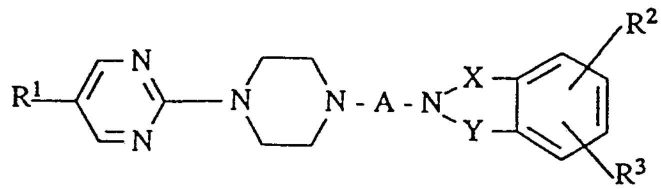

(I)

in which

A represents an alkylene group having 1 to 6 carbon atoms or represents 2-hydroxy-n-propylene, but A does not represent the 2-hydroxy-n-propylene group when X and Y represent carbonyl and $R^1$, $R^2$ and $R^3$ represent hydrogen,

$R^1$ represents hydrogen, trifluoromethyl or represents alkoxy having 1 to 4 carbon atoms, trifluoromethoxy or represents hydroxyl, halogen, cyano, nitro, amino, optionally substituted by 1 or 2 methyl or ethyl, or represents acetylamino or benzoylamino or represents indol-3-yl, or represents phenyl, it being possible for the phenyl radical to be monosubstituted or polysubstituted by methoxy, ethoxy, nitro, halogen, trifluoromethyl, amino, cyano, hydroxyl, methyl or ethyl, acetylamino or benzoylamino,

$R^2$ and $R^3$, which can be identical or different, represent hydrogen or alkoxy having 1 to 4 carbon atoms, or represent phenyl, or represent hydroxyl, halogen, cyano, nitro, amino, in which case the nitrogen atom of the amino radical is substituted by 1 or 2 methyl or ethyl, or represent methylsulphonamido, benzene- and toluenesulphonamido, acetylamino, ethoxycarbonylamino or represent carboxyl, methoxy- or ethoxycarbonyl, or represent phenoxycarbonylamino, or represent carbamoyl or sulphamoyl,

X denotes carbonyl or sulphonyl, and

Y represents the radical

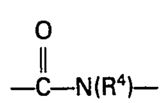

where

$R^4$ denotes hydrogen, methyl or phenyl, characterized in that pyrimidinylpiperazine derivatives of the general formula

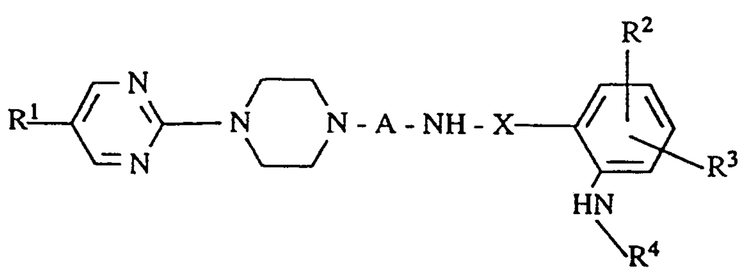

(VIII)

in which

$R^1$, $R^2$, $R^3$, $R^4$, X and A have the abovementioned meaning, are reacted with carbonyl compounds of the general formula

$$R^5—CO—R^6 \quad (IX)$$

in which

$R^5$ denotes halogen, alkoxy, amino or imidazolyl and

$R^6$ denotes halogen, trihalogenoalkyl, alkoxy, aryloxy, alkoxycarbonyloxy, amino or imidazolyl, in the presence of inert solvents at temperatures between 20 and 180°C or without solvent at temperatures between 50 and 200°C, and, in the case of the preparation of the salts, acids are added to convert into the corresponding salt.

8. Medicament containing at least one 2-pyrimidinyl-1-piperazine derivative according to Claim 1.

9. Use of 2-pyrimidinyl-piperazine derivatives according to Claim 1 for the preparation of medicaments.

10. Use of 2-pyrimidinyl-piperazine derivatives according to Claim 1 for the preparation of medicaments acting on the central nervous system.

11. Use of 2-pyrimidinyl-piperazine derivatives according to Claim 1 for the preparation of anxiolytics, tranquillizers, neuroleptics, antidepressants, antiamnesics and nootropics, and agents improving learning, functioning and memory.

12. Piperazine derivatives of the general formula

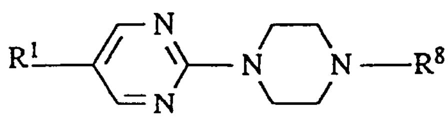

(XII)

in which

$R^1$ represents chlorine, fluorine, iodine, trifluoromethyl, phenyl, 3,4-dimethoxyphenyl, 4-methoxyphenyl, 4-chlorophenyl, 3-trifluoromethylphenyl, 2-chloro-6-fluoro-phenyl, indol-3-yl, and

$R^8$ denotes methoxycarbonyl, ethoxycarbonyl, propoxycarbonyl, isopropoxycarbonyl, butoxycarbonyl or phenoxycarbonyl, where $R^1$ does not represent fluorine when $R^8$ represents ethoxycarbonyl.

13. ω-Aminoalkylpiperazines of the general formula

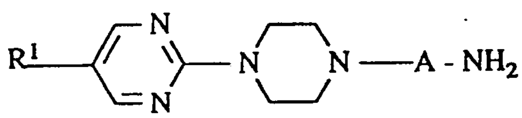

(IV)

in which

$R^1$ represents trifluoromethyl, chlorine, iodine, fluorine, phenyl, 3,4-dimethoxyphenyl, 4-methoxyphenyl, 4-chlorophenyl, 3-(trifluoromethyl)phenyl, 2-chloro-6-fluorophenyl, indol-3-yl and

A represents n-propylene or n-butylene, where $R^1$ does not represent fluorine when A represents n-butylene.

14. Compounds of the general formula

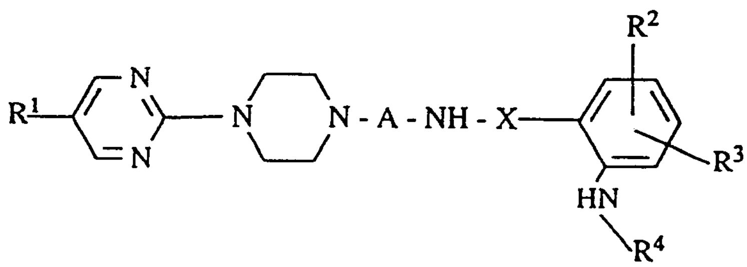

(VIII)

in which

A represents an alkylene group having 1 to 6 carbon atoms or represents 2-hydroxy-n-propylene, but A does not represent the 2-hydroxy-n-propylene group when X and Y represent carbonyl and $R^1$, $R^2$ and $R^3$ represent hydrogen,

$R^1$ represents hydrogen, trifluoromethyl or represents alkoxy having 1 to 4 carbon atoms, trifluoromethoxy or represents hydroxyl, halogen, cyano, nitro, amino, optionally substituted by 1 or 2 methyl or ethyl, or represents acetylamino or benzoylamino or represents indol-3-yl, or represents phenyl, it being possible for the phenyl radical to be monosubstituted or polysubstituted by methoxy, ethoxy, nitro, halogen, trifluoromethyl, amino, cyano, hydroxyl, methyl or ethyl, acetylamino or benzoylamino,

$R^2$ and $R^3$, which can be identical or different, represent hydrogen or alkoxy having 1 to 4 carbon atoms, or represent phenyl, or represent hydroxyl, halogen, cyano, nitro, amino, in which case the nitrogen atom of the amino radical is substituted by 1 or 2 methyl or ethyl, or represent methylsulphonamido, benzene- and toluenesulphonamido, acetylamino, ethoxycarbonylamino or represent carboxyl, methoxy- or ethoxycarbonyl, or represent phenoxycarbonylamino, or represent carbamoyl or sulphamoyl,

X represents carbonyl or sulphonyl and

$R^4$ represents hydrogen or phenyl.

15. Process for the preparation of piperazine derivatives of the formula

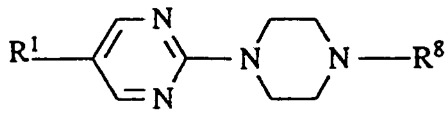

(XII)

in which

$R^1$ represents chlorine, fluorine, iodine, trifluoromethyl, phenyl, 3,4-dimethoxyphenyl, 4-methoxyphenyl, 4-chlorophenyl, 3-trifluoromethylphenyl, 2-chloro-6-fluoro-phenyl, indol-3-yl, and

$R^8$ denotes methoxycarbonyl, ethoxycarbonyl, propoxycarbonyl, isopropoxycarbonyl, butoxycarbonyl or phenoxycarbonyl, where $R^1$ does not represent fluorine when $R^8$ represents ethoxycarbonyl, characterized in that

a) pyrimidine derivatives of the general formula

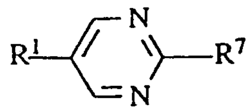

(X)

in which

$R^1$ has the abovementioned meaning, and

$R^7$ represents chlorine or fluorine, are reacted with piperazine derivatives of the formula

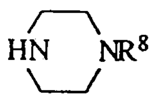

(XI)

in which

$R^8$ represents methoxycarbonyl or ethoxycarbonyl, in a suitable solvent at temperatures from 50 to 150°C, advantageously in the presence of an acid-binding agent, or

b) compounds of the formula

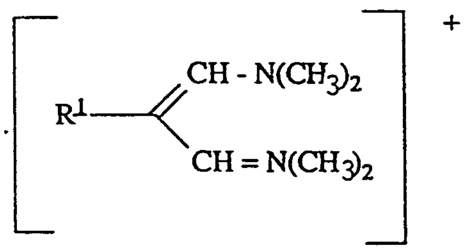

(XIII)

in which

$R^1$ has the abovementioned meaning, and

$X^-$ represents perchlorate, are reacted with piperazine derivatives of the general formula

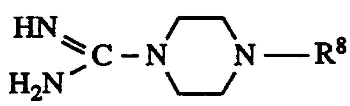

(XIV)

in which

$R^8$ has the abovementioned meaning, in suitable solvents at the boiling point thereof, and worked up in a known manner.

16. Process for the preparation of ω-aminoalkylpiperazines of the general formula

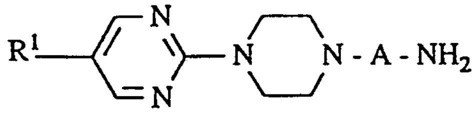

(IV)

in which

$R^1$ represents trifluoromethyl, chlorine, iodine, fluorine, phenyl, 3,4-dimethoxyphenyl, 4-methoxyphenyl, 4-chlorophenyl, 3-(trifluoromethyl)phenyl, 2-chloro-6-fluorophenyl, indol-3-yl and

A represents n-propylene or n-butylene, where $R^1$ does not represent fluorine when A represents n-butylene, characterized in that pyrimidinyl-1-piperazines of the general formula

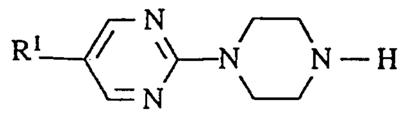

(II)

in which $R^1$ has the abovementioned meaning, are alkylated with halogenoalkyl cyanides of the general formula

$$Cl—(A)_{n-1}—CN$$

where

n denotes the number of methylene groups in chain A, and

A has the abovementioned meaning, and the resulting cyano compounds are reduced.

17. Process for the preparation of the compounds of the general formula

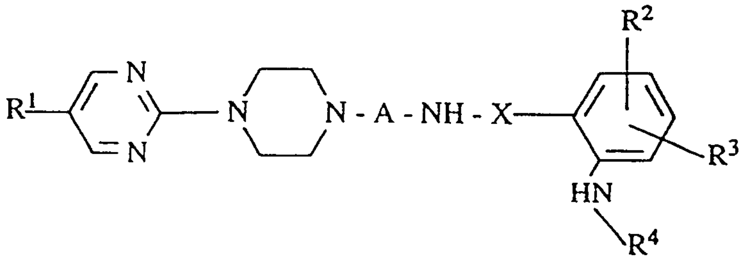

(VIII)

in which

A represents an alkylene group having 1 to 6 carbon atoms or represents 2-hydroxy-n-propylene, but A does not represent the 2-hydroxy-n-propylene group when X and Y represent carbonyl, and $R^1$, $R^2$ and $R^3$ represent hydrogen,

$R^1$ represents hydrogen, trifluoromethyl or represents alkoxy having 1 to 4 carbon atoms, trifluoromethoxy or represents hydroxyl, halogen, cyano, nitro, amino, optionally substituted by 1 or 2 methyl or ethyl, or represents acetylamino or benzoylamino or represents indol-3-yl, or represents phenyl, it being possible for the phenyl radical to be monosubstituted or polysubstituted by methoxy, ethoxy, nitro, halogen, trifluoromethyl, amino, cyano, hydroxyl, methyl or ethyl, acetylamino or benzoylamino,

$R^2$ and $R^3$, which can be identical or different, represent hydrogen or alkoxy having 1 to 4 carbon atoms, or represent phenyl, or represent hydroxy, halogen, cyano, nitro, amino, in which case the nitrogen atom of the amino radical is substituted by 1 or 2 methyl or ethyl, or represent methylsulphonamido, benzene- and toluenesulphonamido, acetylamino, ethoxycarbonylamino or represent carboxyl, methoxy- or ethoxycarbonyl, or represent phenoxycarbonylamino, or represent carbamoyl or sulphamoyl,

X represents carbonyl or sulphonyl and

$R^4$ represents hydrogen or phenyl, characterized in that nitro compounds of the general formula

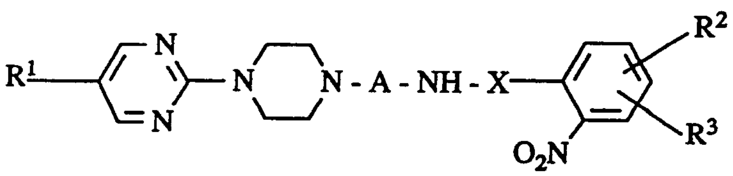

(XXII)

in which

$R^1$, $R^2$, $R^3$, X and A have the abovementioned meaning, are reduced with suitable reducing agents.

18. 2-Pyrimidinyl-1-piperazine derivatives according to Claim 1 for the treatment of diseases.